# EUROPEAN PATENT APPLICATION

(11) **EP 4 556 066 A2**
(43) Date of publication of application: **21.05.2025**
(21) Application number: 25154283.3
(22) Date of filing: 31.01.2019
(51) Int. Cl.: A61P 3/00

(54) **METHODS FOR TREATING FARBER DISEASE**

(30) Priority: 02.02.2018 US 201862625763 P
(62) Divisional of application: 19713837.3
(71) Applicant: Aceragen, Inc., Camden, Kent County, DE 19934 (US)
(72) Inventor: GAUKEL, Eric, Durham (US); SAMPEY, Brante, Durham (US)
(74) Representative: Longland, Emma Louise

(57) **Abstract**

Methods of treating Farber disease using particular doses and pharmacokinetic profiles are disclosed.

## Description

### CROSS REFERENCE TO RELATED APPLICATION

This application claims the benefit to and priority of U.S. Provisional Application No. 62/625,763, filed February 2, 2018, the subject matter of which is incorporated herein by reference.

### SEQUENCE LISTING

The instant application contains a Sequence Listing which has been submitted electronically in ASCII format and is hereby incorporated by reference in its entirety. Said ASCII copy, created on January 17, 2019, is named RVT-801-006WO_SL.txt and is 11,363 bytes in size.

### BACKGROUND

Farber disease, a lysosomal storage disorder (LSD), is a condition that was first described in 1952 in a 14-month-old infant with granulomatous lesions on multiple joints and evidence of lipid storage. Over the ensuing decade other similar cases were described, all of which demonstrated similar lesions and often exhibited a characteristic "hoarse" cry or voice due to the presence of lesions on the larynx. The involvement of other organ systems in some of these patients, including the lung, liver, spleen and central nervous system (CNS), also was noted.

Previously, treatment for Farber disease patients has been symptomatic and principally aimed at reducing pain. Hematopoietic stem cell transplantation (HSCT) has been undertaken in a limited number of patients, and overall the outcome has been positive provided that the transplant procedure itself was successful. Successfully transplanted patients exhibit significant reduction in pain, increased motility and mobility, and in some cases shrinking and complete resolution of the subcutaneous nodules. However, successful transplantation requires histocompatible donor cells, and exposes patients to invasive and potentially dangerous immunosuppressant regimes. One alternative to HSCT is gene therapy in which autologous donor cells are transduced with a vector expressing the therapeutic protein, obviating the need for histocompatible donors. This approach has been evaluated in a Farber disease knock-in mouse model and resulted in reduction of tissue ceramides and macrophage infiltration. However, there continues to be a need for improved therapies for treating Farber disease.

Previous studies in a murine model of severe Farber disease have established a broad therapeutic dose range for recombinant human acid ceramidase ("rhAC"), where efficacy was characterized based on reduction of accumulated tissue ceramide and reduction of pro-inflammatory cytokines. The disclosures in International Application No. PCT/US18/13509 filed January 12, 2018 (published as WO 2018/132667 on July 18, 2018), and in He et al., 2017 (Feb.), "Enzyme replacement therapy for Farber disease: Proof-of-concept studies in cells and mice," BBA Clin. 13 (7): 85-96, are hereby incorporated by reference in their entirety.

However, these studies did not establish exposure targets. Prediction of a human equivalent dose (HED) from a therapeutic dose determined during nonclinical studies in the murine model of severe Farber disease requires an understanding of the enzyme's pharmacokinetics ("PK") and tissue distribution ("TD"). The present subject matter fulfills such needs, as discussed herein.

### SUMMARY OF THE INVENTION

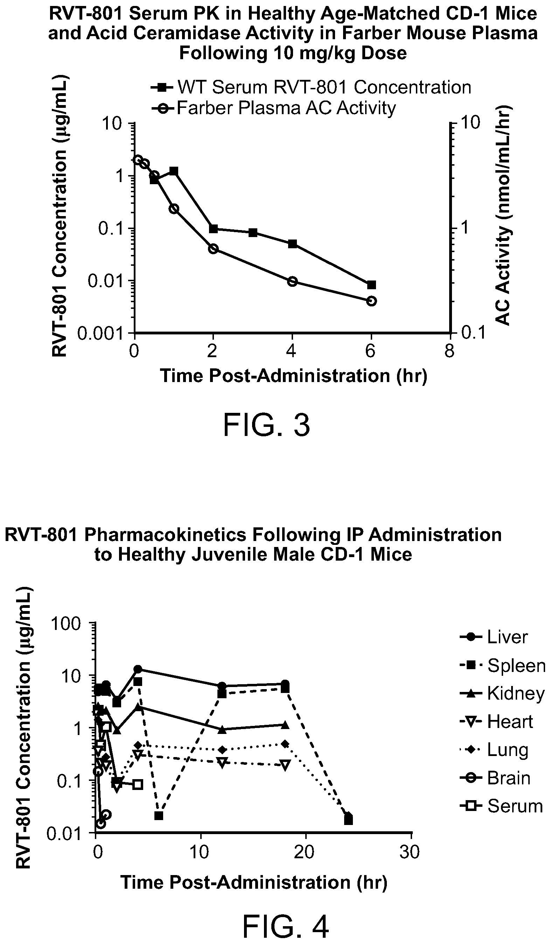

Juvenile, healthy CD-1 mice are considered the parental strain of the Farber mouse (Farber disease mouse model used in this description and Examples). Fig 3 shows that the juvenile CD-1 mouse rhAC (RVT-801) pharmacokinetic profile is similar to the Farber mouse experimental rhAC activity profile in circulation. This indicates that the juvenile CD-1 mouse provides an appropriate approximation of the Farber mouse PK, and as such, CD-1 mice were used to characterize the murine pharmacokinetics of RVT-801, since Farber mice are frail, difficult to mate, and not numerous enough to conduct a full PK assessment.

Thus, based on a small number of Farber mice and with limited overlapping data points between strains, the data reported in the description and Examples herein suggest that systemic rhAC (e.g., RVT-801) exposure in CD-1 mice approximates that in Farber mice, and may represent the minimum systemic levels of rhAC in Farber mice following a single dose of RVT-801 administered intraperitoneally.

Recombinant human AC (*e.g*., RVT-801) has been shown in the description and Examples to distribute extensively to tissues associated with ceramide accumulation in a Farber disease murine model (*e.g.,* liver, spleen, and lung). Measurement of systemic levels of recombinant human AC (*e.g*., RVT-801) could, therefore, potentially underestimate exposure in relevant tissue of human subjects suffering from Farber disease. Thus, relying on simple allometry to scale a dose in nonclinical species to humans may not provide adequate estimates of tissue exposure to impart efficacy.

A human equivalent dose (HED) estimate, based on guidance by the United States Food & Drug Administration ("FDA") for scaling dosages between nonclinical species and humans is based on body surface area ("BSA") (U S. Dept. of Health and Human Services, Food and Drug Administration, Center for Drug Evaluation and Research (CDER), 2005, "Guidance for Industry. Estimating the Maximum Safe Starting Dose in Initial Clinical Trials for Therapeutics in Adult Healthy Volunteers." pp. 1-30), which is hereby incorporated by reference in its entirety. However, the HED estimate approaches described herein also consider physiological factors including, but not limited to, major clearance mechanisms from the vasculature to tissues.

Data reported in this specification in mice indicate that the liver and spleen are major target tissues for the uptake of RVT-801 following systemic administration.

A HED may be derived by combining BSA scaling and the ratios of tissue mass to bodyweight ("BW") in mice and humans. In this manner, it is possible to derive a dose scaling factor that accounts for the relative size of each compartment of recombinant human AC (RVT-801) distribution in each species. The combined HED estimates using BSA and tissue:BW strategies are presented in Table 1.

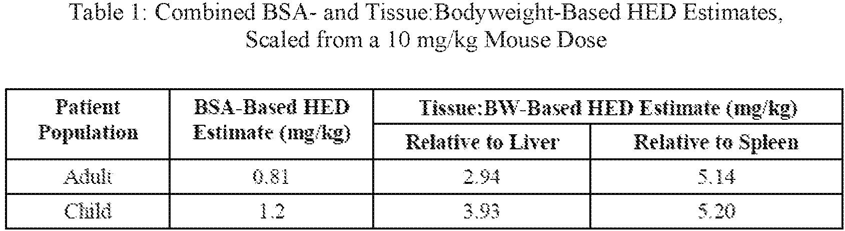

In accordance with the description and Examples, in an embodiment, a method of treating a human adult subject with Farber disease comprising administering to the human subject recombinant human acid ceramidase (rhAC) at a dose of about 0.8 mg/kg, is provided.

Also provided in an embodiment is a method of treating a human child with Farber disease, the method comprising administering to the child recombinant human acid ceramidase (rhAC) at a dose of about 1.2 mg/kg.

Also provided in an embodiment is a method of treating a human child with Farber disease, the method comprising administering to the child recombinant human acid ceramidase (rhAC) at a dose of about 2 mg/kg.

Also provided in an embodiment is a method of treating a human child with Farber disease, the method comprising administering to the child recombinant human acid ceramidase (rhAC) at a dose of about 5 mg/kg.

In some embodiments, a method of treating a human subject with Farber disease may comprise administering to the human subject a therapeutically effective dose of recombinant human acid ceramidase (rhAC) that may be determined based on the pharmacokinetic profile, obtained in juvenile, healthy CD-1 mice receiving a single, maximum effective dose ("MED") of 10mg/kg of rhAC (i.e., RVT-801) intraperitoneally, wherein the rhAC elicits in the healthy CD-1 mouse one or more of: Tₘₐₓ of about 0.25 to 1.0 hours, Cₘₐₓ of about 1.23 to about 2.17 µg/mL, or area under the curve (AUC) of about 1.37 hr*µg/mL to about 1.49 hr*µg/mL (or about 1 hr*µg/mL to about 2 hr*µg/mL), as disclosed below in this specification. The maximum effective dose (MED) in Farber mice was determined to be 10 mg/kg delivered by bolus IP injection. The value for AUCₗₐₛₜ determined following a single 10 mg/kg dose to aged-matched, wild-type CD-1 mice may be 1.37 µg.h/mL with a Cₘₐₓ of 1.23 µg/mL.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse ratios greater than 1 for AUCₗᵢᵥₑᵣ:AUCₛₑᵣᵤₘ, AUCₛₚₗₑₑₙ:AUCₛₑᵣᵤₘ, AUC_{lung}:AUCₛₑᵣᵤₘ, AUC_{kidney}:AUCₛₑᵣᵤₘ, AUCₕₑₐᵣₜ:AUCₛₑᵣᵤₘ. Without being bound by theory, distribution of rhAC (RVT-801) to these tissues is believed to be important with regard to the overall effectiveness of the drug treatment. It is likely that glycosylation of RVT-801 plays a role in the uptake of the drug into these disease-affected tissues, thereby positively impacting the efficacy of the rhAC treatment.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse one or more of: an AUCₗᵢᵥₑᵣ:AUCₛₑᵣᵤₘ ratio of about 92.9, an AUCₛₚₗₑₑₙ:AUCₛₑᵣᵤₘ ratio of about 47.6, an AUC_{lung}:AUCₛₑᵣᵤₘ ratio of about 5.64, an AUC_{kidney}:AUCₛₑᵣᵤₘ ratio of about 17.3, an AUCₕₑₐᵣₜ:AUCₛₑᵣᵤₘ ratio of about 2.69, an AUC_{whole blood}:AUCₛₑᵣᵤₘ ratio of about 0.575, an AUC_{brain}:AUCₛₑᵣᵤₘ ratio of about 0.0281, or an AUC_{BALF}:AUCₛₑᵣᵤₘ ratio of about 0.000165 in mice (where BALF is bronchoalveolar lavage fluid). In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse ratios greater than 1 for AUCₗᵢᵥₑᵣ:AUCₛₑᵣᵤₘ, AUCₛₚₗₑₑₙ:AUCₛₑᵣᵤₘ, AUC_{lung}:AUCₛₑᵣᵤₘ, AUC_{kidney}:AUCₛₑᵣᵤₘ, AUCₕₑₐᵣₜ:AUCₛₑᵣᵤₘ, AUC_{whole blood}:AUCₛₑᵣᵤₘ, AUC_{brain}:AUCₛₑᵣᵤₘ.

Without being bound by theory, distribution of rhAC (RVT-801) to these tissues is believed to be important with regard to the overall effectiveness of the drug treatment.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Tₘₐₓ of about 0.25 to 1.0 hours.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ of about 1.23 µg/mL to 2.17 µg/mL.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC of about 1.37 to 1.49 hr*µg/mL.

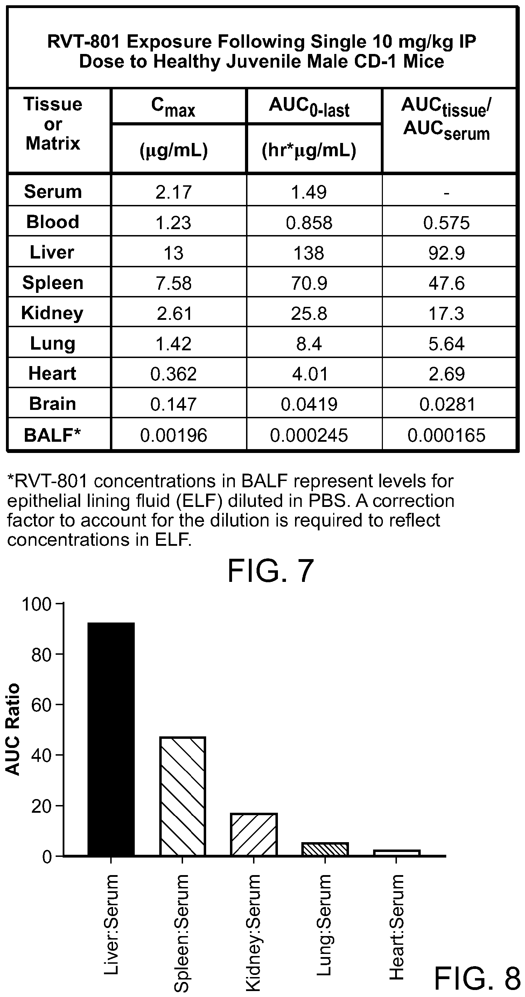

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ, or AUC₀₋ₗₐₛₜ or AUCₜᵢₛₛᵤₑ /AUCₛₑᵣᵤₘ in tissue or matrix in accordance with the values set forth in Fig. 7.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC₀ to last of about 138 hr*µg/mL in liver tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 13 µg/mL in liver tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC_{0 to last} of about 70.9 hr*µg/mL in splenic tissue

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 7.58 µg/mL in splenic.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC_{0 to last} of about 25.8 hr*µg/mL in kidney tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 2.61 µg/mL in kidney tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC_{0 to last} of about 4.01 hr*µg/mL in heart tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 0.362 µg/mL in heart tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC_{0 to last} of about 0.0419 hr*µg/mL in brain tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ of about 0.147 µg/mL in brain.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse AUC_{0 to last} of about 0.858 hr*µg/mL in blood.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 1.23 µg/mL in blood.

In some embodiments, a method of treating a human subject with Farber disease may comprise administering to the human subject a therapeutically effective dose of recombinant human acid ceramidase (rhAC) that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, that provides AUC_{0 to last} of about 0.000245 hr* µg/mL in BALF.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 0.00196 µg/mL in BALF.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse an AUC_{0 to last} of about 8.4 hr*µg/mL in lung tissue.

In some embodiments, a method of treating a human subject with Farber disease may comprise administering to the subject a therapeutically effective dose of rhAC that may be determined based on the pharmacokinetic profile, obtained in juvenile, healthy CD-1 mice receiving a single, maximum effective dose ("MED") of 10 mg/kg dose of rhAC (i.e., RVT-801) intraperitoneally, wherein the rhAC elicits in the healthy CD-1 mouse a Cₘₐₓ for the drug of about 1.42 µg/mL in lung tissue.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawings, which are incorporated in and constitute a part of this specification, illustrate one (several) embodiment(s) and together with the description, serve to explain the principles described herein.

### BRIEF DESCRIPTION OF THE DRAWINGS

Fig. 1 shows a time course of serum RVT-801 levels in healthy, juvenile CD-1 mice, age-matched to Farber disease mice treated with RVT-801, after a single injection of RVT-801 at doses of 1, 3, and 10 mg/kg according to Example 1.
Fig. 2 lists pharmacokinetic metrics of serum RVT-801 in healthy, juvenile CD-1 mice after a single injection of RVT-801 at doses of 1, 3, and 10 mg/kg according to Example 1.
Fig. 3 shows RVT-801 concentrations in healthy age-matched CD-1 mouse serum and rhAC activity in plasma of Farber disease mice after a single injection of RVT-801 at a dose of 10 mg/kg according to Example 1, showing comparability between Farber mouse and age-matched parental CD-1 strain mouse pharmacokinetic profiles.
Fig. 4 shows a time course of RVT-801 concentrations in serum and various tissues (liver, spleen, kidney, heart, lung, and brain) of healthy, juvenile CD-1 mice after a single injection of serum RVT-801 at a dose of 10 mg/kg according to Example 2.
Fig. 5A shows a time course of RVT-801 concentrations in liver tissue of healthy, juvenile CD-1 mice after a single injection of RVT-801 at doses of 1, 3, and 10 mg/kg according to Example 2.
Fig. 5B shows a time course of RVT-801 concentrations in spleen tissue of healthy, juvenile CD-1 mice after a single injection of serum RVT-801 at doses of 1, 3, and10 mg/kg according to Example 2.
Fig. 6A compares AUC₀₋ₗₐₛₜ in serum, whole blood, liver tissue extract, and spleen tissues of healthy, juvenile CD-1 mice after a single injection of serum RVT-801 at doses of 1, 3, and10 mg/kg according to Example 2.
Fig. 6B shows AUCₜᵢₛₛᵤₑ/AUCₛₑᵣᵤₘ AUC ratios calculated from RVT-801 concentrations in serum, along with hepatic and spleenic tissues of healthy, juvenile CD-1 mice after a single injection of RVT-801 at doses of 1, 3, and 10 mg/kg according to Example 2.
Fig. 7 shows Cₘₐₓ, AUC₀₋ₗₐₛₜ, and AUCₜᵢₛₛᵤₑ/AUCₛₑᵣᵤₘ calculated from RVT-801 concentrations in serum, whole blood, and various tissue extracts (liver, spleen, kidney, lung, heart, brain and bronchoalveolar lavage fluid (BALF) of healthy, juvenile CD-1 mice after a single injection of RVT-801 at a dose of 10 mg/kg according to Example 2.
Fig. 8 shows tissue distribution and comparison of AUCₜᵢₛₛᵤₑ/AUCₛₑᵣᵤₘ in various tissues of healthy, juvenile CD-1 mice after a single injection of RVT-801 at a dose of 10 mg/kg according to Example 2.
Fig. 9 shows estimated human equivalent doses (HEDs) scaled by body surface area (BSA), estimated from the MED with respect to reduction of accumulated ceramides in liver and spleen of 10 mg/kg in Farber mice according to Example 3.
Fig. 10 shows tissue-specific HEDs, scaled by tissue-to-body weight ratio, estimated from the MED of 10 mg/kg in Farber mice with respect to reduction of accumulated ceramides in liver and spleen according to Example 3.
Fig. 11 shows results of both (1) BSA-based RED calculations and (2) tissue-to-body weight ratio-based RED calculations according to Example 3.
Fig. 12 shows comparison of an embodiment of HED for RVT-801 in the present application to the doses of approved enzyme replacement therapies (ERTs).
Fig. 13 shows mean rhAC concentration-time data in liver, spleen, and kidney of Farber mice following a single or repeat weekly 10 mg/kg dose of RVT-801.
Fig. 14 shows data on rhAC serum concentrations in healthy age-matched CD-1 mice and acid ceramidase plasma activity in Farber mice following a single 10 mg/kg dose of RVT-801.
Fig. 15 shows final rhAC tissue:serum AUC ratio in healthy juvenile CD-1 mice following a single 10 mg/kg dose of RVT-801.
Fig. 16 shows rhAC in circulation following single 10 mg/kg IP bolus injection of RVT-801 to CD-1 and Farber mice.
Fig. 17 shows rhAC concentrations in circulation and in key tissues following a single 10 mg/kg bolus IP injection of RVT-801 to CD-1 and Farber mice.
Figs. 18A-E present individual rhAC concentration-time data in the liver (Fig. 18A), spleen (Fig. 18B), kidney (Fig. 18C), lung (Fig. 18D), and heart (Fig. 18E) (Farber (open symbols) and age-matched CD-1 (filled symbols)) mice following a single 10 mg/kg bolus IP injection of RVT-801 (n=3 per time point).
Figs. 19A-G present the mean rhAC concentrations in circulation (Fig. 19A), liver (Fig. 19B), spleen (Fig. 19C), kidney (Fig. 19D), heart (Fig. 19E), lung (Fig. 19F), and brain (Fig. 19G) tissue in Farber mice (open symbols) and age-matched CD-1 mice (filled symbols) following either a single 10 mg/kg dose or multiple once-weekly doses at a 10 mg/kg/dose of RVT-801 administered via bolus IP injection, in accordance with Example 5.
Figs. 20A and 20B show mean rhAC tissue concentration-time profiles following IP administration to juvenile CD-1 mice in RVT-801-9013 Part B (Linear Fig. 20A and Log-Linear Fig. 20B), respectively.
Fig. 21 presents RVT-801 tissue: serum exposure ratios in BALF, blood, brain, heart, kidney, liver, lung, and spleen based on AUCₗₐₛₜ following single doses of RVT-801 of 1mg/kg, 3 mg/kg, and 10 mg/kg to juvenile CD-1 mice.
Figs. 22A-D presents dose normalized AUC plotted verses dose level for various tissues following IP administration of RVT-801 at doses of 1 mg/kg, 3 mg/kg and 10 mg/kg in juvenile CD-1 mice.

### DESCRIPTION OF THE SEQUENCES

**Table 2: a listing of certain sequences referenced herein.**

| SEQ ID NO | Description | Sequence |
|---|---|---|
| 1 | recombinant human acid ceramidase (rhAC) (amino acid) | |
| 2 | rhAC (DNA) | GGCTCGGTCCGACTATTGCCCGCGGTGGGGGAGGGG GATGGATCACGCCACGCGCCAAAGGCGATCGCGACT CTCCTTCTGCAGGTAGCCTGGAAGGCTCTCTCTCTTTC TCTACGCCACCCTTTTCGTGGCACTGAAAAGCCCCGT CCTCTCCTCCCAGTCCCGCCTCCTCCGAGCGTTCCCCC TACTGCCTGGAATGGTGCGGTCCCAGGTCGCGGGTCA CGCGGCGGAGGGGGCGTGGCCTGCCCCCGGCCCAGC CGGCTCTTCTTTGCCTCTGCTGGAGTCCGGGGAGTGG CGTTGGCTGCTAGAGCGATGCCGGGCCGGAGTTGCGT CGCCTTAGTCCTCCTGGCTGCCGCCGTCAGCTGTGCC GTCGCGCAGCACGCGCCGCCGTGGACAGAGGACTGC AGAAAATCAACCTATCCTCCTTCAGGACCAACGTACA GAGGTGCAGTTCCATGGTACACCATAAATCTTGACTT ACCACCCTACAAAAGATGGCATGAATTGATGCTTGAC |
| | | AAGGCACCAGTGCTAAAGGTTATAGTGAATTCTCTGA AGAATATGATAAATACATTCGTGCCAAGTGGAAAAA TTATGCAGGTGGTGGATGAAAAATTGCCTGGCCTACT TGGCAACTTTCCTGGCCCTTTTGAAGAGGAAATGAAG GGTATTGCCGCTGTTACTGATATACCTTTAGGAGAGA TTATTTCATTCAATATTTTTTATGAATTATTTACCATT TGTACTTCAATAGTAGCAGAAGACAAAAAAGGTCAT CTAATACATGGGAGAAACATGGATTTTGGAGTATTTC TTGGGTGGAACATAAATAATGATACCTGGGTCATAAC TGAGCAACTAAAACCTTTAACAGTGAATTTGGATTTC CAAAGAAACAACAAAACTGTCTTCAAGGCTTCAAGC TTTGCTGGCTATGTGGGCATGTTAACAGGATTCAAAC CAGGACTGTTCAGTCTTACACTGAATGAACGTTTCAG TATAAATGGTGGTTATCTGGGTATTCTAGAATGGATT CTGGGAAAGAAAGATGTCATGTGGATAGGGTTCCTC ACTAGAACAGTTCTGGAAAATAGCACAAGTTATGAA GAAGCCAAGAATTTATTGACCAAGACCAAGATATTG GCCCCAGCCTACTTTATCCTGGGAGGCAACCAGTCTG GGGAAGGTTGTGTGATTACACGAGACAGAAAGGAAT CATTGGATGTATATGAACTCGATGCTAAGCAGGGTAG ATGGTATGTGGTACAAACAAATTATGACCGTTGGAA ACATCCCTTCTTCCTTGATGATCGCAGAACGCCTGCA AAGATGTGTCTGAACCGCACCAGCCAAGAGAATATC TCATTTGAAACCATGTATGATGTCCTGTCAACAAAAC CTGTCCTCAACAAGCTGACCGTATACACAACCTTGAT AGATGTTACCAAAGGTCAATTCGAAACTTACCTGCGG GACTGCCCTGACCCTTGTATAGGTTGGTGAGCACACG TCTGGCCTACAGAATGCGGCCTCTGAGACATGAAGA CACCATCTCCATGTGACCGAACACTGCAGCTGTCTGA CCTTCCAAAGACTAAGACTCGCGGCAGGTTCTCTTTG AGTCAATAGCTTGTCTTCGTCCATCTGTTGACAAATG |
| | | ACAGATCTTTTTTTTTTCCCCCTATCAGTTGATTTTTCT TATTTACAGATAACTTCTTTAGGGGAAGTAAAACAGT CATCTAGAATTCACTGAGTTTTGTTTCACTTTGACATT TGGGGATCTGGTGGGCAGTCGAACCATGGTGAACTC CACCTCCGTGGAATAAATGGAGATTCAGCGTGGGTGT TGAATCCAGCACGTCTGTGTGAGTAACGGGACAGTA AACACTCCACATTCTTCAGTTTTTCACTTCTACCTACA TATTTGTATGTTTTTCTGTATAACAGCCTTTTCCTTCT GGTTCTAACTGCTGTTAAAATTAATATATCATTATCTT TGCTGTTATTGACAGCGATATAATTTTATTACATATG ATTAGAGGGATGAGACAGACATTCACCTGTATATTTC TTTTAATGGGCACAAAATGGGCCCTTGCCTCTAAATA GCACTTTTTGGGGTTCAAGAAGTAATCAGTATGCAAA GCAATCTTTTATACAATAATTGAAGTGTTCCCTTTTTC ATAATTACTCTACTTCCCAGTAACCCTAAGGAAGTTG CTAACTTAAAAAACTGCATCCCACGTTCTGTTAATTT AGTAAATAAACAAGTCAAAGACTTGTGGAAAATAGG AAGTGAACCCATATTTTAAATTCTCATAAGTAGCATT CATGTAATAAACAGGTTTTTAGTTTGTTCTTCAGATTG ATAGGGAGTTTTAAAGAAATTTTAGTAGTTACTAAAA TTATGTTACTGTATTTTTCAGAAATCAAACTGCTTATG AAAAGTACTAATAGAACTTGTTAACCTTTCTAACCTT CACGATTAACTGTGAAATGTACGTCATTTGTGCAAGA CCGTTTGTCCACTTCATTTTGTATAATCACAGTTGTGT TCCTGACACTCAATAAACAGTCACTGGAAAGAGTGC CAGTCAGCAGTCATGCACGCTGATTGGGTGTGT |
| 3 | rhAC (DNA) | AAGCTTACCGCCACCATGAACTGCTGCATCGGCCTGG GTGAGAAGGCGCGTGGCTCGCACCGCGCCAGCTACC CCTCCCTGAGCGCCCTCTTCACCGAGGCGTCCATCCT CGGATTCGGGAGCTTCGCCGTCAAGGCACAGTGGAC CGAGGATTGCCGCAAGAGTACGTACCCCCCCAGTGG |
| | | CCCGACGTACCGCGGCGCCGTCCCCTGGTACACGATC AACCTGGACCTCCCCCCGTACAAGCGCTGGCACGAGT TGATGCTGGACAAGGCCCCCGTACTGAAGGTCATCGT GAACTCCCTGAAGAACATGATCAACACCTTCGTCCCC TCGGGCAAGATCATGCAGGTCGTGGACGAGAAGCTG CCCGGGCTCCTCGGCAACTTCCCCGGCCCGTTCGAAG AGGAGATGAAGGGCATCGCGGCCGTCACTGACATCC CCCTGGGCGAGATCATCAGCTTCAACATCTTCTACGA GCTGTTCACCATCTGCACCTCCATCGTAGCCGAGGAC AAGAAGGGCCACCTGATCCACGGTCGCAACATGGAC TTCGGCGTCTTCCTGGGCTGGAACATCAACAACGACA CCTGGGTCATCACCGAGCAGCTGAAGCCGCTCACCGT GAACCTCGATTTCCAGCGCAACAACAAGACGGTGTTC AAGGCCAGCTCCTTCGCCGGGTACGTCGGGATGCTCA CGGGCTTCAAGCCGGGACTGTTCTCGCTGACCCTCAA CGAGCGGTTCTCCATCAACGGGGGCTACCTCGGCATC CTGGAGTGGATTCTCGGCAAGAAGGACGTGATGTGG ATCGGCTTCCTCACACGGACCGTGCTGGAAAACTCCA CTAGTTACGAGGAGGCCAAGAACCTGCTGACCAAGA CGAAGATCCTGGCCCCGGCATACTTCATCCTGGGCGG CAACCAGTCGGGCGAGGGGTGCGTCATCACCCGCGA CCGGAAGGAGTCCCTGGACGTCTATGAGCTGGACGC CAAGCAGGGCCGCTGGTACGTCGTCCAGACGAACTA CGACCGATGGAAGCACCCCTTCTTCCTCGACGACCGG CGCACGCCCGCCAAGATGTGCCTGAACCGCACCAGC CAGGAGAACATCTCGTTCGAGACGATGTACGACGTG CTGTCGACCAAGCCCGTGCTCAACAAGCTGACGGTCT ACACCACGCTGATCGACGTGACGAAAGGCCAGTTCG AAACGTACCTGCGGGACTGCCCGGACCCTTGCATCGG CTGGTGATAATCTAGAGTCGGGGCGGCCGGCC |
| 4 | rhAC (DNA) | AAGCTTACCGCCACCATGAACTGCTGCATCGGGCTGG GAGAGAAAGCTCGCGGGTCCCACCGGGCCTCCTACC CAAGTCTCAGCGCGCTTTTCACCGAGGCCTCAATTCT GGGATTTGGCAGCTTTGCTGTGAAAGCCCAATGGACA GAGGACTGCAGAAAATCAACCTATCCTCCTTCAGGAC CAACGTACAGAGGTGCAGTTCCATGGTACACCATAA ATCTTGACTTACCACCCTACAAAAGATGGCATGAATT GATGCTTGACAAGGCACCAGTGCTAAAGGTTATAGT GAATTCTCTGAAGAATATGATAAATACATTCGTGCCA AGTGGAAAAATTATGCAGGTGGTGGATGAAAAATTG CCTGGCCTACTTGGCAACTTTCCTGGCCCTTTTGAAG AGGAAATGAAGGGTATTGCCGCTGTTACTGATATACC TTTAGGAGAGATTATTTCATTCAATATTTTTTATGAAT TATTTACCATTTGTACTTCAATAGTAGCAGAAGACAA AAAAGGTCATCTAATACATGGGAGAAACATGGATTT TGGAGTATTTCTTGGGTGGAACATAAATAATGATACC TGGGTCATAACTGAGCAACTAAAACCTTTAACAGTGA ATTTGGATTTCCAAAGAAACAACAAAACTGTCTTCAA GGCTTCCAGCTTTGCTGGCTATGTGGGCATGTTAACA GGATTCAAACCAGGACTGTTCAGTCTTACACTGAATG AACGTTTCAGTATAAATGGTGGTTATCTGGGTATTCT AGAATGGATTCTGGGAAAGAAAGATGTCATGTGGAT AGGGTTCCTCACTAGAACAGTTCTGGAAAATAGCAC AAGTTATGAAGAAGCCAAGAATTTATTGACCAAGAC CAAGATATTGGCCCCAGCCTACTTTATCCTGGGAGGC AACCAGTCTGGGGAAGGTTGTGTGATTACACGAGAC AGAAAGGAATCATTGGATGTATATGAACTCGATGCT AAGCAGGGTAGATGGTATGTGGTACAAACAAATTAT GACCGTTGGAAACATCCCTTCTTCCTTGATGATCGCA GAACGCCTGCAAAGATGTGTCTGAACCGCACCAGCC AAGAGAATATCTCATTTGAAACCATGTATGATGTCCT |
| | | GTCAACAAAACCTGTCCTCAACAAGCTGACCGTATAC ACAACCTTGATAGATGTTACCAAAGGTCAATTCGAAA CTTACCTGCGGGACTGCCCTGACCCTTGTATAGGTTG GTGATAACCTAGGGTCGGGGCGGCCGGCC |

### DESCRIPTION OF THE EMBODIMENTS

As used herein, the terms "a" or "an" means that "at least one" or "one or more" unless the context clearly indicates otherwise.

As used herein, the term "about" means that the numerical value is approximate and small variations would not significantly affect the practice of the disclosed embodiments. Where a numerical limitation is used, unless indicated otherwise by the context, "about" means the numerical value can vary by ±10% and remain within the scope of the disclosed embodiments.

As used herein, "acid sphingomyelinase activity" or "ASM activity" refers to a related lipid hydrolase that tightly binds to AC and co-purifies with it (Bernardo, K., R. Hurwitz, T. Zenk, R.J. Desnick, K. Ferlinz, E.H. Schuchman, and K. Sandhoff, 1995, "Purification, characterization, and biosynthesis of human acid ceramidase," J Biol Chem, 270:11098-11102).

As used herein, the term "animal" includes, but is not limited to, humans and non-human vertebrates such as wild, domestic, and farm animals. The animal can also be referred to as a "subject."

As used herein, the term "carrier" means a diluent, adjuvant, or excipient with which a compound is administered. Pharmaceutical carriers can be liquids, such as water and oils, including those of petroleum, animal, vegetable or synthetic origin, such as peanut oil, soybean oil, mineral oil, sesame oil and the like. The pharmaceutical carriers can also be saline, gum acacia, gelatin, starch paste, talc, keratin, colloidal silica, urea, and the like. In addition, auxiliary, stabilizing, thickening, lubricating and coloring agents can be used.

As used herein, "child" refers to an age that is from newborn to 18 years old.

As used herein, the terms "comprising" (and any form of comprising, such as "comprise", "comprises", and "comprised"), "having" (and any form of having, such as "have" and "has"), "including" (and any form of including, such as "includes" and "include"), or "containing" (and any form of containing, such as "contains" and "contain"), are inclusive or open-ended and do not exclude additional, unrecited elements or method steps. Additionally, a term that is used in conjunction with the term "comprising" is also understood to be able to be used in conjunction with the term "consisting of" or "consisting essentially of."

As used herein, the term "contacting" means bringing together of two elements in an in vitro system or an in vivo system. For example, "contacting" rhAC polypeptide an individual, subject, or cell includes the administration of the polypeptide to an individual or patient, such as a human, as well as, for example, introducing a compound into a sample containing a cellular or purified preparation containing the polypeptide. Additionally, contacting can refer to transfecting or infecting a cell with a nucleic acid molecule encoding the polypeptide.

As used herein, "Farber mouse," "Farber mice," "Farber disease mice," and "Farber disease mouse" means severe Farber disease mouse model (*Asah1*^{*P361R*/*P31R6*}). The Farber mouse is a murine model based on a severe Farber disease patient genotype. Based on the severe Farber disease patient genotype, knock-in mice that are homozygous for a single nucleotide *Asah1*^{*P361R*/*P361R*} mutation were derived from a mixed genetic background colony (W4/129Sv/CD-1) ("CD-1 mice") to establish a murine model of severe Farber disease, as previously described (Alayoubi, A.M., J.C. Wang, B.C. Au, S. Carpentier, V. Garcia, S. Dworski, S. El-Ghamrasni, K.N. Kirouac, M.J. Exertier, Z.J. Xiong, G.G. Prive, C.M. Simonaro, J. Casas, G. Fabrias, E.H. Schuchman, P.V. Turner, R. Hakem, T. Levade, and J.A. Medin, 2013, Systemic ceramide accumulation leads to severe and varied pathological consequences, EMBO Mol Med, 5:827-842). Farber mice have been established in knock-in mice homozygous for a single nucleotide mutation in the Asah1 gene. Farber (Asah1^{P361R/P31R6}) mice produce an altered AC, incapable of hydrolyzing ceramides to their sphingosine and fatty acids constituents. These Farber mice exhibit features characteristic of clinical Farber disease including disruption of bone formation and the morphology of intra-articular tissues, presence of lipid-laden macrophages, and systemic inflammation, along with a significantly stunted rate of growth and shortened lifespan compared to their wild-type (Asah1^{WT/WT}) or heterozygous (Asah1^{WT/P361R}) littermates.

An "effective amount" of an enzyme delivered to a subject is an amount sufficient to improve the clinical course of a Farber disease where clinical improvement is measured by any of the variety of defined parameters well known to the skilled artisan.

As used herein, the terms "subject," "individual" or "patient," used interchangeably, means any animal, including mammals, such as mice, rats, other rodents, rabbits, dogs, cats, swine, cattle, sheep, horses, or primates, such as humans.

As used herein, the phrase "in need thereof" means that the subject has been identified as having a need for the particular method or treatment. In some embodiments, the identification can be by any means of diagnosis. In any of the methods and treatments described herein, the subject can be in need thereof.

As used herein, the phrase "integer from X to Y" means any integer that includes the endpoints. For example, the phrase "integer from 1 to 5" means 1, 2, 3, 4, or 5.

As used herein, the term "isolated" means that the compounds described herein are separated from other components of either (a) a natural source, such as a plant or cell, or (b) a synthetic organic chemical reaction mixture, such as by conventional techniques.

As used herein, the term "mammal" means a rodent (i.e., a mouse, a rat, or a guinea pig), a monkey, a cat, a dog, a cow, a horse, a pig, or a human. In some embodiments, the mammal is a human.

As used herein, the phrase "pharmaceutically acceptable" means those compounds, materials, compositions, and/or dosage forms which are, within the scope of sound medical judgment, suitable for use in contact with tissues of humans and animals. In some embodiments, "pharmaceutically acceptable" means approved by a regulatory agency of the Federal or a state government or listed in the U.S. Pharmacopeia or other generally recognized pharmacopeia for use in animals, and more particularly in humans.

As used herein, the term "serum" means any blood-derived matrix, including, without limitation, "serum," "plasma," or "whole blood."

As used herein, the phrase "substantially isolated" means a compound that is at least partially or substantially separated from the environment in which it is formed or detected.

As used herein, the phrase "therapeutically effective amount" means the amount of active compound or pharmaceutical agent that elicits the biological or medicinal response that is being sought in a tissue, system, animal, individual or human by a researcher, veterinarian, medical doctor, or other clinician. The therapeutic effect is dependent upon the disorder being treated or the biological effect desired. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size and sex of the subject. Optimal amounts can also be determined based on monitoring of the subject's response to treatment.

Any method known to the skilled artisan may be used to monitor disease status and the effectiveness of the therapy. Clinical monitors of disease status may include but are not limited to ceramide levels, weight, joint length, inflammation, or any other clinical phenotype known to be associated with Farber disease.

As used herein, the terms "treat," "treated," or "treating" mean both therapeutic treatment and prophylactic measures wherein the object is to slow down (lessen) an undesired physiological condition, disorder or disease, or obtain beneficial or desired clinical results. For example, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; an amelioration of at least one measurable physical parameter, not necessarily discernible by the patient; or enhancement or improvement of condition, disorder or disease. Thus, "treatment of Farber disease" or "treating Farber disease" means an activity that alleviates or ameliorates any of the primary phenomena or secondary symptoms associated with Farber disease or other condition described herein.

It is further appreciated that certain features described herein, which are, for clarity, described in the context of separate embodiments, can also be provided in combination in a single embodiment. Conversely, various features which are, for brevity, described in the context of a single embodiment, can also be provided separately or in any suitable subcombination.

In some embodiments, methods of treating Farber disease are provided. In some embodiments, the subject is a subject in need thereof. In some embodiments, the subject in need thereof is diagnosed with Farber disease. In some embodiments, the subject is also identified as having: 1) subcutaneous nodules; 2) an acid ceramidase activity value in white blood cells, cultured skin fibroblasts, or other biological sources (e.g., plasma) that is less than 30% of control values; and/or 3) nucleotide changes within both alleles of the acid ceramidase gene (*ASAH*1) that indicate, through bioinformatic, gene expression studies, and/or other methods, a possible loss of function of the acid ceramidase protein. In some embodiments, the methods comprising administering to the subject a pharmaceutical composition comprising a recombinant human acid ceramidase in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing lipogranulomas in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing absolute or normalized spleen weight in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing ceramide levels in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

Reducing ceramide can also refer to decreasing ceramide or increasing the metabolizing of ceramide, which would lead to reduced ceramide levels.

In some embodiments, methods of increasing sphingosine levels in a subject with, or suspected of having, Farber disease are provided. In some embodiments, the subject is a subject in need thereof. In some embodiments, the methods comprising administering to the subject a pharmaceutical composition comprising a recombinant human acid ceramidase in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 ,g/kg or 10 mg/kg.

Various pharmaceutical compositions are described herein and can be used based upon the patient's and doctor's preferences. However, in some embodiments, the pharmaceutical composition is a solution. In some embodiments, the pharmaceutical composition comprises cell conditioned media comprising the rhAC. As used herein, the term "cell conditioned media" refers to cell culture media that has been used to culture cells expressing rhAC and where the protein is secreted into the media and then the protein is isolated or purified from the media. In some embodiments, the media is used to treat the subject. The media, for example, can be applied to the skin of a subject to treat any of the conditions, symptoms, or disorders described herein.

In addition to the routes of administration described herein, in some embodiments, the pharmaceutical composition is administered by contacting the skin of the subject. In some embodiments, the administration is parenteral administration. In some embodiments, the administration comprises injecting the pharmaceutical composition to the subject. In some embodiments, the administration is an intraperitoneal injection or intravenous injection. In some embodiments, the administration is oral administration.

In some embodiments, methods of treating Farber disease in a subject in need thereof are provided, wherein the method comprises expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing lipogranulomas in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments, the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, the method comprises treating lipogranulomatosis (now, Farber disease) in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing spleen weight (absolute or normalized to body weight) in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of reducing ceramide in a subject with, or suspected of having, Farber disease are provided, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, methods of increasing sphingosine in a subject with, or suspected of having, Farber disease, the methods comprising expressing recombinant human acid ceramidase (rhAC) in a cell; isolating the expressed rhAC from the cell; and administering to the subject a pharmaceutical composition comprising the isolated expressed rhAC in an effective amount of about 0.1 mg/kg to about 50 mg/kg. In some embodiments, the methods comprising administering to a human adult subject a pharmaceutical composition comprising a recombinant human acid ceramidase in an effective amount of about 0.8 mg/kg. In some embodiments, the methods comprising administering to a human child a pharmaceutical composition comprising a recombinant human acid ceramidase in an effective amount of about 1.2 mg/kg. In some embodiments the methods comprising administering to the subject a pharmaceutical composition comprising a recombinant human acid ceramidase in an effective amount of about 1 mg/kg to about 5 mg/kg. In some embodiments the methods comprising administering to the subject a pharmaceutical composition comprising a recombinant human acid ceramidase in an effective amount of about 2 mg/kg, 3 mg/kg, 4 mg/kg, 5 mg/kg, 6 mg/kg, 7 mg/kg, 8 mg/kg, 9 mg/kg or 10 mg/kg.

In some embodiments, the expressing recombinant human acid ceramidase (rhAC) in a cell comprises transferring a vector encoding rhAC into the cell. In some embodiments, the vector comprises a nucleic acid molecule encoding rhAC. In some embodiments, the nucleic acid molecule is a molecule as described herein or any other nucleic acid molecule that encodes the rhAC polypeptide or homolog thereof, which is described in more detail herein. In some embodiments, the vector is a viral vector. For example, the vector can be a retroviral vector or a DNA virus vector, such as adenovirus, AAV, and the like. In some embodiments, the vector is a plasmid. In some embodiments, the vector comprises a promoter operably linked to the rhAC. In some embodiments, the promoter is a constitutive promoter. In some embodiments, the promoter is the SV40 promoter, CMV promoter, EF1 alpha promoter, or any combination thereof, or any other promoter that is active in a mammalian cell.

In some embodiments, the vector is transfected or infected into the cell. The methods of introducing the vector in the cell are not critical and any method can be used to provide sufficient expression of the rhAC polypeptide in the cell.

In some embodiments, the cell is a mammalian cell. In some embodiments, the cell is not a human cell. In some embodiments, the cell is a hamster cell. In some embodiments, the cell is a Chinese hamster ovarian (CHO) cell. In some embodiments, the cell can be grown in a serum-free or substantially free of serum environment. In some embodiments, the cell is derived from a CHO-K1 cell. In some embodiments, the cell is a murine cell. In some embodiments, the cell is a murine myeloma cell. In some embodiments, the cell is a NS0 cell. In some embodiments, the effective amount that is administered is as described herein, above and below.

In some embodiments, the pharmaceutical composition is administered as described herein. For example, in some embodiments, the composition is administered to a subject orally, by inhalation, by intranasal instillation, topically, transdermally, parenterally, subcutaneously, intravenous injection, intra-arterial injection, intramuscular injection, intraplurally, intraperitoneally, intrathecally, or by application to a mucous membrane.

As used herein, the term "rhAC" or "AC" refers to recombinant human acid ceramidase encoded by the *ASAH*1 gene (NCBI UniGene GeneID No. 427). AC hydrolyzes the amide bond linking the sphingosine and fatty acid moieties of the lipid ceramide (Park, J.-H., Schuchman E. H., 2006, "Acid ceramidase and human disease," Biochim. Biophys. Acta. 1758(12): 2133-2138; Nikolova-Karakashian et al., 2000, "Ceramidases," Methods Enzymol. 311:194-201 (2000); Hassler et al., 1993, "Ceramidase: Enzymology and metabolic roles," Adv. Lipid Res. 26:49-57). Mutations of both *ASAH1* alleles can lead to Farber's disease.

There are three types of ceramidases described to date (Nikolova-Karakashian, 2000). These are classified as acid, neutral, and alkaline ceramidases according to their pH optimum of enzymatic activity. ACs have optimal enzymatic activity at a pH<5. The human AC was the first ceramidase to be cloned (Koch et al., 1996, "Molecular Cloning and Characterization of a Full-length Complementary DNA Encoding Human Acid Ceramidase: Identification Of The First Molecular Lesion Causing Farber Disease," J. Biol. Chem. 271:33110-33115). It is localized in the lysosome and is mainly responsible for the catabolism of ceramide. Dysfunction of this enzyme because of a genetic defect leads to a sphingolipidosis disease called Lipogranulomatosis or Farber disease (Koch et al., 1996, "Molecular Cloning and Characterization of a Full-length Complementary DNA Encoding Human Acid Ceramidase: Identification Of The First Molecular Lesion Causing Farber Disease," J. Biol. Chem. 271:33110-33115, Young et al., 2013, "Sphingolipids: regulators of crosstalk between apotosis and autophagy," .J. Lipid. Res. 54:5-19*).*

AC (N-acylsphingosine deacylase, I.U.B.M.B. Enzyme No. EC 3.5.1.23) protein has been purified from several sources, and the human and mouse cDNAs and genes have been obtained. See Bernardo et al., J. Biol. Chem. 270:11098-102 (1995); Koch et al., J. Biol. Chem. 2711:33110-5 (1996); Li et al., Genomics 50:267-74 (1998); Li et al., Genomics 62:223-31 (1999). It is produced through cleavage of the AC precursor protein (see Ferlinz et al., J. Biol. Chem. 276(38):35352-60 (2001)), which is the product of the ASAH1 gene (NCBI UniGene GeneID No. 427). AC protein [Homo sapiens] (NCBI Accession No. AAC50907) is shown in SEQ ID NO: 1.

The AC alpha subunit begins at the amino acid at position 22 and continues through position 142 (as shown in bold in SEQ ID NO: 1 in the Table of Sequences), while the beta subunit of the AC begins with the amino acid at position 143 and continues through position 395 (as shown in italics in SEQ ID NO: 1).

As used herein, "active acid ceramidase," "active AC" or "AC in activated form," or like phrases, refers to AC precursor proteins that have undergone autoproteolytic cleavage into the active form (composed of α- and β-subunits). The mechanism of human AC cleavage and activation is reported in (Shtraizent, N., E. Eliyahu, J.H. Park, X. He, R. Shalgi and E.H. Schuchman, 2008, "Autoproteolytic cleavage and activation of human acid ceramidase," J Biol Chem, 283(17):11253-11259). Activation is also promoted by the intracellular environment, and, based on highly conserved sequences at the cleavage site of ceramidase precursor proteins across species, is expected to occur in most, if not all, cell types.

As used herein, "inactive acid ceramidase," "inactive AC," or "inactive acid ceramidase precursor," "inactive AC precursor," or (AC preprotein) refers to AC precursor protein that has not undergone autoproteolytic cleavage into the active form. Inactive AC precursors and active ACs suitable for use in the recombinant acid ceramidase of this and all aspects of the present invention can be homologous (i.e., derived from the same species) or heterologous (i.e., derived from a different species) to the tissue, cells, and/or subject being treated. Acid ceramidase (e.g., AC) precursor proteins undergo autoproteolytic cleavage into the active form (composed of α- and β-subunits). The mechanism of human AC cleavage and activation is reported in (Shtraizent, 2008). This is promoted by the intracellular environment, and, based on highly conserved sequences at the cleavage site of ceramidase precursor proteins across species, is expected to occur in most, if not all, cell types. Thus, ceramidase as used herein includes both active ceramidases and ceramidase precursor proteins, where ceramidase precursor proteins are converted into active ceramidase proteins through autoproteolytic cleavage. Embodiments in which the precursor protein is taken up by the cell of interest and converted into active ceramidase thereby, as well as embodiments in which the precursor protein is converted into active ceramidase by a different cell or agent (present, for example, in a culture medium), are both contemplated.

Active ACs and inactive AC precursor proteins that can be used in this and all aspects of the present invention include, without limitation, those set forth in Table 1 of US 2016/0038574, the contents of which are hereby incorporated by reference.in their entirety.

### Table 1 of US 2016/0038574 (herein incorporated in its entirety by reference)

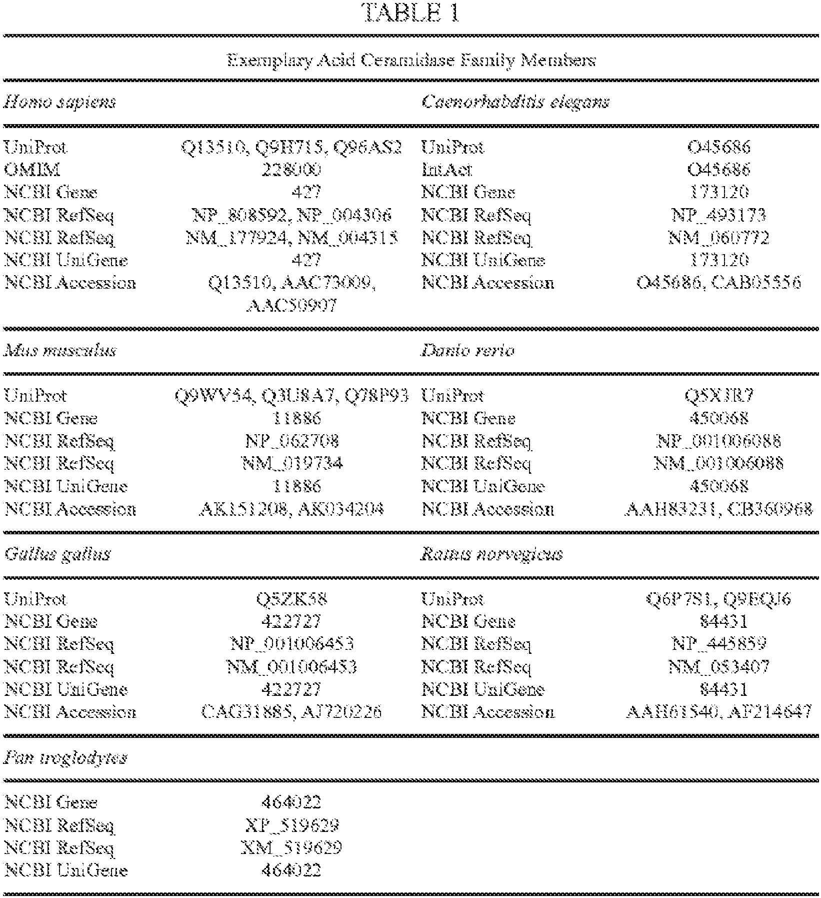

In some embodiments, the rhAC comprises an amino acid sequence of SEQ ID NO: 1.

"RVT-801" is a recombinant human acid ceramidase (rhAC) in activated form for the treatment of Farber disease. The alpha and beta subunits of the activated rhAC are joined by a disulfide bond. The molecule is a recombinant human acid ceramidase (rhAC) derived from CHO-M cells transfected with a DNA plasmid vector expressing rhAC. RVT-801 is based on UniProtKB Code: Q13510.

The therapeutic effect of RVT-801rhAC has been was established in a murine model of severe Farber disease (He, et al, 2017) and has been characterized over multiple studies with endpoints describing positive impacts on histopathological and immunological outcomes along with concomitant reduction of accumulated ceramides.

In some embodiments, the rhAC is a protein that is a homolog of SEQ ID NO: 1. In some embodiments, the rhAC is encoded by a nucleic acid molecule of SEQ ID NO: 2. In some embodiments, the rhAC is encoded by a nucleic acid molecule of SEQ ID NO: 3. In some embodiments, the rhAC is encoded by a nucleic acid molecule of SEQ ID NO: 4. In some embodiments, the sequence is as defined in GenBank accession number NM_177924.3 or NM_177924.4, each of which is incorporated by reference in its entirety. The nucleotide sequence encoding the protein can be the complete sequence shown in SEQ ID NO: 2, SEQ ID NO: 3, or SEQ ID NO: 4, or be simply the coding region of the sequence The coding region, for example, could be nucleotides 313 to 1500 of SEQ ID NO: 2 or the corresponding coding region found in SEQ ID NO: 3 or SEQ ID NO: 4. However, as is well known to one of skill in the art, the genetic code is degenerate and, therefore other codons can be used to encode the same protein without being outside of what is disclosed. Since the amino acid sequence is known any nucleotide sequence that encodes the amino acid sequence is acceptable. In some embodiments, the nucleotide sequence comprises a signal peptide. In some embodiments, the signal peptide is an amino acid sequence encoded by nucleotides 313 to 375 of SEQ ID NO: 2. In some embodiments, the protein that is produced comprises a signal peptide of amino acid residues 1-21 of SEQ ID NO: 1. In some embodiments, the protein that is produced does not comprises a signal peptide, such as the signal peptide of amino acid residues 1-21 of SEQ ID NO: 1. In some embodiments, the signal peptide is removed during a post-translational processing where the enzyme is processed into its different subunits. In some embodiments, the nucleotide sequence is codon optimized for the cell that it the protein is being expressed from. In some embodiments, the protein comprises an alpha-subunit, a beta-subunit, and the like. In some embodiments, the protein that is produced comprises a peptide of amino acid residues 22-142, 45-139, 134-379, 143-395, or 1-395 of SEQ ID NO: 1. The peptide can be a single protein or a polypeptide of different sequences to form the enzyme. In some embodiments, the protein is free of amino acid residues 1-21. These regions can be encoded by a single nucleotide sequence or separate nucleotide sequences or a combination of nucleotide sequences. As discussed herein, any nucleotide sequence encoding the protein can be used and is not limited to the sequence described herein as SEQ ID NO: 2, SEQ ID NO: 3, or SEQ ID NO: 4.

In some embodiments, the rhAC has acid ceramidase (AC) activity but does not have any detectable acid sphingomyelinase activity, such as the rhAC produced in Examples 1 and 2 below. The acid sphingomyelinase activity may be removed, for example, by heat inactivation. Heat inactivation may also remove other contaminating proteins from an rhAC preparation. See, e.g., U.S. Patent Application Publication No. 20160038574, which is incorporated herein in its entirety.

The term "homolog" refers to protein sequences having between 80% and 100% sequence identity to a reference sequence. Percent identity between two peptide chains can be determined by pair wise alignment using the default settings of the AlignX module of Vector NTI v.9.0.0 (Invitrogen Corp., Carslbad, Calif.). In some embodiments, the homolog has at least, or about, 80, 85, 86, 87, 88, 89, 90, 91, 92, 93, 94, 95, 96, 97, 98, or 99% identity to a sequence described herein, such as SEQ ID NO: 1.

In some embodiments, the protein delivered to the subject conservative substitutions as compared to a sequence described herein. Non-limiting exemplary conservative substitutions are shown in Table 3 are encompassed within the scope of the disclosed subject matter. Substitutions may also be made to improve function of the enzyme, for example stability or enzyme activity. Conservative substitutions will produce molecules having functional and chemical characteristics similar to those molecules into which such modifications are made. Exemplary amino acid substitutions are shown in Table 3 below.

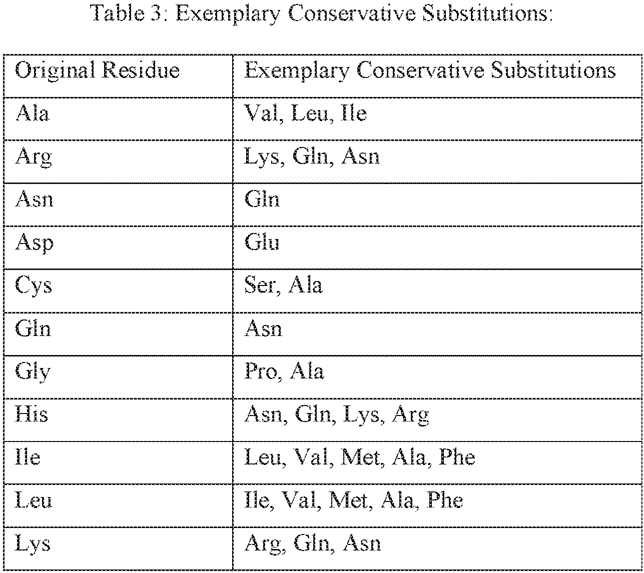

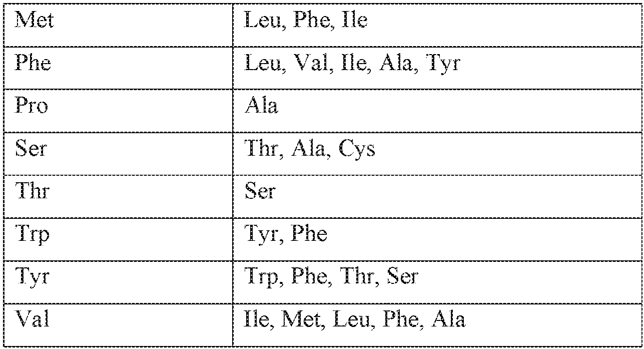

The term "in combination with" as used herein means that the described agents can be administered to a subject together in a mixture, concurrently as single agents or sequentially as single agents in any order.

As described herein, in some embodiments, the protein is produced from a cell. In some embodiments, the cell is a Chinese Hamster Ovarian cell, "CHO cell." A nucleic acid sequence encoding the proteins described herein can be genomic DNA or cDNA, or RNA (e.g. mRNA) which encodes at least one of proteins described herein. The use of cDNA requires that gene expression elements appropriate for the host cell be combined with the gene in order to achieve synthesis of the desired protein. The use of cDNA sequences can advantageous over genomic sequences (which contain introns), in that cDNA sequences can be expressed in bacteria or other hosts which lack appropriate RNA splicing systems. One of skill in the art can determine the best system for expressing the protein.

In some embodiments, the protein is produced according to U.S. Patent Application Publication No. 20160038574, which is incorporated by reference in its entirety.

Because the genetic code is degenerate, more than one codon can be used to encode a particular amino acid. Using the genetic code, one or more different oligonucleotides can be identified, each of which would be capable of encoding the amino acid sequences described herein.

The enzyme that is administered to the subject to treat Farber disease or a condition associate therewith can be purified. The term "purified" with referenced to a protein refers to a protein that is substantially free of other material that associates with the molecule in its natural environment. For instance, a purified protein is substantially free of the cellular material or other proteins from the cell or tissue from which it is derived. The term refers to preparations where the isolated protein is sufficiently pure to be analyzed, or at least 70% to 80% (w/w) pure, at least 80%-90% (w/w) pure, 90-95% pure; and, at least 95%, 96%, 97%, 98%, 99%, or 100% (w/w) pure. In some embodiments, the protein is purified from a cell, such as but not limited to a CHO cell.

### Administration, Compositions, and Kits Comprising the Proteins

As described herein, embodiments provided herein provide methods of treating Farber disease. In some embodiments, the methods comprise administering a therapeutically or prophylactically effective amount of one or more proteins described herein to a subject with Farber disease or suspected of having Farber disease.

Treatment of subjects may comprise the administration of a therapeutically effective amount of the proteins described herein.

The proteins can be provided in a kit as described herein.

The proteins can be used or administered alone or in admixture with an additional therapeutic. Examples of additional therapeutics include, but are not limited to, inhibitors of acid sphingomyelinase (e.g., amitriptyline (Becker et al., 2010, "Acid Sphingomyelinase Inhibitors Normalize Pulmonary Ceramide and Inflammation in Cystic Fibrosis," Am. J. Respir. Cell. Mol. Biol., 42:716-724, which is hereby incorporated by reference in its entirety) and inhibitors of ceramide synthases (e.g., Shiffmann, S., Hartmann, D., Birod, K., Ferreiròs, N., Schreiber, Y., Zivkovic, A., Geisslinger, G., Grösch, S., and Stark, H., 2012, "Inhibitors of Specific Ceramide Synthases," Biochimie, 94(2):558-565, which is hereby incorporated by reference in its entirety)). The additional therapeutic can also be ceramidase mixtures described in U.S. Patent Application Publication No. 20160038574, which is hereby incorporated by reference in its entirety.

While enzyme replacement therapies (ERTs) can be effective, as shown in our current study for Farber disease where reduction of ceramide accumulation was demonstrated, antibodies can develop against the drug, i.e., the replacement enzyme that may reduce its efficacy. Here, we have shown that repeat dosages are well tolerated, which supports a treatment regimen of repeated administration of the replacement enzyme resulting in reduction of the symptoms of the disease, particularly the enzyme that is produced according to the methods described herein and, e.g., in U.S. Patent Application Publication No. 20160038574.

In some embodiments, methods of treating Farber disease in a subject in need thereof comprise administering to the subject a pharmaceutical composition comprising a recombinant human acid ceramidase in an effective amount about once a week, once every 2, 3, or 4 weeks, or once a month, for about 10, about 20, or about 30 weeks, 1, 5, 10, or 25 years, or the duration of a patient's life.

Suitable vehicles and their formulation and packaging are described, for example, in Remington: The Science and Practice of Pharmacy (21st ed., Troy, D. ed., Lippincott Williams & Wilkins, Baltimore, Md. (2005) Chapters 40 and 41). Additional pharmaceutical methods may be employed to control the duration of action. Controlled release preparations may be achieved through the use of polymers to complex or absorb the compounds. Another possible method to control the duration of action by controlled release preparations is to incorporate the compounds of into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, interfacial polymerization, for example, hydroxymethylcellulose or gelatin-microcapsules and poly(methylmethacylate)-microcapsules, respectively, or in colloidal drug delivery systems, for example, liposomes, albumin microspheres, microemulsions, nanoparticles, and nanocapsules or in macroemulsions.

In general, if administering a systemic dose of the protein, it is desirable to provide the recipient with a dosage of protein which is in the range of from about 1 ng/kg-100 ng/kg, 100 ng/kg-500 ng/kg, 500 ng/kg-1 µg/kg, 1 µg /kg-100 µg /kg, 100 µg/kg-500 µg /kg, 500 µg /kg-1 mg/kg, 1 mg/kg-50 mg/kg, 50 mg/kg-100 mg/kg, 100 mg/kg-500 mg/kg (body weight of recipient), although a lower or higher dosage may be administered.

In some embodiments, the human dose of rhAC in adults is about 0.8 mg/kg. In some embodiments, the human dose of rhAC in children is about ~1.2 mg/kg.

In some embodiments, the effective amount of rhAC that is administered is about 0.1 mg/kg to about 10 mg/kg. In some embodiments, the effective amount is about 1 mg/kg to about 5 mg/kg. In some embodiments, the effective amount is about 10 mg/kg to about 50 mg/kg. In some embodiments, the effective amount is about 10 mg/kg to about 20 mg/kg. In some embodiments, the effective amount is about 20 mg/kg to about 30 mg/kg. In some embodiments, the effective amount is about 30 mg/kg to about 40 mg/kg. In some embodiments, the effective amount is about 40 mg/kg to about 50 mg/kg. In some embodiments, the effective amount is about 1, 2, 3, 4, 5, 6, 7, 8, 9, or 10 mg/kg.

The dosage can be administered once a day, twice a day, three times a day, four times a day, once a week, twice a week, once every two weeks, or once a month. In some embodiments, the dose is administered once a week. In some embodiments, the dose is administered once every two weeks. The treatment may also be given in a single dose schedule, or a multiple dose schedule in which a primary course of treatment may be with 1-10 separate doses, followed by other doses given at subsequent time intervals required to maintain and or reinforce the response, for example, once a week for 1-4 months for a second dose, and if needed, a subsequent dose(s) after several months. Examples of suitable treatment schedules include: (i) 0, 1 month and 6 months, (ii) 0, 7 days and 1 month, (iii) 0 and 1 month, (iv) 0 and 6 months, or other schedules sufficient to elicit the desired responses expected to reduce disease symptoms, or reduce severity of disease. Other treatment schedules, such as, but not limited to, those described above, can also be used.

In certain aspects of the disclosure, the treatment is started when the subject is newborn, under 1, 2, 3, 4, 5, 6, 7, 8, 9, or 10 years of age, or between 1 and 2, 3, 4, 5, 6, 7, 8, 9, 10, 25, 50, 60, 70, or 80 years of age (e.g., between 1 and 2, between 1 and 3, etc.). In some embodiments, the subject is between 16 and 61. In some embodiments, the subject starts treatment at age 16. In some embodiments, the subject is between 12 and 69. In some embodiments, the subject starts treatment at age 12. In some embodiments, the subject is between 19 and 74. In some embodiments, the subject starts treatment at age 19. In some embodiments, the subject is between 4 and 62. In some embodiments, the subject starts treatment at age 4. In some embodiments, the subject is between 7 and 42. In some embodiments, the subject starts treatment at age 7. In some embodiments, the subject is newborn. In some embodiments, the subject is between 1 and 6 months. In some embodiments, the subject starts treatment at age 1 month, 2 months, 3 months, 4 months, 5 months, or 6 months. In some embodiments, the subject is between 6 and 43. In some embodiments, the subject starts treatment at age 6. In some embodiments, the subject is between 5 and 31. In some embodiments, the subject starts treatment at age 5. In some embodiments, the subject is between 5 and 57. In some embodiments, the subject is between 5 and 29. In some embodiments, the subject is between 1 and 3. In some embodiments, the subject starts treatment at age 1. In some embodiments, the subject is between 10 and 70. In some embodiments, the subject starts treatment at age 10. In some embodiments, the subject is between 5 and 80, between 10 and 70, between 20 and 75, between 5 and 60, or between 5 and 30 years of age.

In some embodiments, a subject diagnosed with Farber disease is administered rhAC at about 1 mg/kg to about 5 mg/kg rhAC or about 1 mg/kg to about 5 mg/kg rhAC every two weeks. In one embodiment, the dosage escalates from 1 mg/kg or 2 mg/kg to 5 mg/kg at week 4. If a dose level is not tolerated by an individual subject, the dose for that subject may be reduced from 2 mg/kg to 1 mg/kg, or 5 mg/kg to 2 mg/kg, as appropriate. The rhAC may be administered every 2 weeks for at least 10, 20, or 30 weeks or for the duration of the subject's life. In one embodiment, a subject is diagnosed with Farber disease and is identified as having: 1) subcutaneous nodules; and/or 2) an acid ceramidase activity value in white blood cells, cultured skin fibroblasts or other biological sources (e.g., plasma) that is less than 30% of control values; and/or 3) nucleotide changes within both alleles of the acid ceramidase gene (ASAHT1) that indicate, through bioinformatic, gene expression studies, and/or other methods, a possible loss of function of the acid ceramidase protein. In some embodiments, the subject is administered rhAC every two weeks for 28 weeks. In some embodiments, the delivery of rhAC is by intravenous infusion (e.g., saline infusion). In some embodiments, starting at about 1 mg/kg and escalating to about 5 mg/kg rhAC (e.g., to 5 mg/kg at week 4).

In some embodiments, the intravenous infusion may be 3 mL/hr. over time (generally 3.5 to 4 hours). In some embodiments, the dose is infused at a rate 3% of the total volume in the first hour and the remainder of the dose may be infused over the following 2.5 hours (*i.e.,* infusion in the second hour may be approximately 40% of the nominal volume per hour until the dose is administered). No maximum time of infusion is established in order to ensure that individual tolerability may be addressed on a case-by-case basis. The infusion is administered through an in-line, low-protein binding, 0.2-micron filter. The dose is normalized to weight in kg which will be determined at each dosing interval, based on the weight collected at the previous study visit.

For example, site specific administration may be to body compartment or cavity such as intraarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, vaginal, rectal, oral, , buccal, sublingual, intranasal, or transdermal means.

The therapeutic compositions described herein can be prepared for use for parenteral (subcutaneous, intramuscular or intravenous) or any other administration particularly in the form of liquid solutions or suspensions. The formulation can also be suitable for an injectable formulation. In some embodiments, the injectable formulation is sterile. In some embodiments, the injectable formulation is pyrogen free. In some embodiments, the formulation is free of other antibodies that bind to other antigens other than an antigen described herein.

A protein of rhAC capable of treating Farber disease or other condition associated with rhAC activity or use to treat a rhAC related pathology, is intended to be provided to subjects in an amount sufficient to affect a reduction, resolution, or amelioration in the related symptom or pathology. Such a pathology includes the symptoms of Farber disease as described herein in a subject. An amount is said to be sufficient or a "therapeutically effective amount" to "affect" the reduction of symptoms if the dosage, route of administration, and dosing schedule of the agent are sufficient to influence such a response. Responses to the protein can be measured by analysis of subject's affected tissues, organs, or cells as by imaging techniques or by ex vivo analysis of tissue samples. An agent is physiologically significant if its presence results in a detectable change in the physiology of a recipient patient. In some embodiments, an amount is a therapeutically effective amount if it is an amount that can be used to treat, ameliorate, or inhibit symptoms of Farber disease that a subject is subject to. Non-limiting examples of such amounts are provided herein, but are not intended to be limited to such amount if context dictates another amount.

In some embodiments, efficacy of treatment is assessed by any of the following means:
- Percent change from baseline in net nodule (≥5 mm) count after treatment with rhAC for 28 weeks;
- Percent change from baseline in net nodule (≥10 mm) count and comparison to placebo after treatment with rhAC for 28 weeks;
- Percent change from baseline in total nodule count (regardless of size) and comparison to placebo after treatment with rhAC for 28 weeks;
- Change and percent change from baseline of joint range of motion in selected joints and comparison to placebo after treatment with rhAC for 28 weeks;
- Change and percent change from baseline of 6-minute walk distance and comparison to placebo after treatment with rhAC for 28 weeks;
- Change and percent change from baseline of pulmonary function tests and comparison to placebo after treatment with rhAC for 28 weeks;
- Change and percent change from baseline of FDT score and comparison to placebo after treatment with rhAC for 28 weeks;
- Change and percent change from baseline in Z-score of body weight and height for age during treatment with rhAC or placebo over 28 weeks.

In some embodiments, pharmacokinetics of RVT-801 following administration to Farber mice or healthy mice at different doses is assessed based on noncompartmental methods. Noncompartmental pharmacokinetics methods estimate the exposure to a drug by estimating the area under the curve of a concentration-time graph, among others, with the follow metrics know in the art:

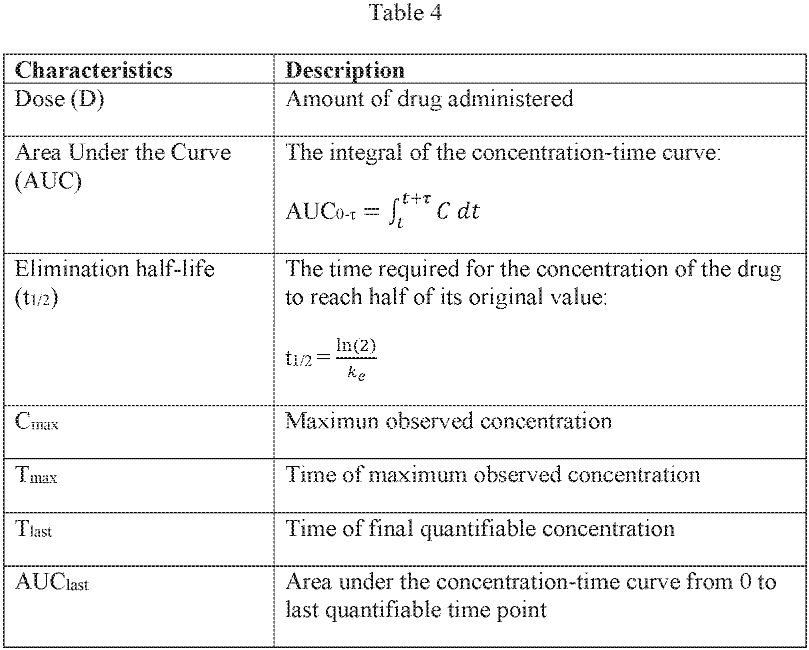

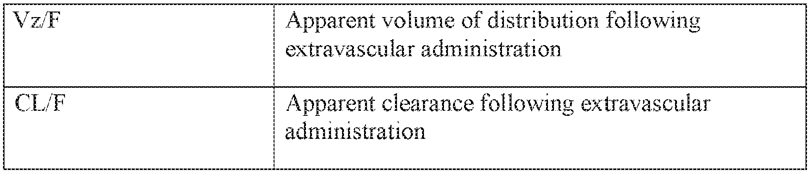

In some embodiments, tissue-specific efficacy of treatment is assessed by determining tissue-specific pharmacokinetics of RVT-801 based on the above described noncompartmental pharmacokinetics methods.

In some embodiments, Human Equivalent Dose (HED) of RVT-801 corresponding to effective dose for Farber disease mice is estimated. Nonclinical assessments of HED herein have been based on two methods: 1) FDA guidance for scaling between nonclinical species and humans by body surface area (BSA), and 2) organ:bodyweight ratios between species for liver and spleen as the major tissues for ceramide accumulation and in which uptake of RVT-801 predominated.

Scaling by body surface area: The HED, benchmarking against the Farber mouse MED (maximally effective dose) and based on BSA and bodyweight for a human adult or child (where HED = animal dose * (animal body weight/hunian bodyweight)^{0.33} indicates a dose of ~0.81 mg/kg for a 60 kg adult and ~1.2 mg/kg for a 15 kg child.

Scaling by organ : body weight ratios: PK studies demonstrated the mechanism for clearance from the vasculature is associated with uptake and/or distribution into tissues, thus the tissue weight-to-bodyweight ratio may impact dose and the BSA model may not be sufficiently predictive on its own. Using HED = MED * (Tissueₕᵤₘₐₙ/BWₕᵤₘₐₙ)/(Tissueₘₒᵤₛₑ/BWₘₒᵤₛₑ) a 10 mg/kg dose in mouse is equivalent to a ~3-5 mg/kg dose in an adult human or a ~4-5 mg/kg dose in a 15 kg child based on liver and spleen in human adults and children.

In some embodiments, HED may be determined by combining the two scaling approaches above.

The proteins can be formulated according to known methods to prepare pharmaceutically useful compositions, whereby these materials, or their functional derivatives, are combined in admixture with a pharmaceutically acceptable carrier vehicle.

Kits, which are described herein and below, are also provided which are useful for carrying out embodiments described herein. In some embodiments, the kits comprise a first container containing or packaged in association with the above-described polypeptides. The kit may also comprise another container containing or packaged in association solutions necessary or convenient for carrying out the embodiments. The containers can be made of glass, plastic, or foil and can be a vial, bottle, pouch, tube, bag, etc. The kit may also contain written information, such as procedures for carrying out the embodiments or analytical information, such as the amount of reagent contained in the first container means. The container may be in another container apparatus, e.g. a box or a bag, along with the written information.

Yet another aspect provided for herein is a kit for treating Farber disease. In some embodiments, the kit comprises at least one container comprising a rhAC polypeptide or a nucleic acid molecule encoding the same. In some embodiments, the kit comprises a container comprising a cell that is configured to express rhAC. In some embodiments, the cell is a CHO cell. In some embodiments, the kit comprises conditioned media from a cell that expresses rhAC. In some embodiments, the conditioned media is from a CHO cell.

The subject matter is now described with reference to the following examples. These examples are provided for the purpose of illustration only and the claims should in no way be construed as being limited to these examples, but rather should be construed to encompass any and all variations which become evident as a result of the teaching provided herein. Those of skill in the art will readily recognize a variety of non-critical parameters that could be changed or modified to yield essentially similar results.

### EXAMPLES

The present Examples provide the disposition of rhAC in key tissue compartments following administration across a range of efficacious doses to allow subsequent pharmacokinetics and tissue distribution modeling, and, based on the efficacious dose and tissue exposures, further provide prediction of a human equivalent dose (HED), scaled by body surface area or organ:body weight ratios.

### EXAMPLE 1

The serum pharmacokinetic profile of rhAC (RVT-801) following administration of RVT-801 by intraperitoneal (i.p.) injection at a range of efficacious doses were assessed in healthy, juvenile, wild-type CD-1 mice (parental strain of Asah1^{P361R/P361R} mice, (*See,* He et al., 2017, which is incorporated herein by reference in its entirety). Production and characterization of rhAC (RVT-801) were conducted according to PCT/2018/052463 (not yet published), filed on September 24, 2018, which is incorporated herein by reference in its entirety.

### In-life protocol

Male CD-1 mice, aged approximately 3.5 weeks (juvenile), were administered RVT-801 as a single bolus IP injection at either 1, 3, or 10 mg/kg. Blood samples for pharmacokinetic analysis were collected from three (3) animals per time point (pre-dose, 0.5, 1, 2, 3, 4, 6 post-injection) in each dose group by cardiac puncture or other approved means to generate the maximum volume blood sample from each mouse. Each blood sample was processed in its entirety to serum immediately for RVT-801 concentration analysis by ELISA.

### RVT-801 Bioanalysis by ELISA

Sandwich enzyme-linked immunosorbent assay (ELISA) have been qualified for the quantification of RVT-801 in mouse serum. The method uses an affinity-purified rabbit anti-recombinant human AC (rhAC, RVT-801) polyclonal antibody for capture, and an affinity-purified horseradish peroxidase (HRP)-conjugated rabbit anti-rhAC polyclonal antibody for detection. RVT-801 is used to establish the standard curve and control ranges. The signal generated is proportional to the concentration of RVT-801 in the sample within the defined range of the assay.

The detailed 2-day assay procedure is as follows: On day 1, a 96-well clear polystyrene plate was coated with 100 µL of Capture Reagent (1 µg/mL rabbit anti-rhAC in PBS), and incubated overnight at 2-8°C. All other steps were performed on Day 2. All incubations on Day 2 took place at room temperature (RT), with shaking (~300-400 rpm). Briefly, the plate was washed 3 times with 300 µL of Wash Buffer (1X PBS containing 0.05% Tween-20, PBST) and tapped on paper towels after the final wash. Next, the plate was blocked with 300 µL of Assay Buffer (Casein/TBS containing 0.05% Tween 20) per well for at least 1 hour and washed as described above. After washing, 100 µL of calibration standards, controls, and samples, diluted to the MRD (50-fold in Assay Buffer), were added to each well, and the plate was incubated for approximately 2 hours and washed as described above. Next, 100 µL of Detection Reagent (0.2 µg/mL HRP-conjugated rabbit anti-rhAC in Assay Buffer) were added to each well, and the plate was incubated for approximately 1 hour and washed as described above. After washing, 100 µL of cold Substrate Solution were added to each well, and the plate was incubated in the dark for up to 30 minutes. Color development was monitored by measuring absorbance at 650 nm (A650). The reaction was stopped when the A650 was between 0.9 - 1.2 for the highest standard, or as appropriate based on color development. To stop color development, 100 µL of Stop Solution were added to each well, and the plate was read at 450 nm with wavelength correction set to 650 nm.

### Noncompartmental Pharmacokinetics Analysis

Noncompartmental pharmacokinetics analysis was performed by means of Certara WinNonlin Phoenix Version 7.0. Exposures ratios and graphs were generated with Microsoft Excel and GraphPad Prism.

### Results

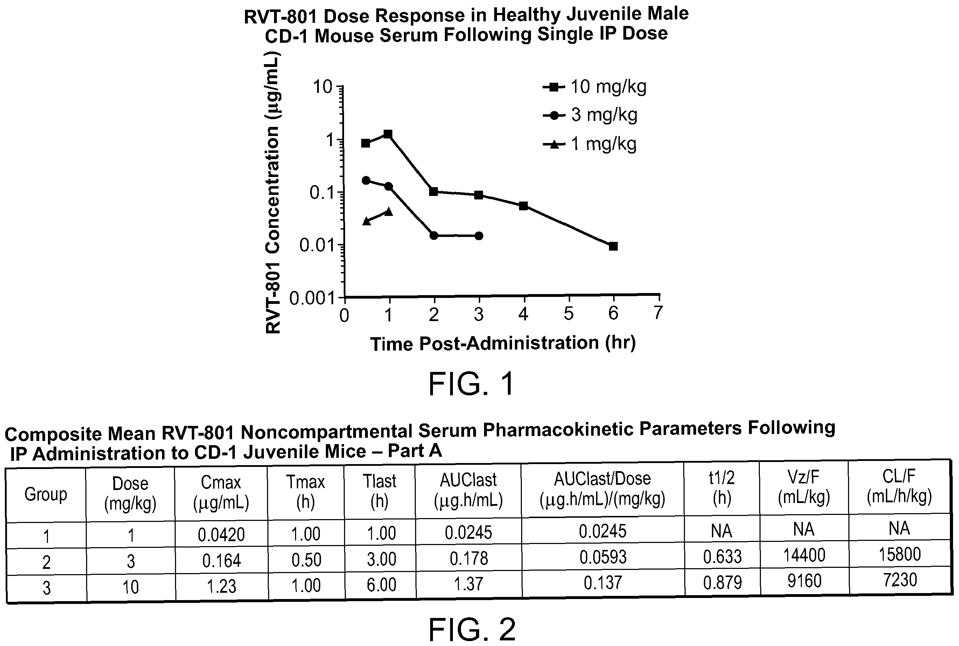

To evaluate the pharmacokinetic profiles of the enzyme *in vivo,* healthy, juvenile CD-1 mice received a single rhAC (RVT-801) injection at doses of 1, 3, and 10 mg/kg, and RVT-801 concentrations were determined in the serum (Fig. 1) at 0.5, 1, 2, 3, 4, and 6 h post-injection. As shown in Fig. 2, Serum exposure (AUC, Cₘₐₓ) increased in a greater than dose-proportional manner following single IP doses of RVT-801 at 1, 3, and 10 mg/kg.

The juvenile CD-1 mouse PK data for RVT-801 are discussed below. Two studies in juvenile CD-1 mice dosed with RVT-801 at 10 mg/kg were conducted:
RVT-801-9013A - focused on characterizing serum PK. The following PK parameters were observed.
   AUC 1.37 hr*µg/mL
   Cₘₐₓ 1.23 hr*µg/mL
   Tₘₐₓ 1.0 hr
RVT-801-9013B - focused on tissue distribution of RVT-801 and tissue: serum exposure ratios. The following PK parameters were observed.
   AUC 1.49 hr*µg/mL
   Cₘₐₓ 2.17 hr*µg/mL
   Tₘₐₓ 0.25 hr

Further, as shown in Fig. 3, duration of RVT-801 (i.p. injection, 10 mg/kg) exposure in serum of wild-type CD-1 mice correlated closely with systemic acid ceramidase (AC) activity in Farber disease mice (a solid line with black circles; reproduced from International Application No. PCT/US18/13509, the disclosure of which is incorporated by reference in its entirety). Based on this finding, further assessments of RVT-801 pharmacokinetics and tissue specific distribution were performed with wild-type CD-1 mice age-matched to juvenile Farber mice used in the efficacy studies.

### EXAMPLE 2

Further, pharmacokinetics and tissue specific distribution of RVT-801 were assessed in a variety of organ tissues of wild-type CD-1 mice.

### Tissue processing before analysis

Tissue homogenates were prepared in CHAPS buffer with the addition of a protease inhibitor cocktail by processing the samples with a Qiagen TissueLyser II (3 cycles of 3 minutes each at a frequency of 30 Hz). The tissue homogenates were diluted to a standard 1 mg/mL total protein concentration prior to ELISA detection. Due to low protein content BALF samples were processed for detection on the basis of volume rather than protein concentration.

### Quantification of RVT-801 in tissue extract by modified ELISA

ELISA method qualified for the quantification of RVT-801 mouse serum has been adapted for quantification in mouse tissues (blood, liver, spleen, kidney, heart, lung, and brain).

The modified method included preparation of tissue lysates, quantification of total protein using a bicinchoninic acid (BCA) assay, and preparation of RVT-801 standard curve in lysis buffer. The adapted method had a minimum required dilution (MRD) of 50-fold only for mouse serum quality control samples. Standards and study samples were not subjected to the MRD. The signal generated was proportional to the concentration of RVT-801 in the sample. The lower and upper limits of quantification of the adapted assay were 0.400 ng/mL and 24.5 ng/mL, respectively. Measured RVT-801 concentrations were corrected for tissue homogenization and lysate dilution, as appropriate.

Before ELISA was performed, a feasibility study was performed. The feasibility study included: 1) comparison of RVT-801 standard curves prepared in RIPA buffer (150 mM Sodium Chloride, 50 mM Tris-HCl, pH 7.5, 0.25% (w/v) Deoxycholic Acid, 1% (v/v) NP-40), CHAPS buffer (150 mM Sodium Chloride, 50 mM Tris-HCl, pH 7.5, 2% (w/v) CHAPS), and mouse serum, 2) comparison of recoveries of mouse serum control samples against RVT-801 standard curves prepared in RIPA and CHAPS buffers, 3) comparison of lysosome disruption using an acid phosphatase assay kit (Sigma CS0740) following different tissue homogenization methods (incubation on ice, 3 TissueLyser cycles at 30 Hz, 5 TissueLyser cycles at 30 Hz, and sonication) using different lysis buffers (PBS, RIPA and CHAPS), 4) confirmation of lysosome disruption using a β- galactosidase kit (Pierce 75705) following homogenization in CHAPS buffer, and 5) analysis of recoveries of tissue lysates spiked with RVT-801 at different concentration levels against a CHAPS standard curve. All solid samples, except AM pellets, were weighed in tubes prior to homogenization. An empty tube was weighed at the beginning of each batch, and this weight was used to calculate the weight of the tissue in each tube. Because mouse hepatic density was known from the literature, this value (1.051 g/mL) was used in calculating the lysis correction factor for liver tissues; however, all other tissues assumed a density of 1 g/mL (see Fig. 7). Epithelial lining fluid (ELF) concentrations may be determined by applying correction factors based on urea content to bronchoalveolar lavage fluid (BALF) data to correct for sample dilution upon lavage and collection.

### Pharmacokinetics analysis

In the same manner as Example 1, Non-compartmental PK analysis was performed by means of Certara WinNonlin Phoenix Version 7.0.

### Results

Major mechanism for clearance of RVT-801 from circulation is uptake into key tissues implicated in ceramide accumulations. As shown in Fig. 4, RVT-801 achieves extensive distribution across various organ tissues (liver, spleen, kidney, lung and heart). Exposure of RVT-801 in key tissues is markedly greater than in serum and persists for an extended period after RVT-801 becomes unmeasurable in systemic circulation. This finding may support less frequent dosing than indicated based on serum exposure. The prolonged duration of exposure in key organ tissues (liver, spleen) following acute RVT-801 treatment correlates to maximal reduction of tissue ceramides occurring beyond the duration of systemic exposure.

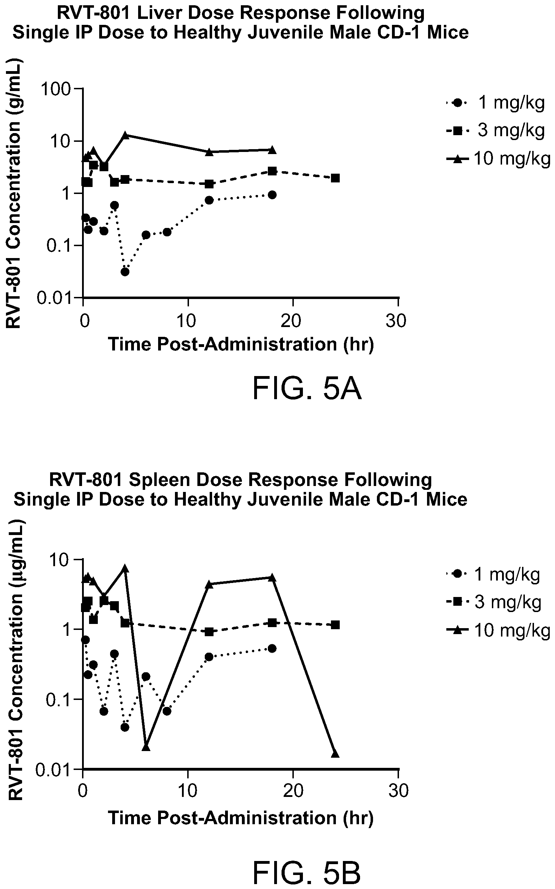

Liver and spleen are two key organs of ceramide accumulation in Farber mice and of RVT-801 uptake, as discussed above in Example 1. Figs. 5A and 5B show a time course of RVT-801 dose response in liver and spleen tissue extracts, respectively, indicating that RVT-801 exposure in tissues increased with dose (1, 3, and 10 mg/kg). The highest RVT-801 concentrations were achieved in liver at ~4 hr post dose and remained elevated beyond the last sampling time point (24 hr) where serum levels were undetectable. Variability between sampling time-points was observed likely owing to complexity of dosing juvenile mice (~16 g bodyweight).

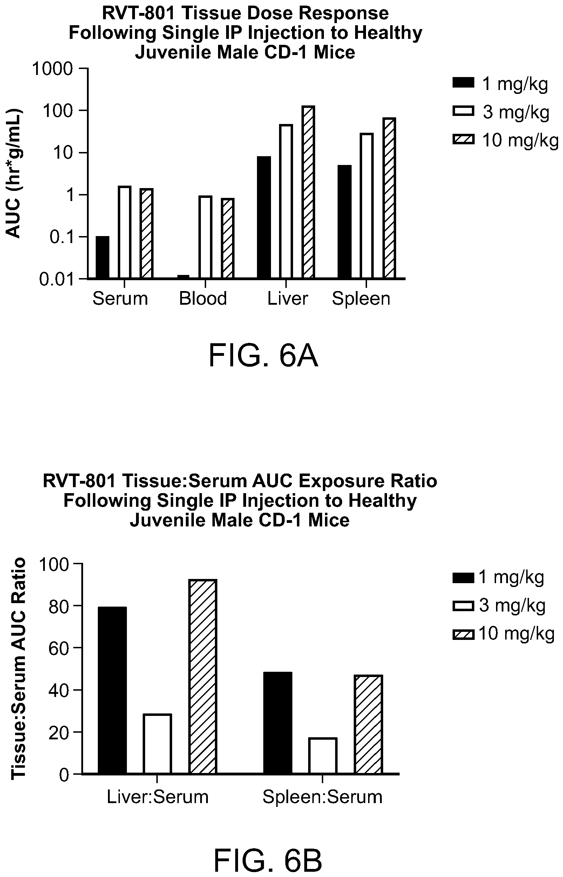

Further, pharmacokinetics of RVT-801 were assessed in these two key organs. As shown in Fig. 6A, as compared to serum and whole blood RVT-801, AUC₀₋ₗₐₛₜ increased for liver and spleen at each of efficacious doses. As shown in Fig. 6B, tissue: serum ratio (AUCₜᵢₛₛᵤₑ : AUCₛₑᵣᵤₘ) demonstrated markedly higher exposure per organ relative to systemic exposure over a range of efficacious dose. The liver:serum ratios are depicted in Fig. 6B.

Further, pharmacokinetics of RVT-801 in these two organs were assessed in comparison with those in a variety of other organs. As shown in Fig. 7, RVT-801 distributes extensively to tissues associated with ceramide accumulation in the Farber mouse as discussed above in Example 1. Across other tissues, 10 mg/kg RVT-801 achieved tissue: serum ratios as depicted in Fig. 8.

**Discussion:** These results establish the serum and tissue exposure target required for the rhAC RVT-801 to achieve maximal efficacy endpoints in lowering tissue ceramides in Farber disease mice. The markedly higher and prolonged exposure achieved in key tissues (liver, spleen) associated with ceramide accumulation supports an extended duration of ceramide reduction in tissues by RVT-801 beyond detectable levels of enzyme in serum.

### EXAMPLE 3

An equivalent, therapeutically meaningful dose in humans (HED: Human Equivalent Dose) of RVT-801was assessed by applying the above-discussed scaling strategies employing body surface area or organ tissue: body weight ratios.

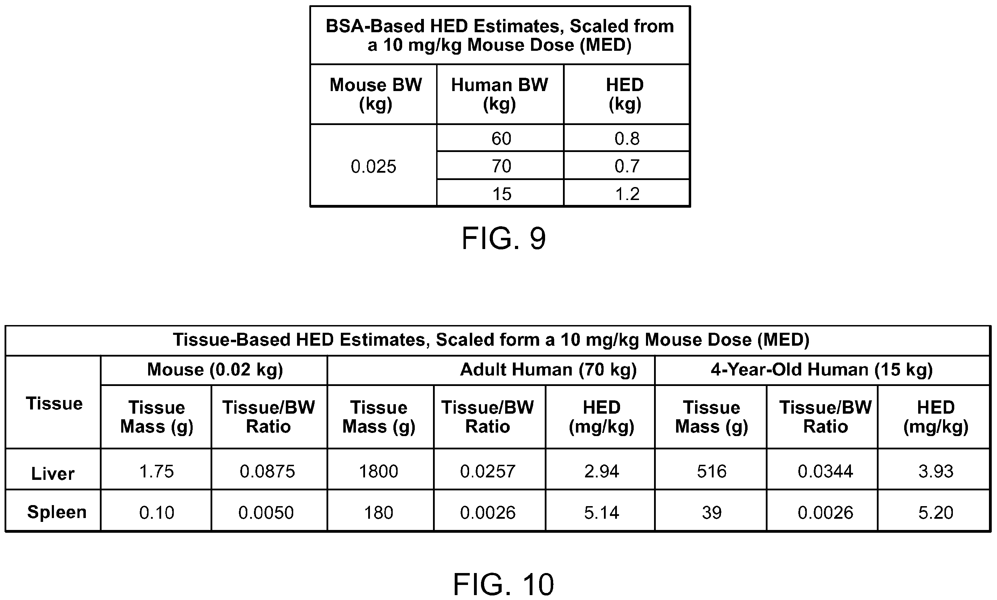

As shown in Fig. 9, the 10 mg/kg dose of RVT-801 was scaled by body surface area (BSA) to account for general physiological trends between species based on the following formula:HED = animal dose x (animal bodyweight/human bodyweight)^{0.33} (where animal dose is 10 mg/kg). Based on the 10mg/kg mouse dose, adult HED was ~ 0.8 mg/kg and child HED was ~1.2 mg/kg.

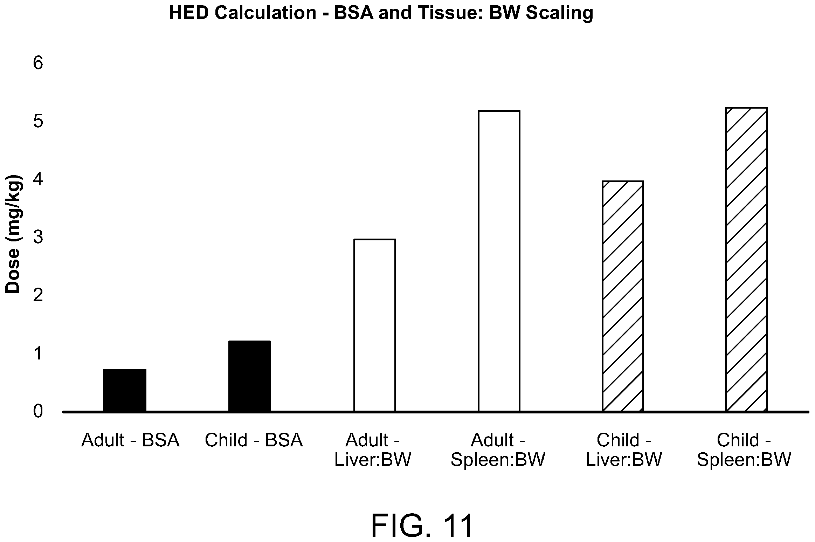

As shown in Figs. 10 and 11, the 10 mg/kg dose of RVT-801 was scaled by tissue: bodyweight ratios to account for the relative size of each compartment of RVT-801 distribution within each species, using the following formula: HED= animal dose x (Tissue Mass human/BWhuman)/(Tissue Mass mouse/BWmouse) (where animal dose is 10 mg/kg).

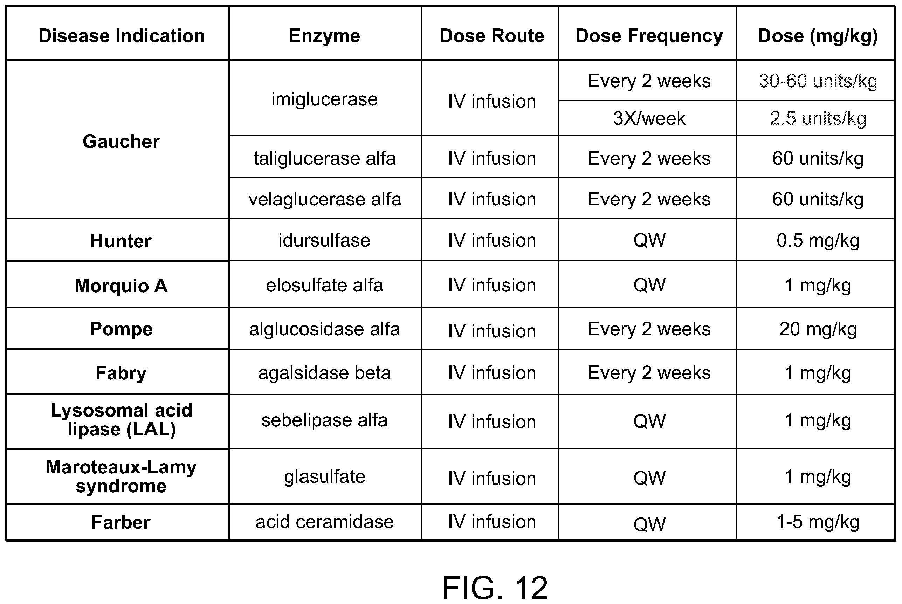

Combining the two scaling factors based on body surface area and organ to bodyweight proportionality provided a conservative human equivalent dose (HED) range of approximately 1-5 mg/kg per administration, as shown in Fig. 12. This HED range is consistent with approved dosages of other marketed enzyme replacement therapies (ERT's). (Fig. 12). All dosing information of other ERT's summarized in the table of Fig. 12 are from the products' respective labels.

### EXAMPLE 4

The objective of this study was to determine the serum and tissue pharmacokinetics of RVT-801 following a single dose treatment to juvenile male CD-1 mice of 1, 3, or 10 mg/kg, administered as a bolus IP injection.

Following IP administration to juvenile mice, mean serum RVT-801 concentrations were highest between 0.25 and 1 h following dose administration for all dose groups and declined rapidly with an estimated half-life of 1 h. Serum RVT-801 increased with increasing dose, with Cₘₐₓ and AUCₗₐₛₜ increasing greater than proportional with respect to dose. The Vz/F (apparent volume of distribution following extravascular administration) exceeded total body water and the CL/F (apparent clearance following extravascular administration) exceeded liver blood flow. There was a trend towards decreasing Vz/F and CL/F as the dose increased from 3 mg/kg to 10 mg/kg suggesting saturation in tissue distribution and clearance.

The maximum effective dose (MED) in Farber mice was 10 mg/kg delivered by bolus IP injection consistent with the current study. The value for AUCₗₐₛₜ. Cₘₐₓ and Tₘₐₓ determined following a single 10 mg/kg dose to aged-matched, wild-type CD-1 mice were as reported above:
RVT-801-9013A - focused on characterizing serum PK:
AUC 1.37 hr*µg/mL
Cₘₐₓ 1.23 hr*µg/mL
Tₘₐₓ 1.0 hr

RVT-801-9013B - focused on tissue distribution of RVT-801 and tissue: serum exposure ratios
AUC 1.49 hr*µg/mL
Cₘₐₓ 2.17 hr*µg/mL
Tₘₐₓ 0.25 hr

This study was conducted in two parts. The objective of part A of this study was to determine the serum pharmacokinetics of RVT-801 following a single dose to juvenile male CD-1 mice of 1, 3, or 10 mg/kg, administered as a bolus intraperitoneal injection. The objective of part B of the study was to characterize the disposition of RVT-801 in key tissue implicated in ceramide accumulation in the Farber mouse model following a single dose of 1, 3, or 10 mg/kg administered as a bolus intraperitoneal injection to juvenile male CD-1 mice.

RVT-801 Drug Substance Source 753-01-16-002 was used in this study. The vehicle formulation used for IP administration was sterile saline.

RVT-801 was administered via bolus intraperitoneal injection to male CD-1 mice, aged approximately 3.5 weeks. A total of 108 mice were used in part A of the study and 102 mice were used in part B of the study. NeoSome coordinated animal shipment and supply dates with their supplier (Charles River Laboratories) to allow sufficient acclimation of the pups at the vivarium prior to dosing.

Dose material was prepared by diluting the provided stock solution of RVT-801 with sterile PBS according to Table 5.

Individual doses were calculated based on body weights recorded on the day of dose administration. Animals were administered RVT-801 via bolus IP injection as detailed in Table 6.

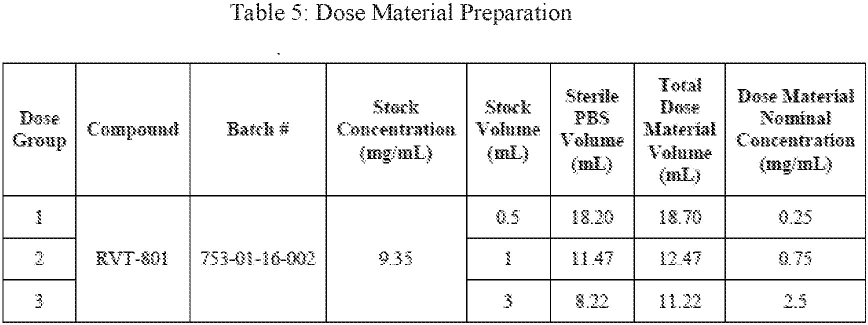

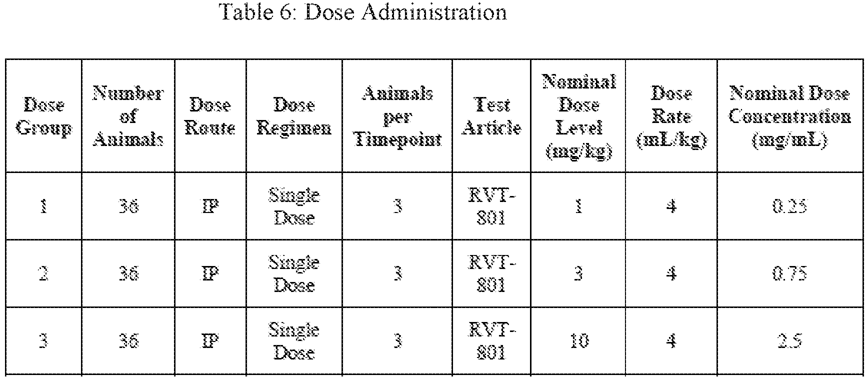

Pharmacokinetic samples were collected on the schedule detailed in Table 7, with whole blood collections from three animals per time point per dose group. The entire volume of whole blood collected from each animal was processed to serum.

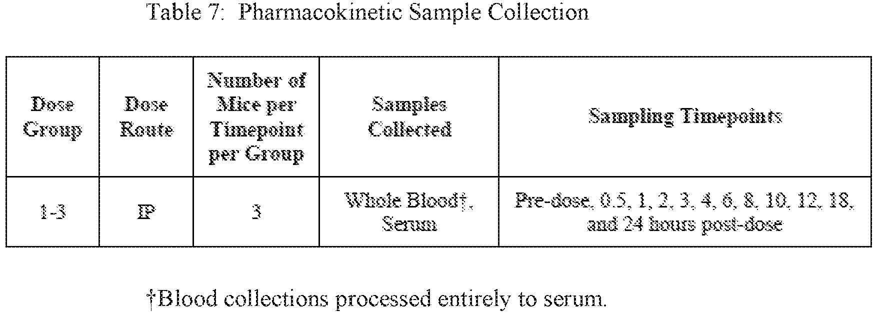

In Part B, dose material was prepared by diluting the provided stock solution of RVT-801 with sterile PBS according to Table 8.

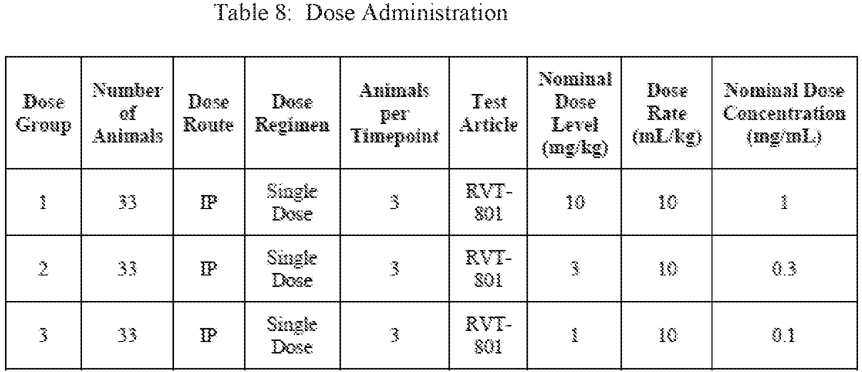

Pharmacokinetic samples were collected on the schedule detailed in Table 9.

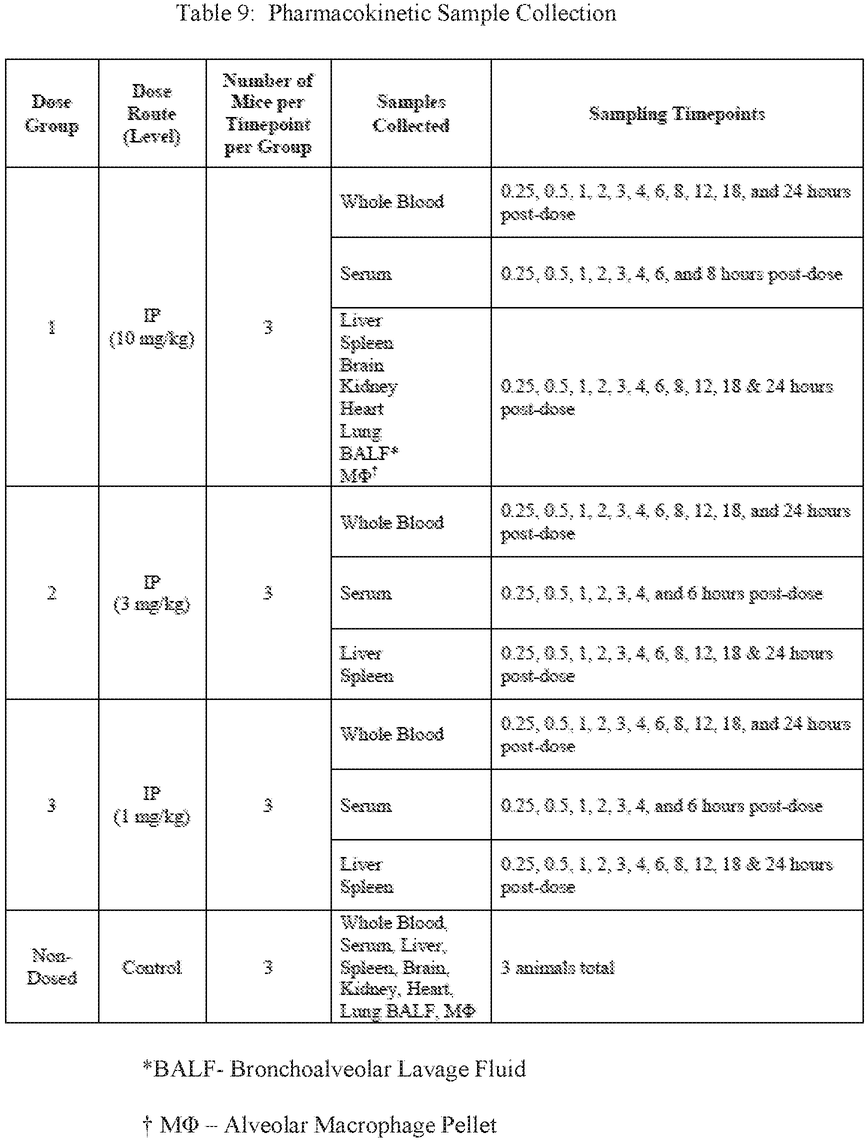

Blood collections were divided to provide whole blood and serum samples (where indicated) for each animal.

The serum PK samples were stored at approximately -70°C and were shipped on dry ice via overnight courier to BioAgilytix (the bioanalytical vendor), where determination of RVT-801 concentrations was performed by sandwich enzyme-linked immunosorbent assay (ELISA).

Liver and spleen were collected from all dose groups directly following blood collection at the appropriate time point. Tissues were gently blotted dry and placed into pre-labeled (pre-weighed) vials. The weights of each vial containing tissues was recorded, and samples were stored at -70°C until shipment. No buffers, preservative, or antibiotics were added to the tissues, and no tissue perfusions were performed at any stage of the study. For the 10 mg/kg dose group, brain, kidney, heart, and lung were collected and processed as described for liver and spleen. Additionally, bronchoalveolar lavage fluid (BALF) was collected from the 10 mg/kg dose group directly following blood collection at the appropriate time point via a pulmonary lavage technique instilling lungs with 0.5 mL phosphate buffered saline and subsequent collection of approximately 0.35 mL of BALF. BALF samples were placed in a suitable container and centrifuged to generate supernatant and an alveolar macrophage pellet. The supernatant was removed and placed into a separate vial. Supernatant and cell pellet were stored at -70°C until shipment.

Three additional mice were used to provide drug-free matrices. The control matrix samples were used as pre-dose samples for each dose group. Sample collection, processing and storage was performed as described above. Concentrations of RVT-801 in serum were determined using a qualified sandwich ELISA method (BioAgilytix BAL-17-333-036.01-REP). The LLOQ of the serum assay was 20 ng/mL, and the ULOQ of the assay was 1224 ng/mL.

The serum method was adapted for quantification in blood, liver, spleen, kidney, heart, lung, and brain. The modified method included preparation of tissue lysates, quantification of total protein using a bicinchoninic acid (BCA) assay and dilution to a total protein concentration of 1 mg/mL in lysis buffer, and preparation of RVT-801 standard curve in lysis buffer. Due to low protein content, BALF samples were processed on a volumetric basis rather than on the basis of standardized protein concentration. The adapted method had a minimum required dilution (MRD) of 50-fold only for mouse serum quality control samples. Standards and study samples were not subjected to the MRD. The signal generated was proportional to the concentration of RVT-801 in the sample. The LLOQ of the adapted assay was 0.400 ng/mL, and the ULOQ of the assay was 24.5 ng/mL. Measured RVT-801 concentrations were corrected for tissue homogenization and lysate dilution. A description of the bioanalytical method and results are provided in the final bioanalytical report.

Pharmacokinetic parameters in Table 10 were derived using noncompartmental methods employing Phoenix WinNonlin^{®} version 7.0 (Certara, Princeton, NJ) using composite serum and tissue concentration-time data. The area under the concentration-time curve from zero time (predose) to the last non-zero time point was calculated by a combination of linear and logarithmic trapezoidal methods. The linear trapezoidal method was employed for all incremental trapezoids arising from increasing concentrations and the logarithmic trapezoidal method was used for those arising from decreasing concentrations (linear up/log down method). Nominal blood sampling and tissue collection times were used in the analysis.

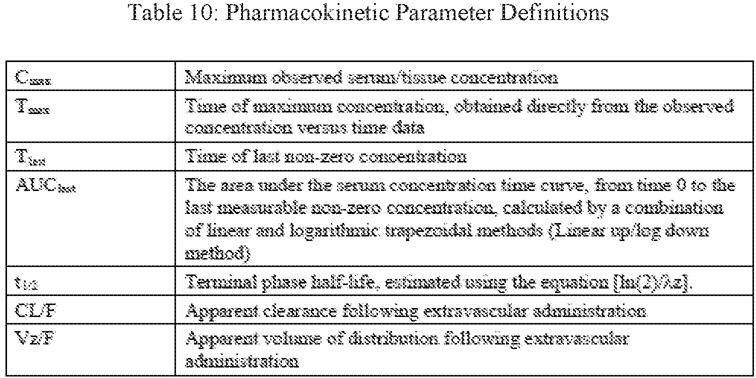

RVT-801 concentration values were received from the bioanalytical lab in units of ng/mL and were converted to units of µg/mL for PK analysis and reporting. Concentration data and PK parameters were reported to 3 significant figures except for Tₘₐₓ and Tₗₐₛₜ values that were reported to 2 decimal places.

All concentrations below the limit of quantification (BLQ) were set to zero, and the mean concentrations at each time point for each dose group was calculated for reporting of concentration-time summary statistics and mean concentration-time figures. Mean concentrations that were BLQ were used in the noncompartmental analysis without adjustment.

Any mean concentrations equal to 0 (all values BLQ) were considered BLQ and handled as follows for the NCA:

If a BLQ value occurred after a measurable concentration in a profile and was followed by a quantifiable value, it was treated as missing data.

If a BLQ value occurred at the end of the collection interval (after the last quantifiable concentration), it was treated as missing data.

If two BLQ values occurred in succession after Cₘₐₓ, the profile was deemed to have terminated at the first BLQ value and any subsequent concentrations were omitted from PK calculations.

### Results.

The individual mouse serum and tissue concentration-time data for RVT-801 used in the PK analysis of part B are presented in Table 11.

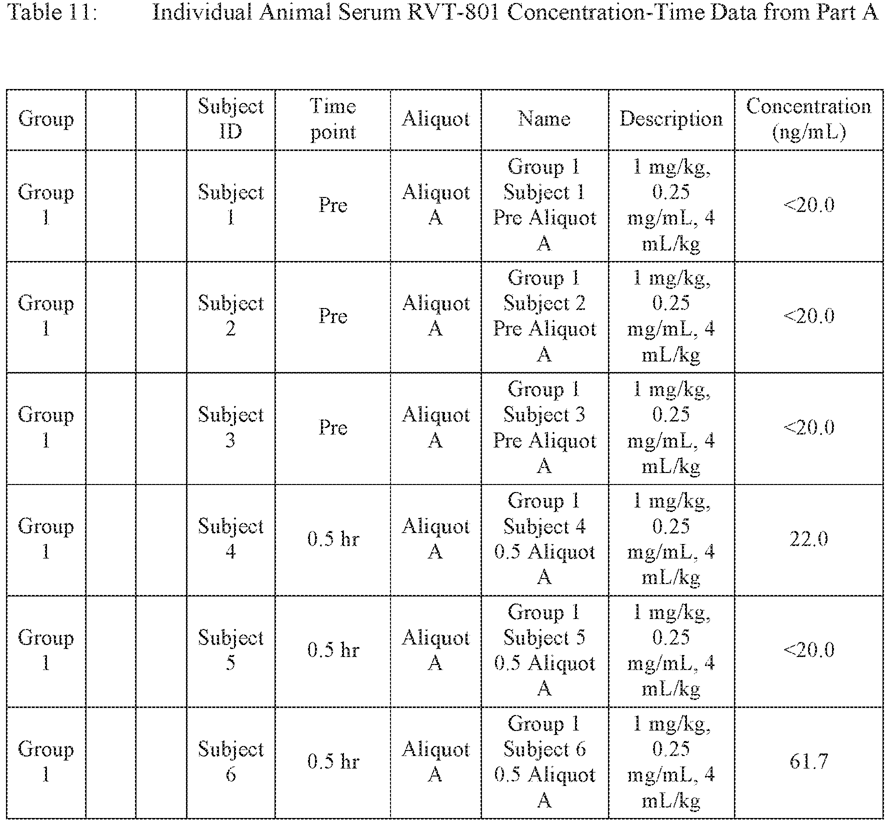

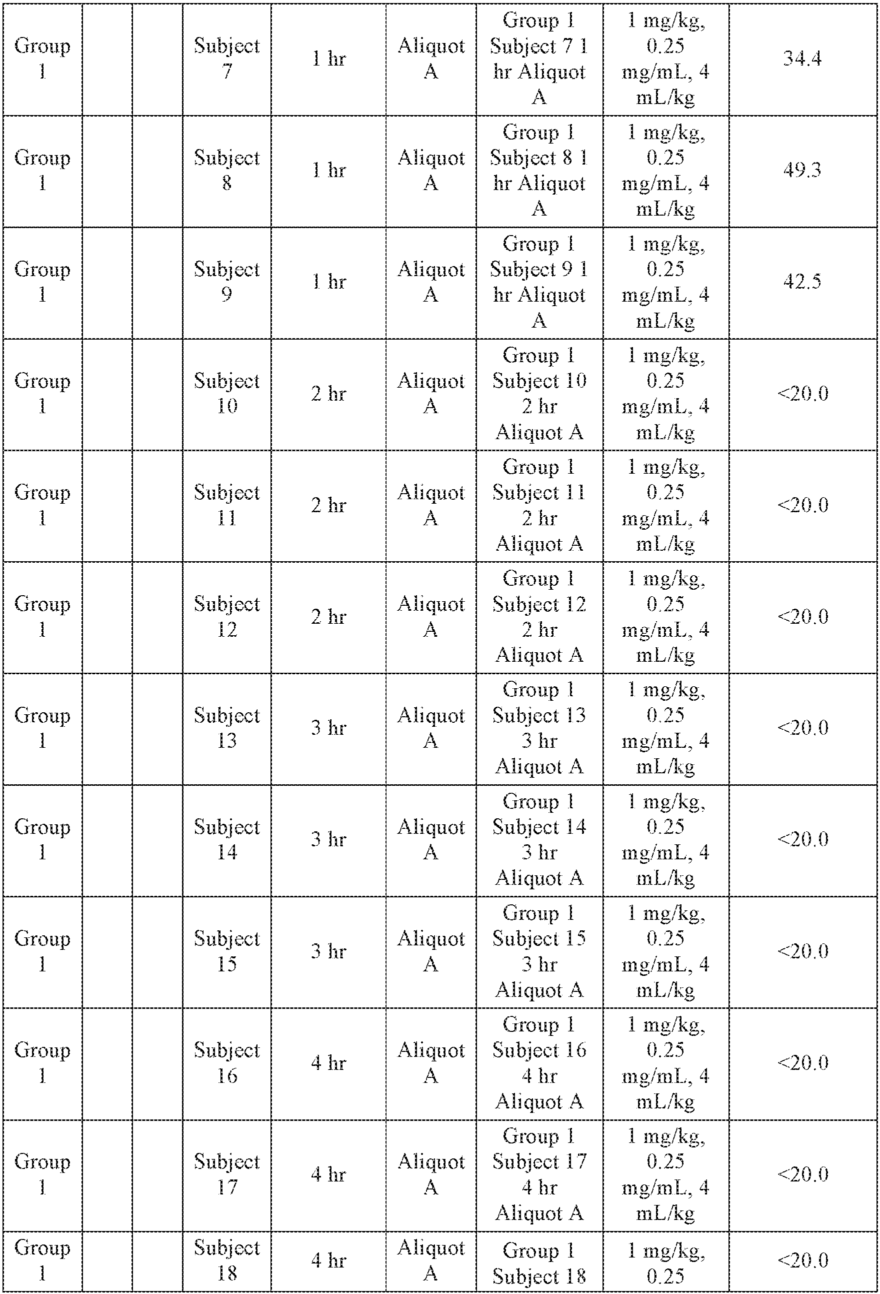

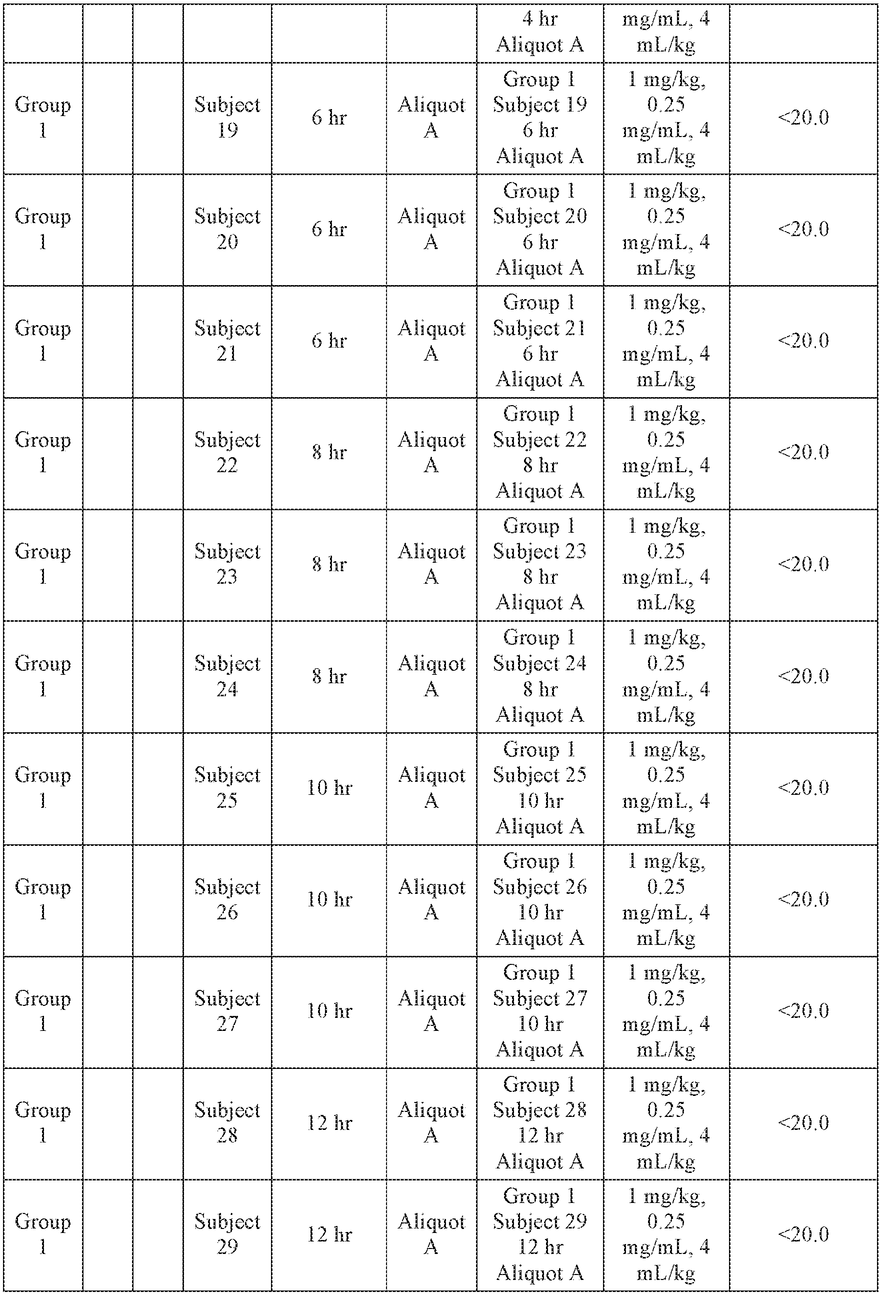

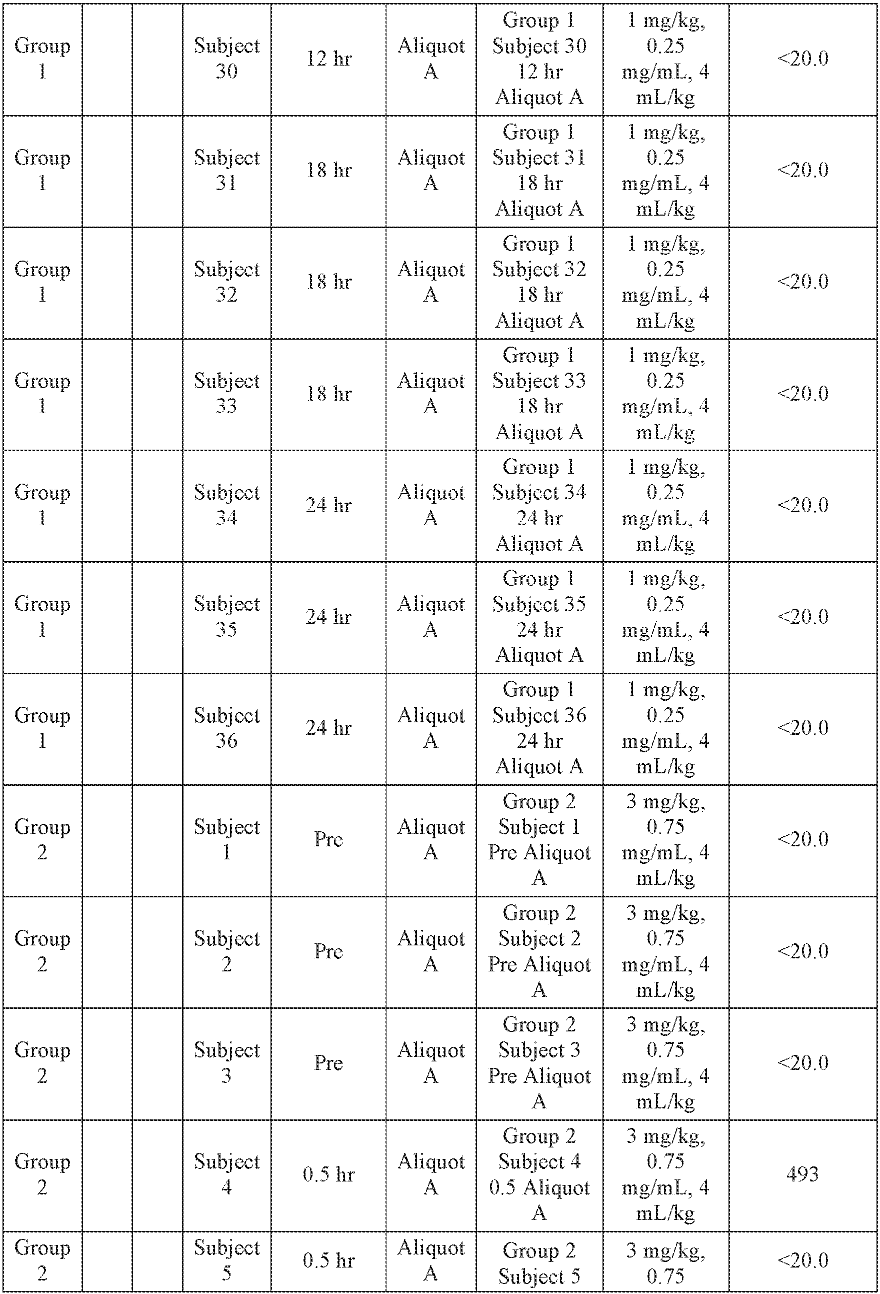

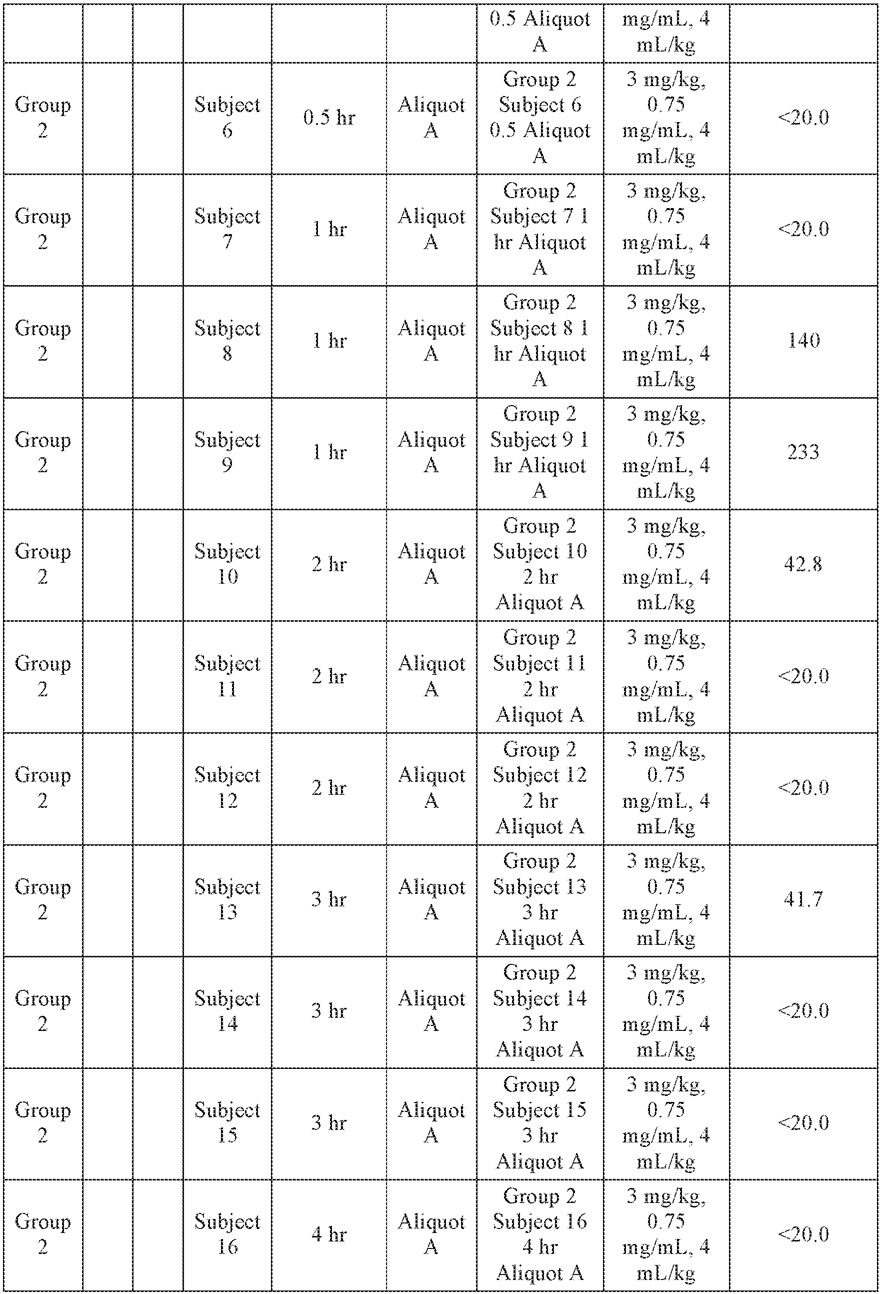

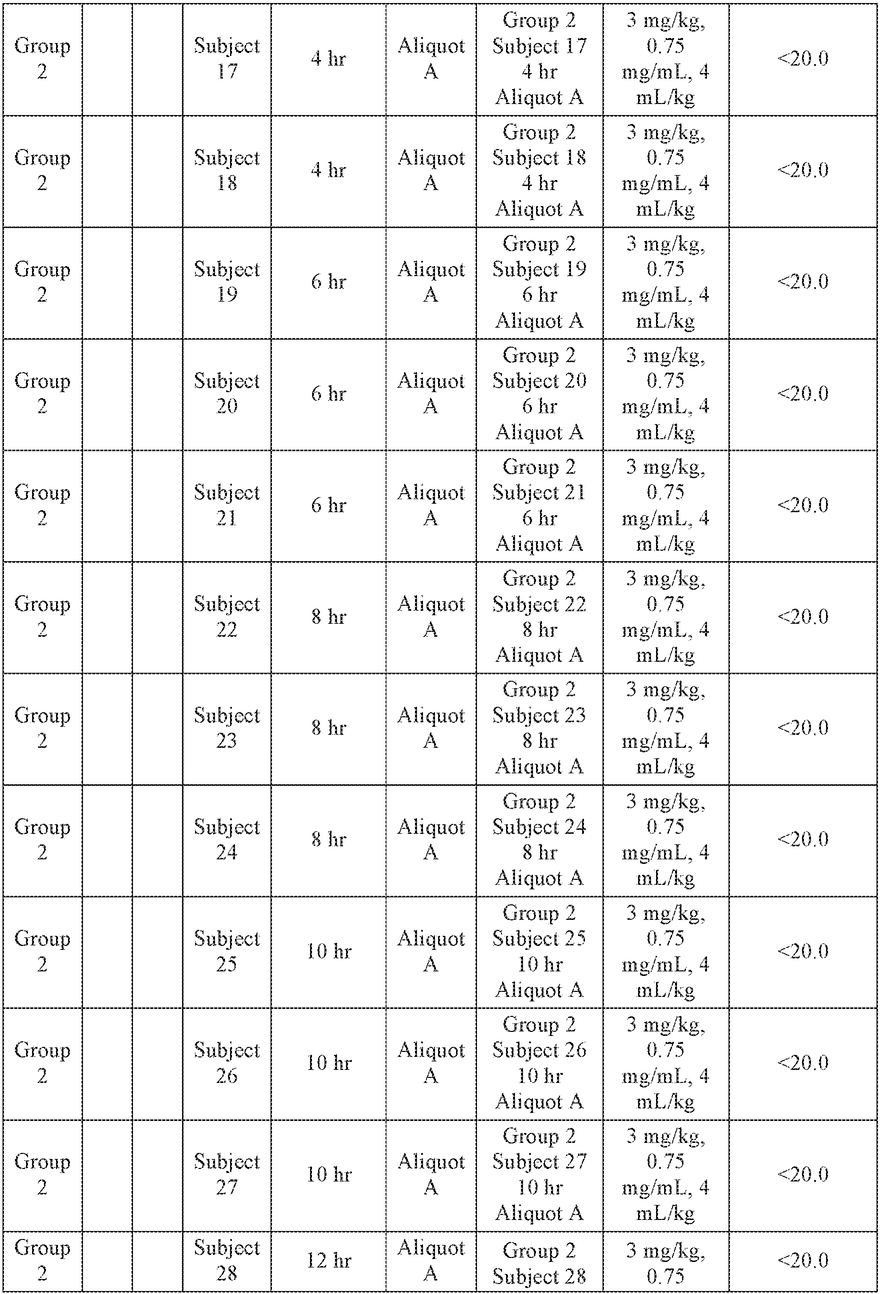

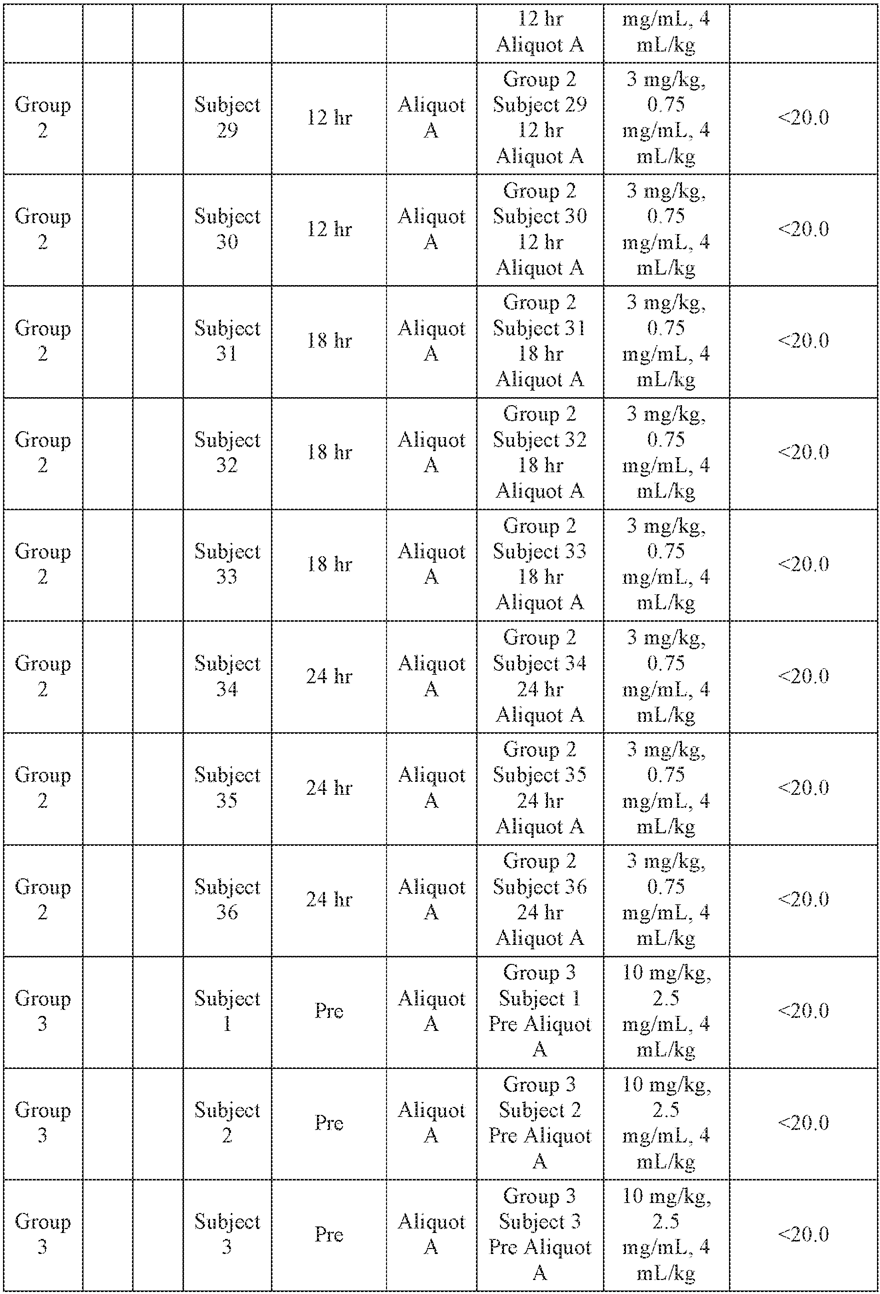

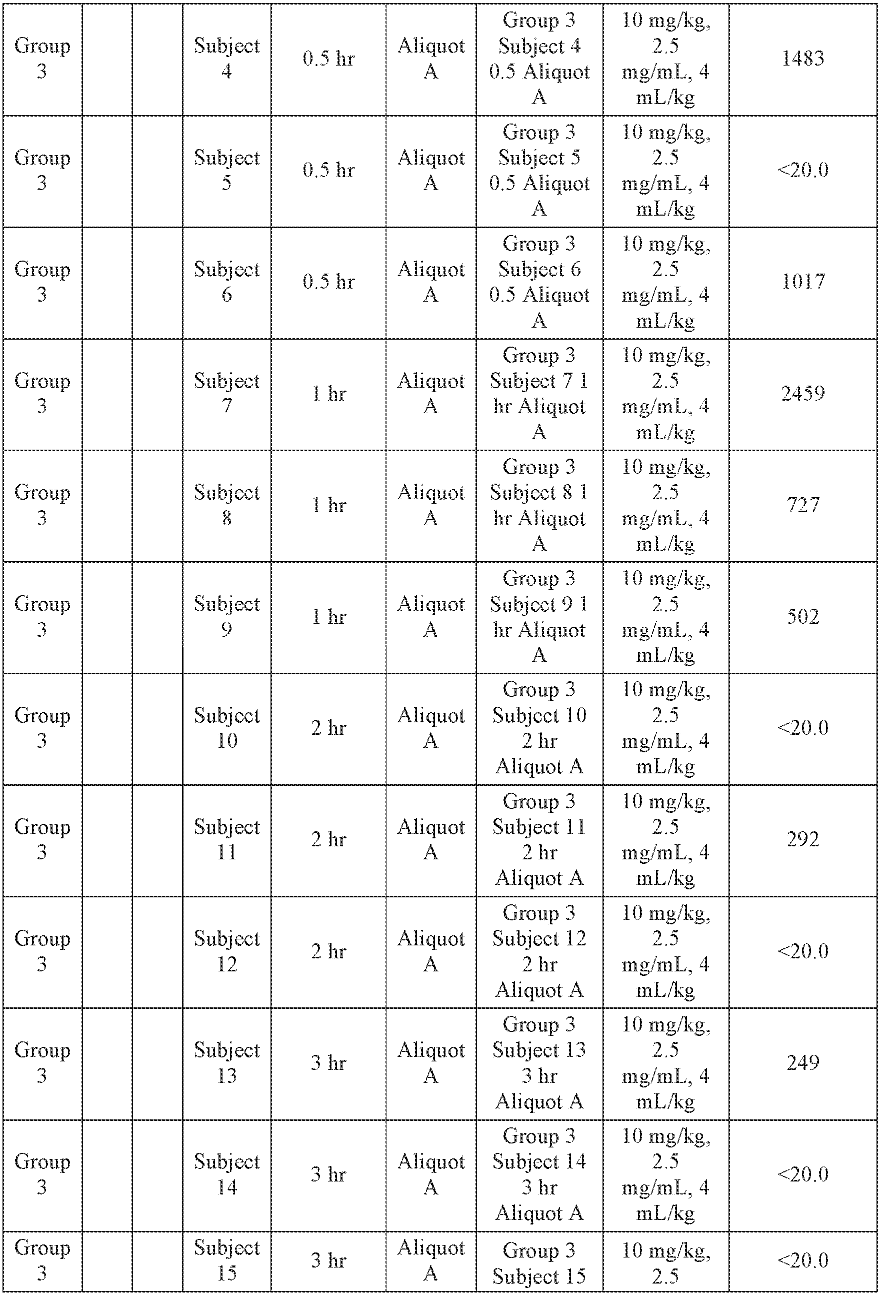

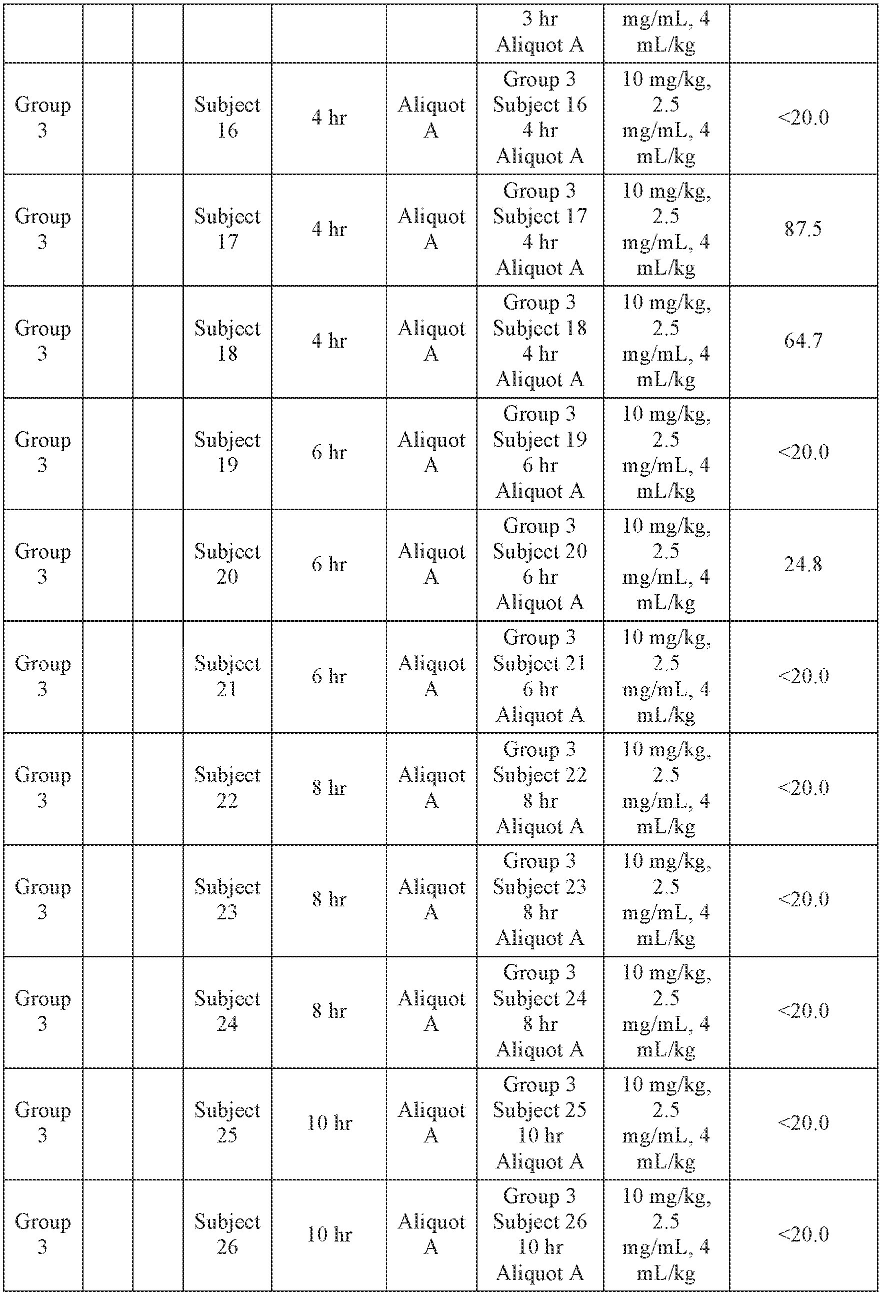

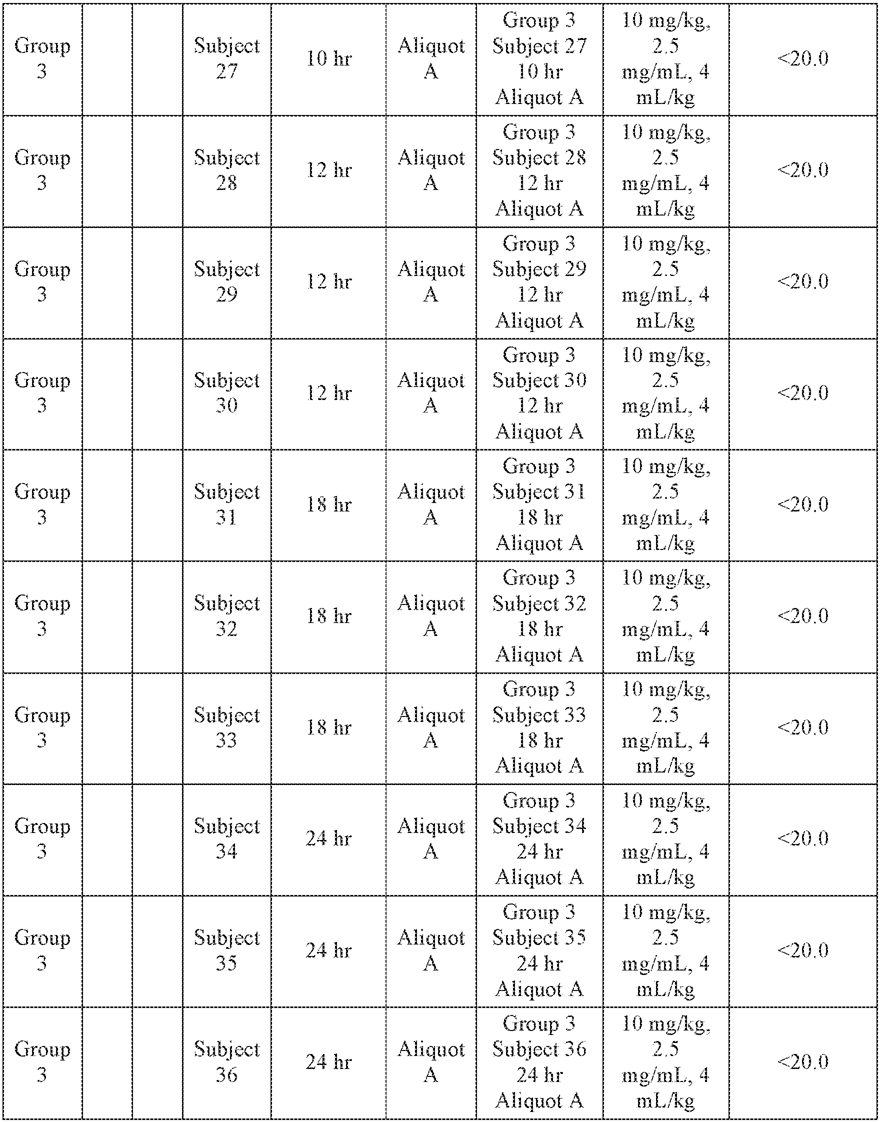

The composite mean RVT-801 concentration-time data grouped by tissue and dose are presented in Table 12.

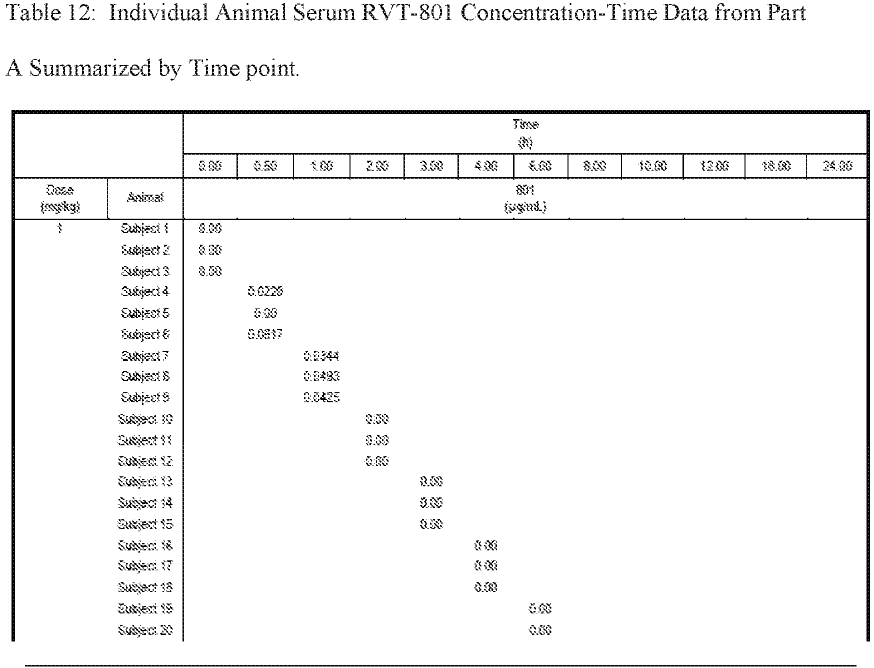

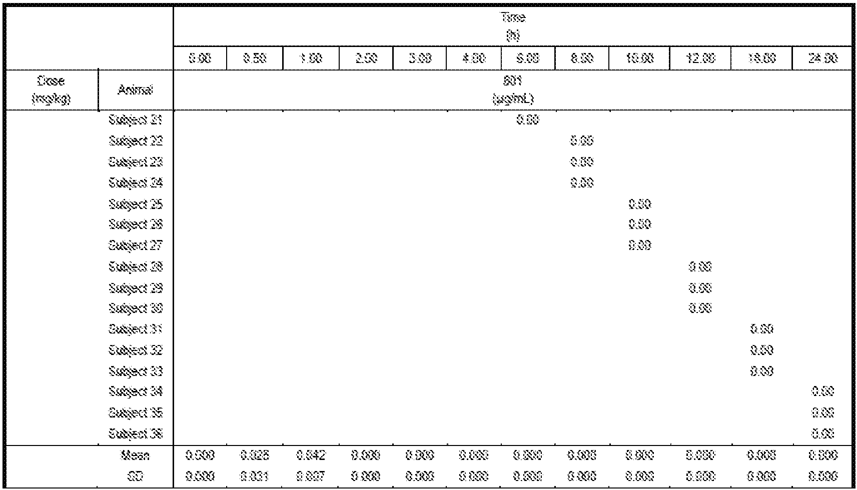

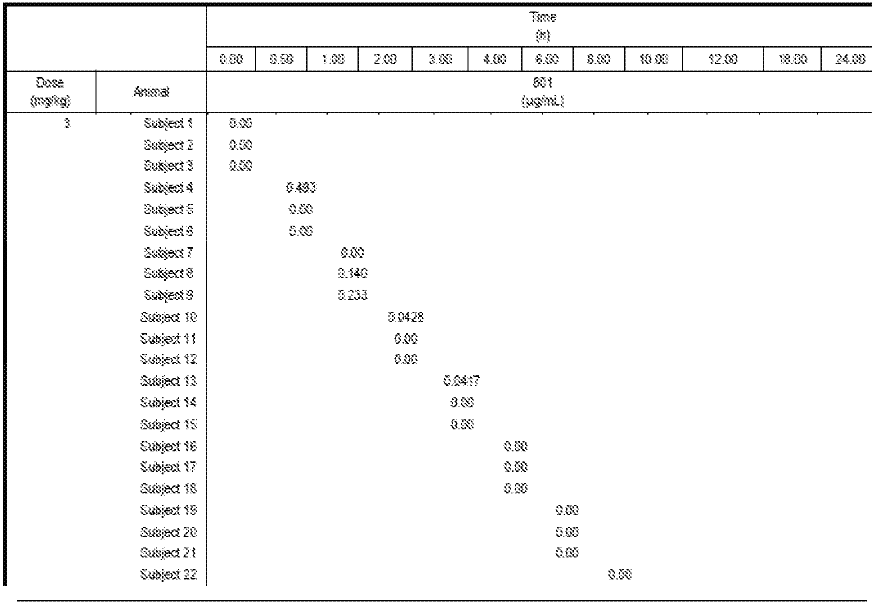

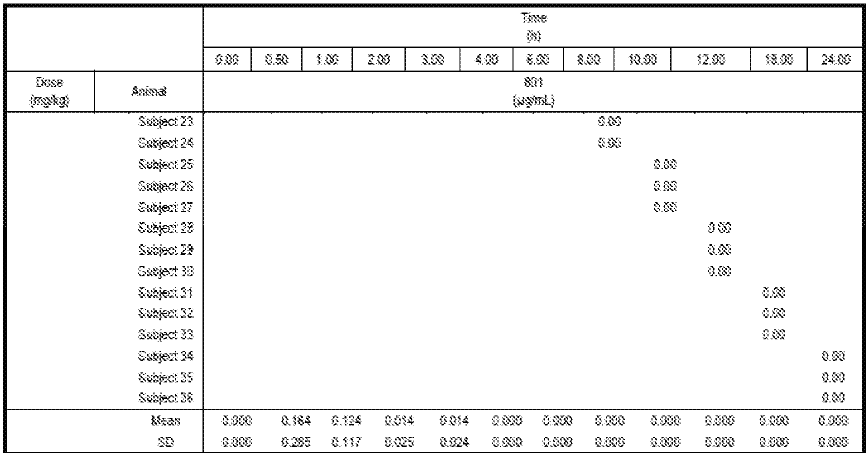

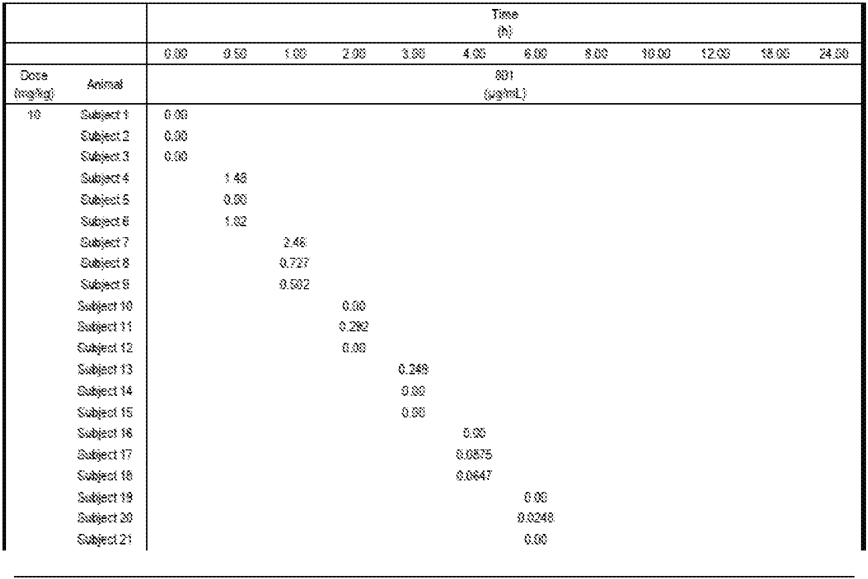

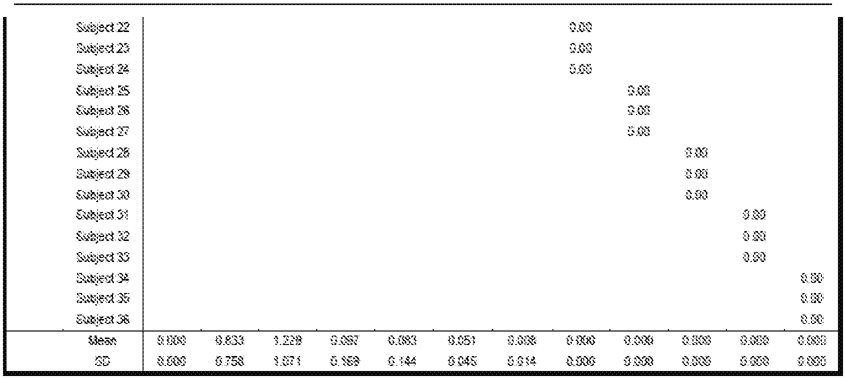

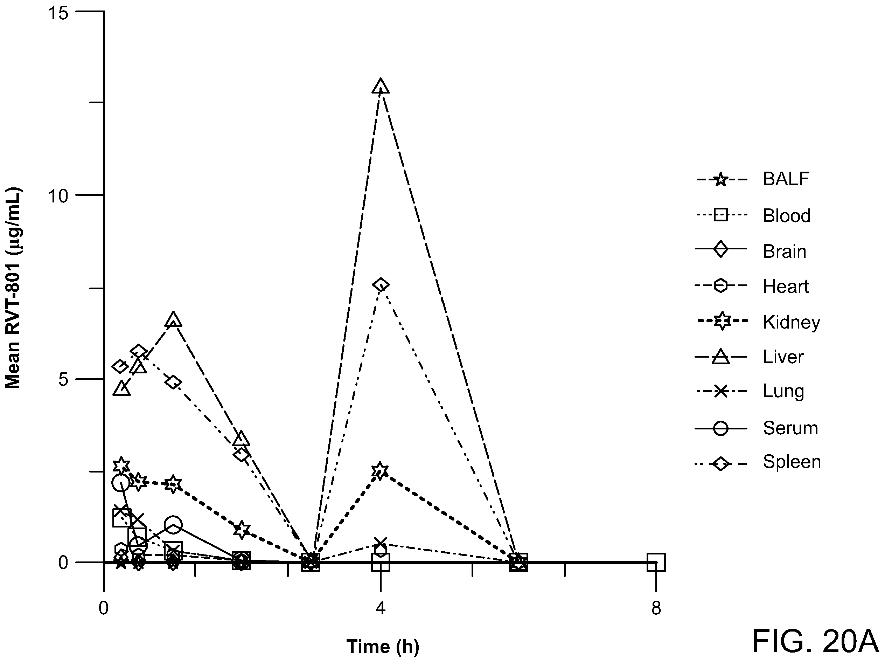

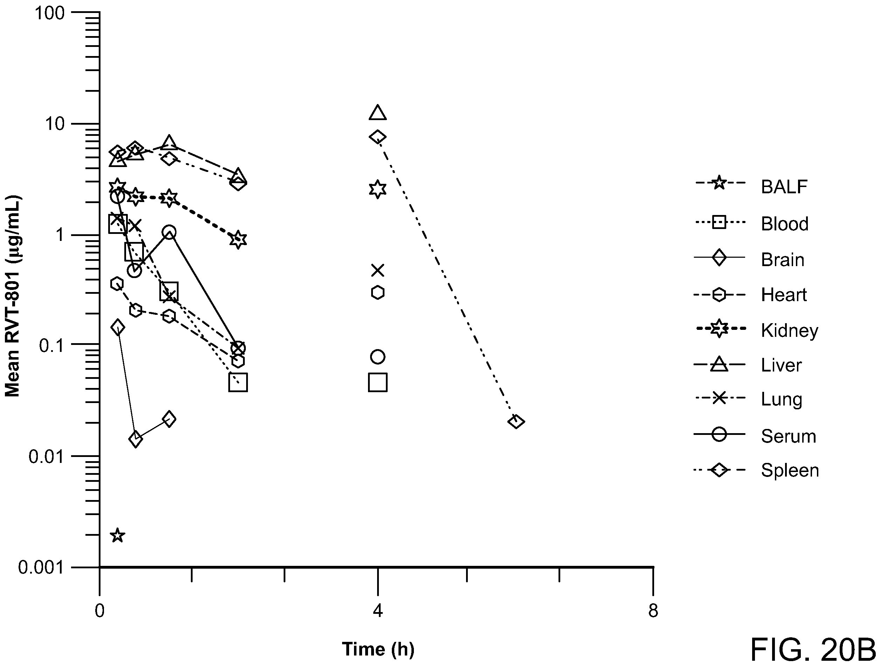

Mean RVT-801 concentration-time data for the 10 mg/kg dose group are displayed with tissues overlaid on linear axes in Fig. 20.

Figs. 20A and 20B show Mean RVT-801 Tissue Concentration-Time Profiles Following IP Administration to Juvenile Mice in Part B (Linear and Log-Linear), respectively.

Following IP administration to juvenile mice, mean serum RVT-801 concentrations were highest at 0.25 h following dose administration for all dose groups. Serum RVT-801 concentrations declined rapidly. The last non-zero mean concentration occurred at 3 h for the 1 mg/kg dose, 4 h post-dose for the 3 mg/kg dose, and 4 h post-dose for the 10 mg/kg dose. The variability in serum concentrations across dose groups was large. The serum concentrations in Part B of the study were generally consistent with serum concentrations from Part A.

Following a dose of 10 mg/kg, RVT-801 concentrations in tissues were quantifiable at the first time point post dose and were highest at 0.5 h post dose for lung and 1 h post dose in liver, spleen, kidney, and heart. Concentrations of RVT-801 generally remained quantifiable in tissues through 18 h post dose.

The composite mean RVT- 801 serum concentration-time data from Part A and Part B grouped by dose are tabulated in Table 13 and 14, respectively

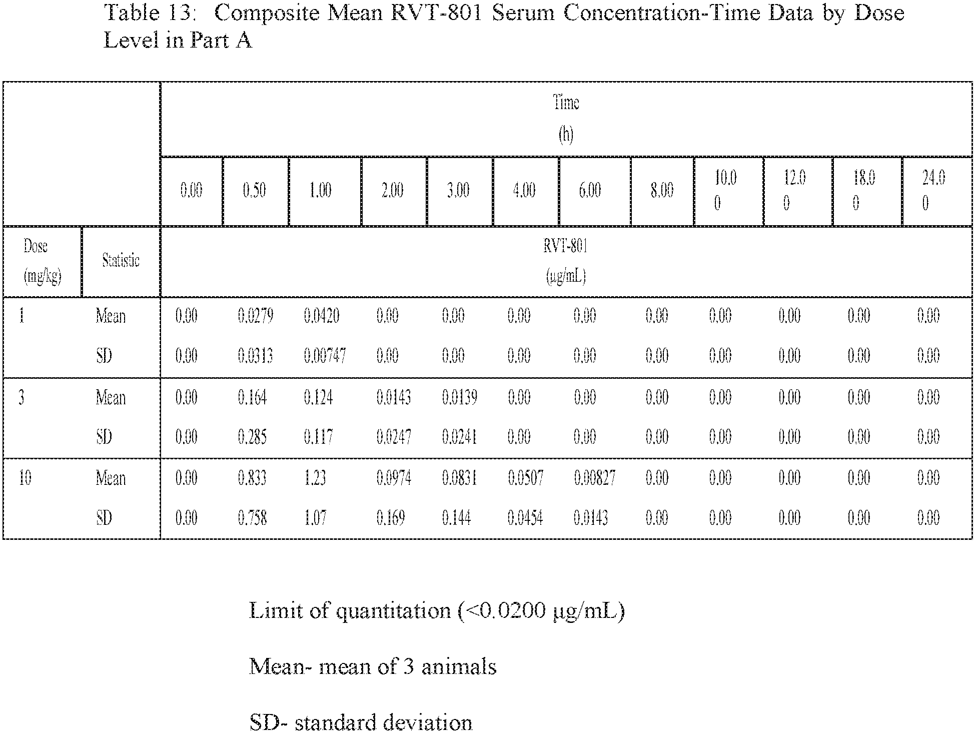

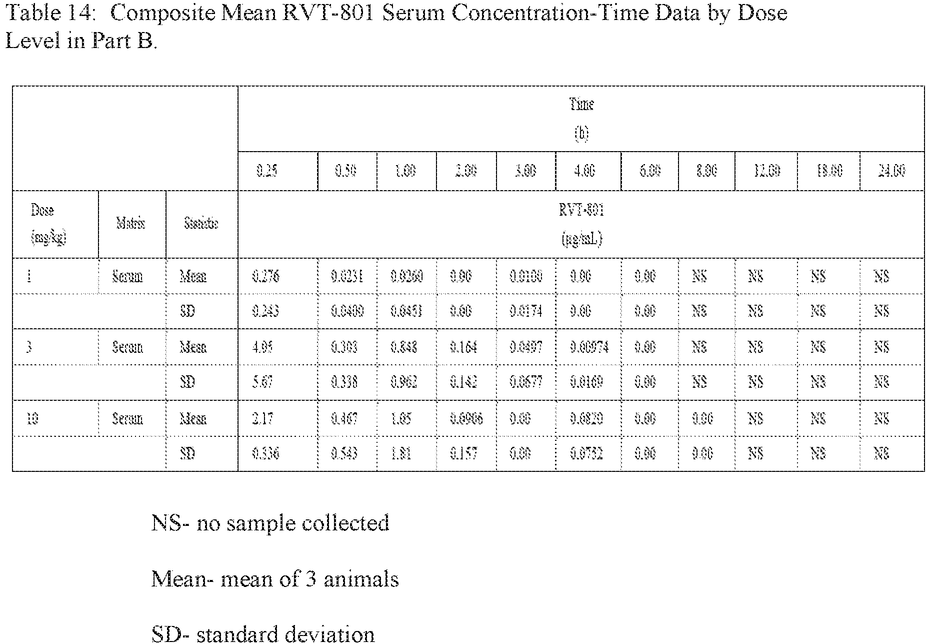

Composite mean non-compartmental PK parameters for RVT-801 by matrix are presented in Table 15.

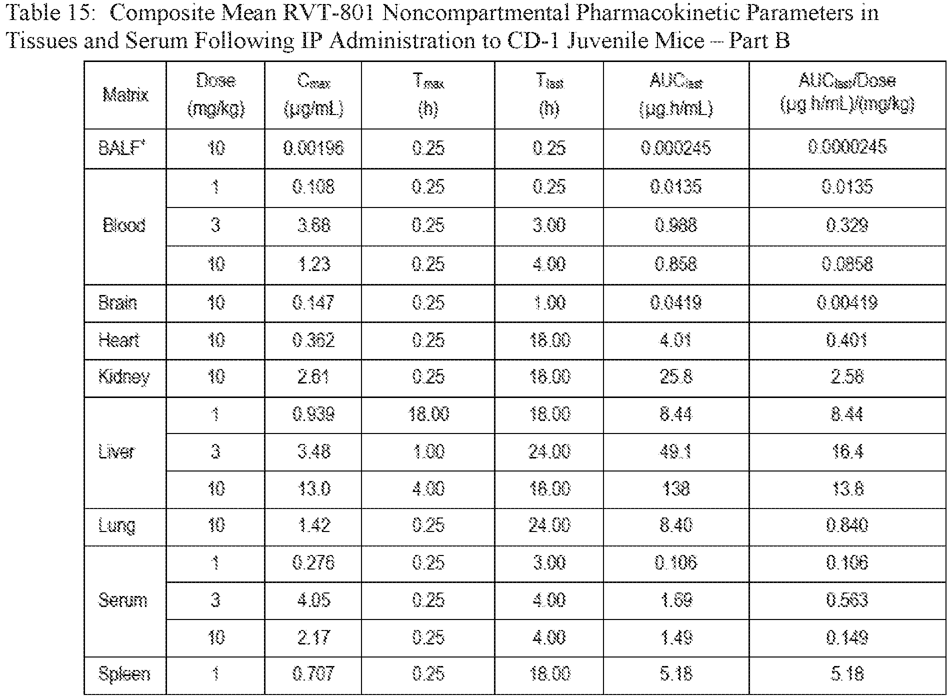

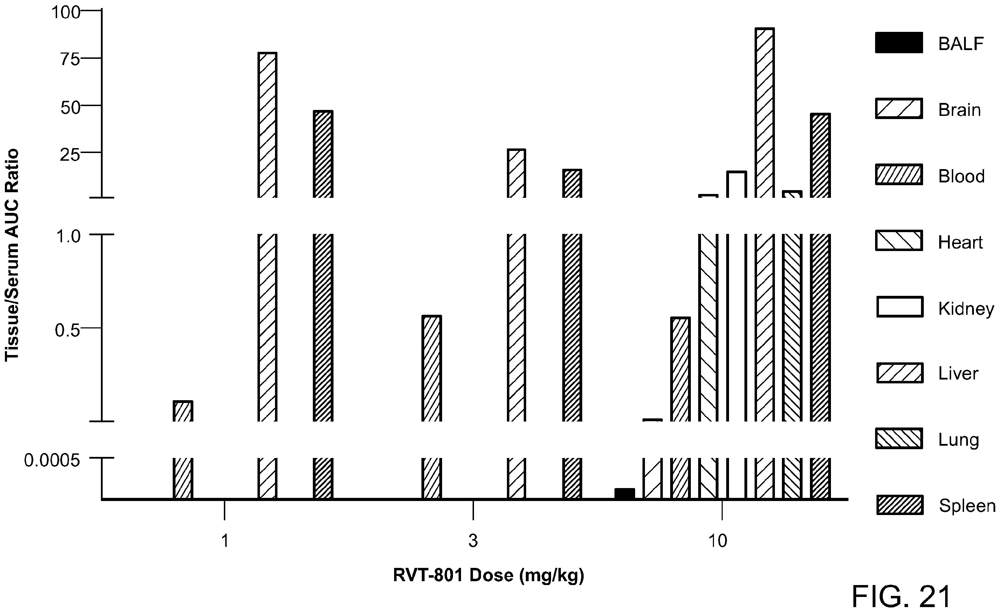

Tissue to serum exposure ratios are provided in Table 16 and displayed in Fig. 21. Fig. 21 presents RVT-801 Tissue: Serum Exposure Ratios based on AUCₗₐₛₜ.

BALF, brain, heart, kidney, and lung were only collected for the 10 mg/kg dose group.

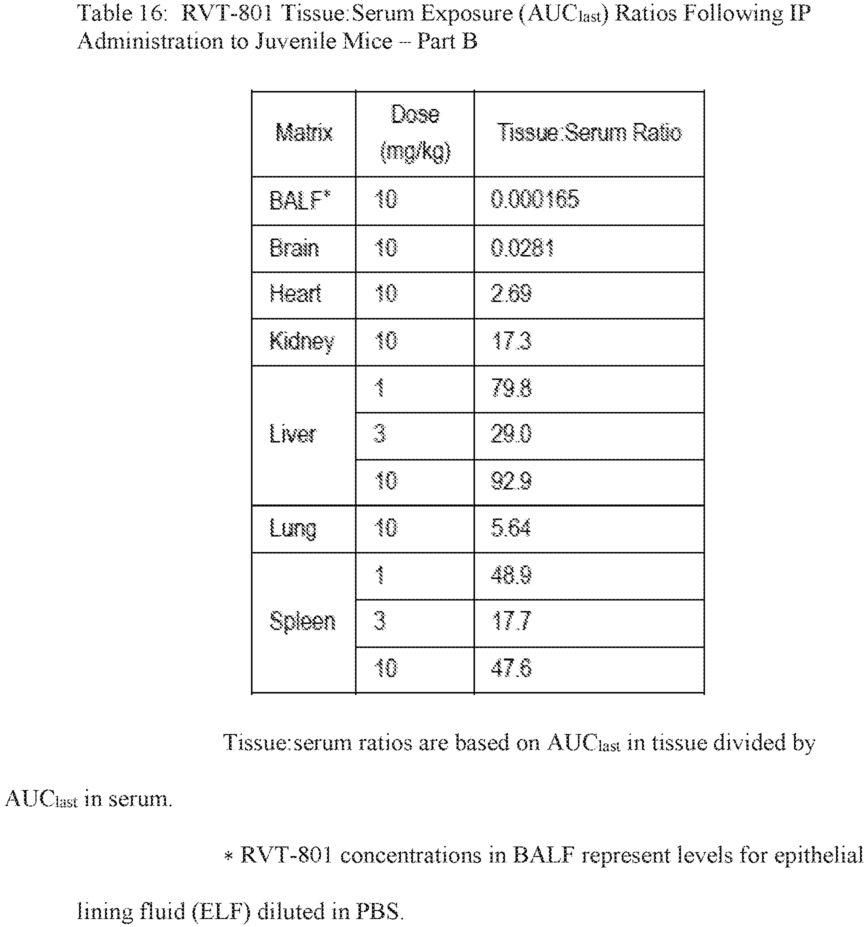

A correction factor to account for the dilution is required to reflect concentrations in ELF..

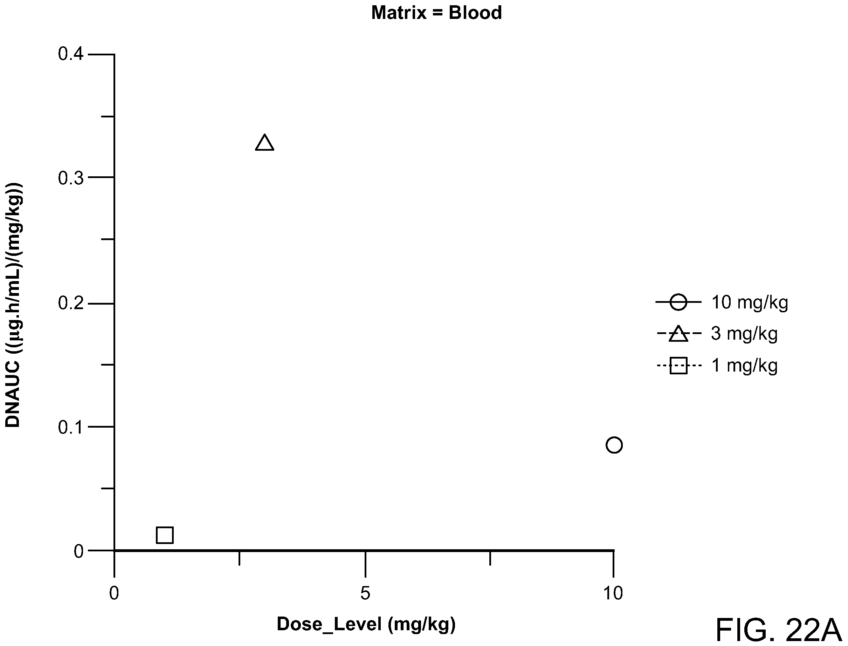

Dose normalized PK parameters plotted verses dose level for various tissues are presented in Figs. 22A-D.

Fig. 22A depicts dose normalized area under the curve (DNAUC) data in blood after administration of RVT-801 at 1 mg/kg, 3 mg/kg, and 10 mg/kg.

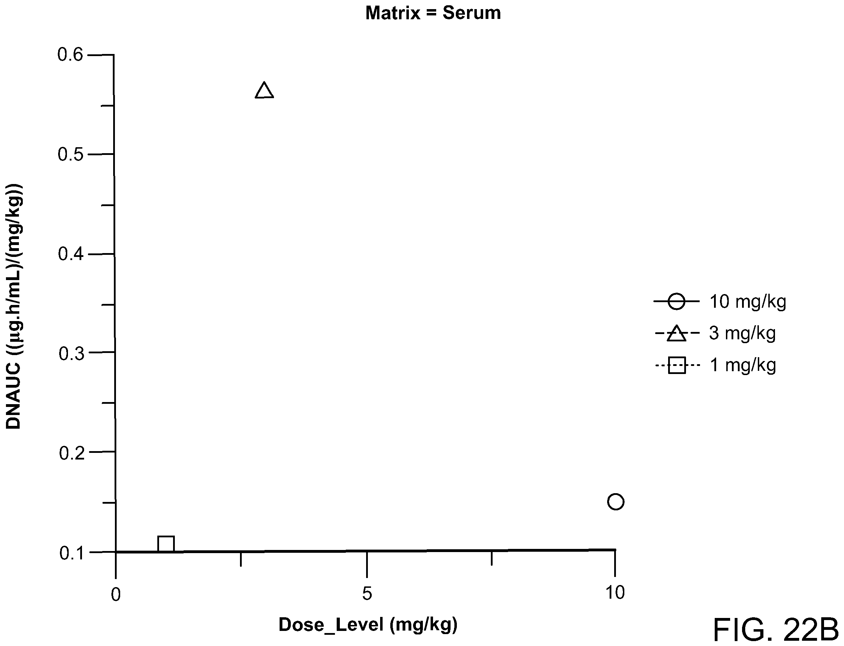

Fig. 22B depicts dose normalized area under the curve (DNAUC) data in serum after administration of RVT-801 at 1 mg/kg, 3 mg/kg, and 10 mg/kg.

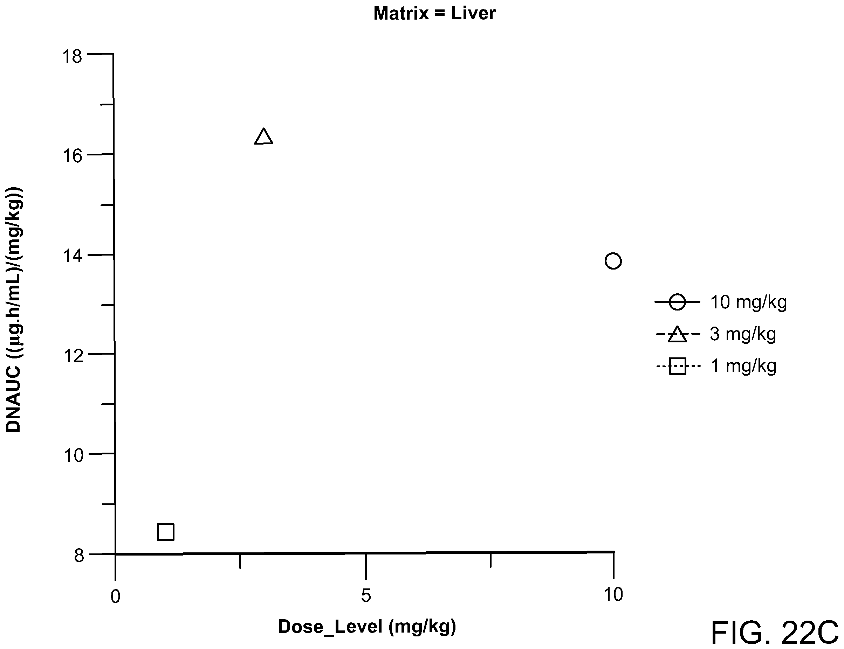

Fig. 22C depicts dose normalized area under the curve (DNAUC) data in liver tissue after administration of RVT-801 at 1 mg/kg, 3 mg/kg, and 10 mg/kg.

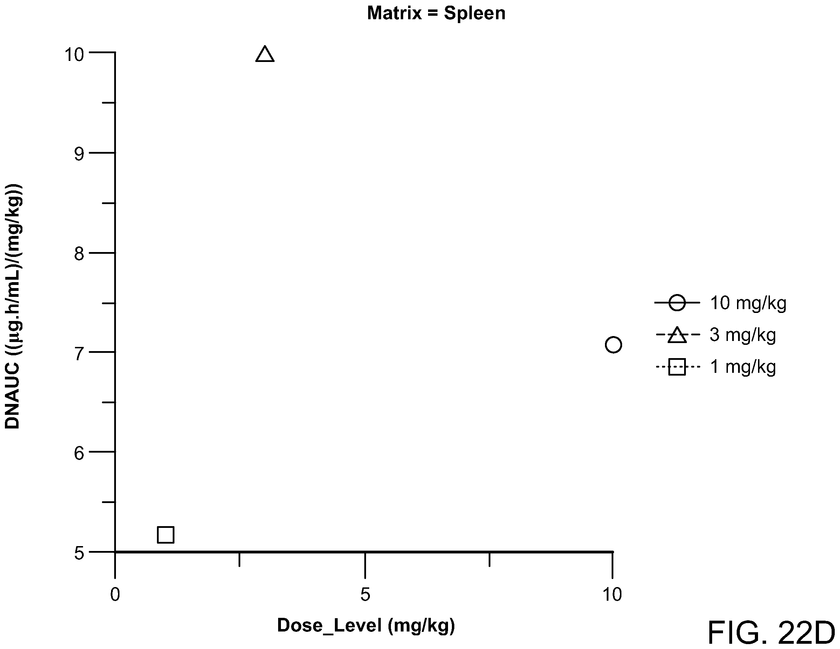

Fig. 22D depicts dose normalized area under the curve (DNAUC) data in spleen after administration of RVT-801 at 1 mg/kg, 3 mg/kg, and 10 mg/kg.

Systemic serum exposure to RVT-801 was short-lived, with concentrations in serum remaining quantifiable for a much shorter duration than tissue concentrations; 4 h in serum vs 18-24 h in tissues. Tₘₐₓ occurred within the first hour following administration, consistent with rapid distribution of compound to tissues.

RVT-801 was widely distributed to tissues, with tissue to serum exposure (AUCₗₐₛₜ) ratios ranging from 0.000165 (HALF: Serum) to 92.9 (Liver: Serum). Highest exposure was observed in liver > spleen > kidney > lung > heart > whole blood > BALF with Tₘₐₓ ranging from 0.25 h to 2 h post-dose. Exposures in liver, spleen, kidney, heart, and lung were quantifiable through 18 to 24 hours post-dose.

BALF samples consisted of ELF (epithelial lining fluid) diluted in the phosphate buffered saline (PBS) instillation fluid. The alveolar cellular population was not present in the assayed samples as cells were removed by centrifugation immediately following BALF collection. A correction factor reflecting the dilution of native lung fluid in PBS is required to convert RVT-801 concentrations in BALF to levels in ELF. Determination of the absolute dilution factor was not performed as part of this exploratory study. The correction factor can be determined by comparing the urea content in BALF to the urea content in the plasma from the same animal as native urea levels in epithelial fluid are generally accepted to reflect plasma urea content (Rennard SI, Basset G, Lecossier D, O'Donnell KM, Pinkston P, Martin PG, Crystal RG, 1986, "Estimation of volume of epithelial lining fluid recovered by lavage using urea as marker of dilution," J of Applied Physiology. 1986, 60(2): 532-538). The correction factors determined by Berkhout et al using a similar collection method (Berkhout J, Melchers MJ, van Mil AC, Seyedmousavi S, Lagarde CM, Nichols WW, Mouton JW, 2015 "Pharmacokinetics and penetration of ceftazidime and avibactam into epithelial lining fluid in thigh- and lung-infected mice,". Antimicrob Agents Chemother., 59(4): 2299-2304) were highly variable (mean, 11.6-fold; range, 4.3 to 144.2-fold for a 2mL total instillation volume) and indicate the likely dilution in the current study (0.5 mL instillation volume) may be up to 36-fold.

Concentrations of RVT-801 were lower in blood than in serum with blood:serum ratios ranging from 0.127 to 0.584 suggesting RVT-801 does not preferentially associate with erythrocytes. See Table 17.

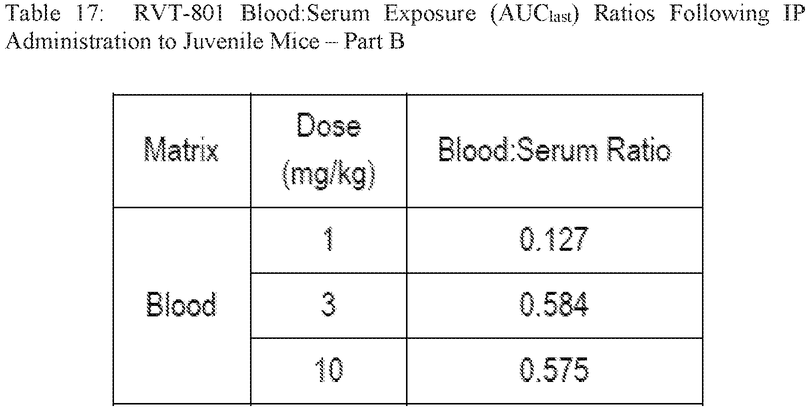

The objective of this study was to determine the serum and tissue pharmacokinetics of RVT-801 following a single dose treatment to juvenile male CD-1 mice of 1, 3, or 10 mg/kg, administered as a bolus IP injection. Following IP administration to juvenile mice, mean serum RVT-801 concentrations were highest between 0.25 and 1 h following dose administration for all dose groups and declined rapidly with an estimated half-life of 1 h. Serum RVT-801 increased with increasing dose, with Cₘₐₓ and AUCₗₐₛₜ increasing greater than proportional with respect to dose. The Vz/F exceeded total body water and the CL/F exceeded liver blood flow. There was a trend towards decreasing Vz/F and CL/F as the dose increased from 3 mg/kg to 10 mg/kg suggesting saturation in tissue distribution and clearance. The maximum effective dose (MED) in Farber mice was 10 mg/kg delivered by bolus IP injection consistent with the current study. The value for AUCₗₐₛₜ determined following a single 10 mg/kg dose to aged-matched, wild-type CD-1 mice was 1.37 µg.h/mL with a Cₘₐₓ of 1.23 µg/mL.

RVT-801 underwent rapid clearance from systemic circulation consistent with extensive distribution from serum to other tissues. Highest exposures of RVT-801 were observed in liver > spleen > kidney > lung > heart > whole blood > BALF. Tissue to serum exposure ratios based on AUCₗₐₛₜ ranged from 0.000165 (BALF:Serum) to 92.9 (Liver: Serum). Tissue exposure persisted generally through 18-24 hours post dose. Concentrations of RVT-801 were lower in blood than in serum with blood:serum ratios ranging from 0.127 to 0.584 suggesting RVT-801 does not preferentially associate with erythrocytes.

### EXAMPLE 5

A tissue distribution study in Farber mice aged 4-5 weeks at dosing was conducted utilizing a total of 12 Farber mice (Study RVT-801-9021; Part A). Six animals each were administered either a single dose of RVT-801 at 10 mg/kg or three, once-weekly doses of RVT-801 at 10 mg/kg/dose. Dose groups were comprised of animals of either sex as the supply of *Asah1*^{*P361R*/*P361R*/*P361R*} animals was limited and no gender-specific effects of rhAC have been observed in these animals. A summary of the dosing phase of the study is presented in Table 18.

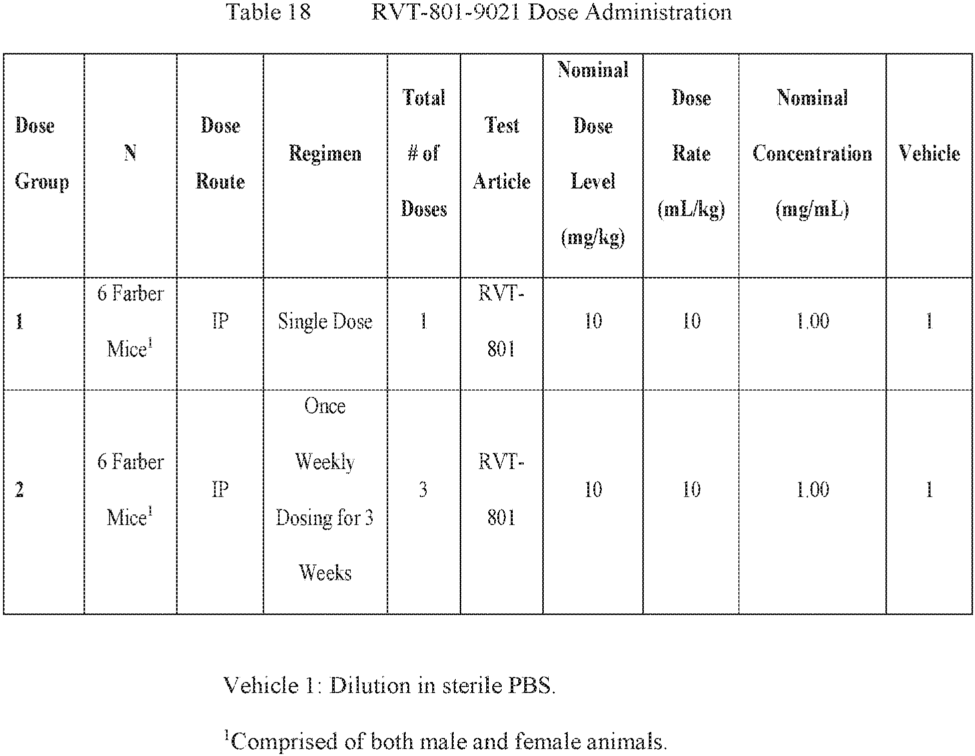

Male and female Farber mice, aged 4-5 weeks at the time of dose initiation, were administered either single or repeat, once-weekly doses of RVT-801 via bolus intraperitoneal (IP) injection. Dose material was prepared as indicated in Table 19. Individual doses were calculated based on bodyweights recorded on the day of dose administration. RVT-801 was diluted in sterile saline and dose material was prepared fresh on each day of administration.

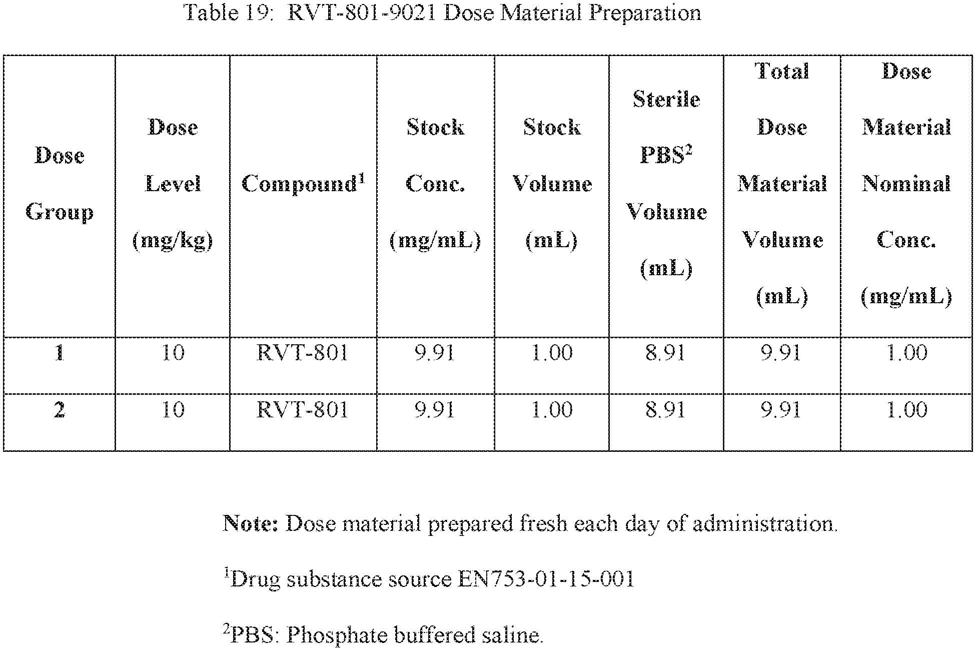

The experiment was designed to determine the concentration of rhAC in circulation and in tissues at selected time points following either single or repeat administration of RVT-801 to Farber mice and to assess the range and extent of RVT-801 distribution to key tissues in relation to the pharmacokinetics established in the CD-1 mouse.

### EXAMPLE 6

An immunotyping study in untreated and treated Farber mice was conducted. The untreated Farber mice and wild type (WT) mice were dosed at 4, 6 and 8 weeks. The treated Farber mice were dosed at approximately 4 weeks. The study was conducted utilizing 11 male/female) untreated Farber mice, 8 (male/female) untreated WT mice and 7 (male/female) treated Farber mice. Dosing was administered in once weekly doses of RVT-801. This arm of the study was designated as RVT-801-9025 Part B.

The rhAC administered in Examples 5 and 6 comprised a 10 mg/ml (actually 9.91 mg/ml) solution of rhAC at pH 7.4.

Subsequent to the administration of RVT-801, mice were euthanized in accordance with standard operating procedure and PK samples were collected at 6- and 24-hours post-administration (sample collection followed the third weekly injection of RVT-801 in the repeat dose group). Pharmacokinetic sample collection was restricted to these two time points due to limited available of Farber mice. Whole blood was collected via cardiocentesis into lithium heparin vials and processed in its entirety to plasma. Intact livers, spleens, kidneys, lungs, hearts, brains, and thymic tissue were harvested from each animal, gently blotted dry, and placed into individual vials. Tissues were neither fixed nor perfused with buffer at any stage, and samples were frozen without the addition of buffers, preservatives, protease inhibitors, or antibiotics. Plasma and tissue samples were stored at approximately -70°C and shipped on dry ice to the bioanalytical facilities for analysis. PK sample collection is detailed in Table 20.

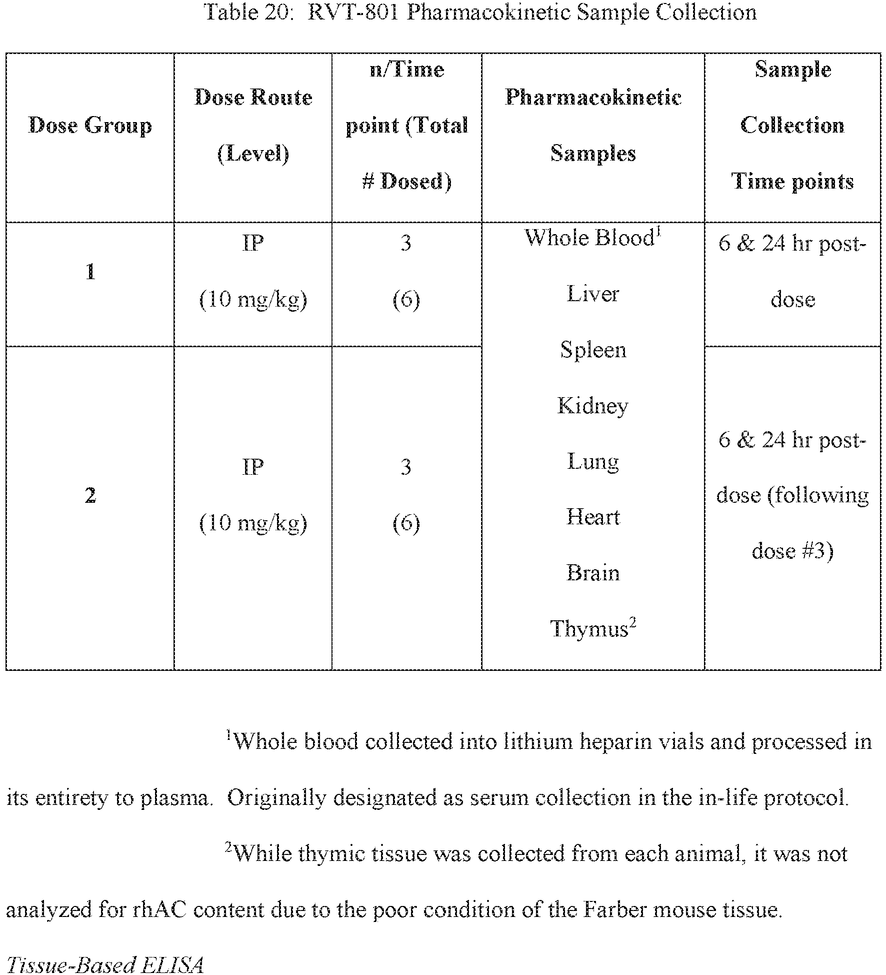

The concentration of rhAC in Farber mouse tissue following administration of RVT-801 was measured via ELISA conducted by BioAgilytix (Durham, NC).

Tissue samples were run against calibration standards prepared in homogenization buffer with either serum or buffer-based quality control (QC) samples.

Incurred tissue samples were homogenized in buffer (150 mM sodium chloride, 50 mM Tris-HCl, pH 7.5, 2% (w/v) CHAPS, Thermo Scientific) containing protease inhibitor cocktail (HALT, Thermo Scientific). Samples were processed with a single 5 mm stainless steel bead at 30 Hz using a TissueLyser II (Qiagen) for three cycles of three minutes apiece. Homogenates were placed at 2-8°C between cycles to control sample temperature. The total protein concentration of each tissue homogenate was determined with a colorimetric bicinchoninic acid (BCA) protein quantitation kit (Pierce), and samples were diluted to a nominal total protein concentration of 1 mg/mL with buffer prior to analysis by ELISA.

Capture antibody (rabbit anti-rhAC) was coated onto a microtiter plate. The plate was blocked with proteinaceous buffer and washed to removed excess capture and blocking reagents. Diluted tissue homogenates were added to the plate along with calibration standards and QCs and incubated to allow conjugation of the rhAC in the samples and controls with the antibody coating the plate. The plate was washed to remove unconjugated rhAC and incubated with the detection antibody (rabbit anti-rhAC-HRP). The plate was washed to remove unbound detection antibody and incubated with the addition of the colorimetric substrate (3,3',5,5'-tetramethylbenzidine (TMB)) to visualize rhAC conjugated with the detection antibody. Sample absorbance was read following the addition of stop solution. The assay generated a signal proportional to the concentration of rhAC in the sample using the parameters outlined in Table 21. RVT-801 concentrations in tissue homogenates were interpolated from the standard curve and corrected by application of dilution factors to calculate the concentration in native tissue.

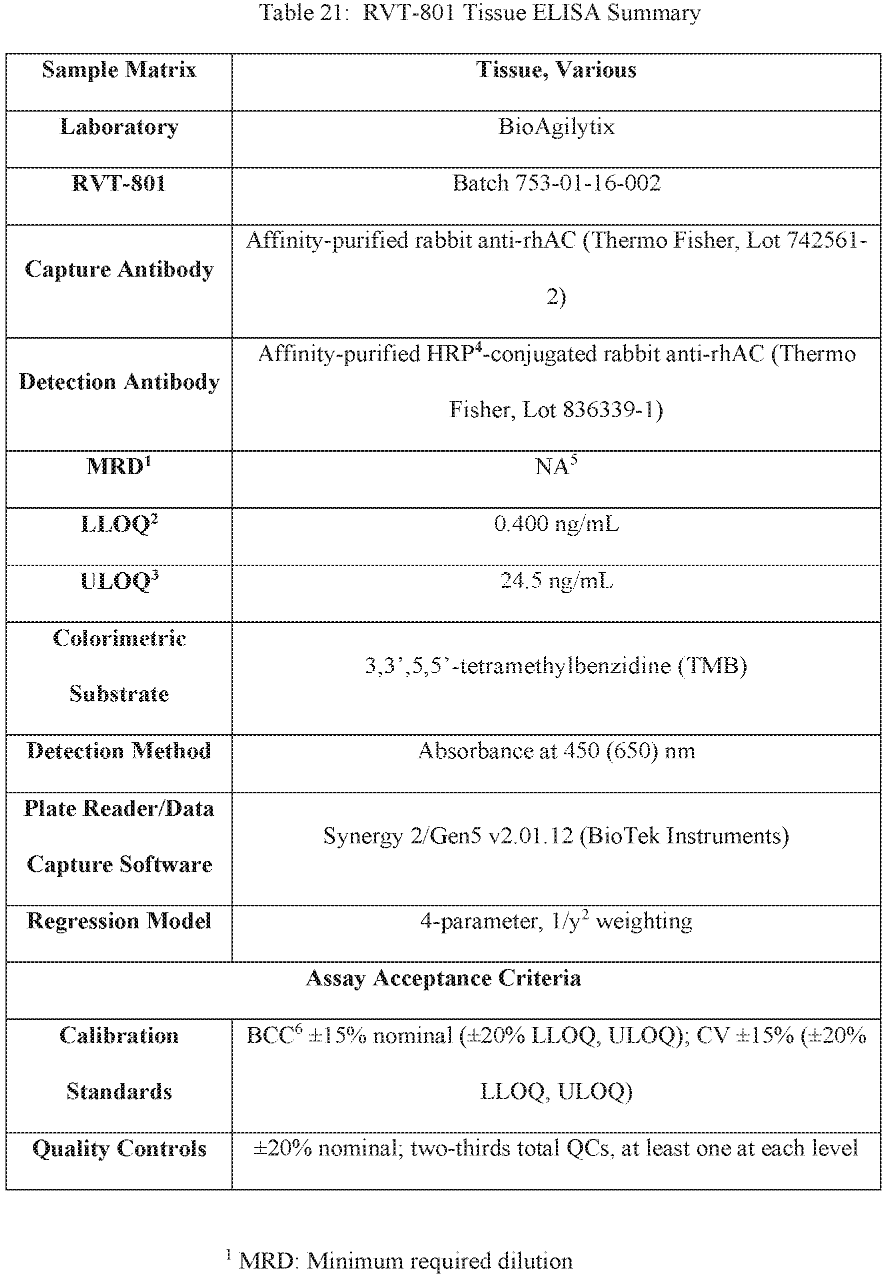

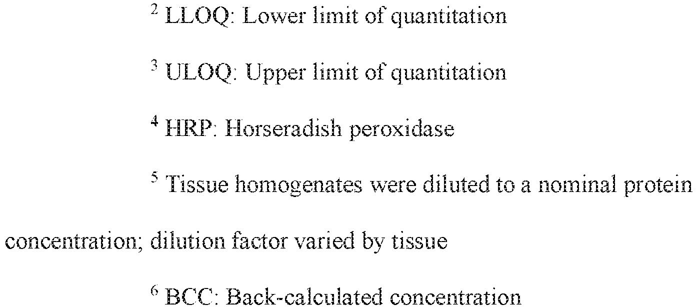

The content of rhAC in Farber mouse plasma was determined by qualified ELISA analysis. Briefly, a microtiter plate was coated with capture reagent (rabbit anti-rhAC), blocked, washed, and incubated with incurred plasma samples and matrix-matched calibration standards and QCs. Following incubation, the plate was washed to remove unbound rhAC and then incubated with the detection reagent (rabbit anti-rhAC-HRP). Excess (unbound) detection reagent was removed by further washing and the colorimetric substrate (3,3',5,5'-tetramethylbenzidine (TMB)) was added to the plate. Finally, stop solution was added to quench the colorimetric development and the absorbance of the samples was read. The intensity of the colorimetric signal generated in the assay was proportional to the concentration of rhAC in the samples. See Table 22 for a summary of the assay parameters. RVT-801 concentration in Farber mouse plasma samples was back-calculated from the standard curve.

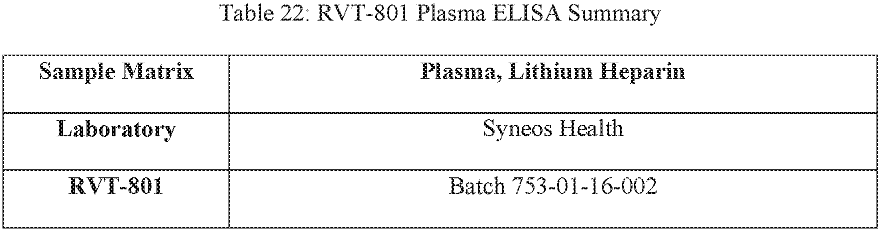

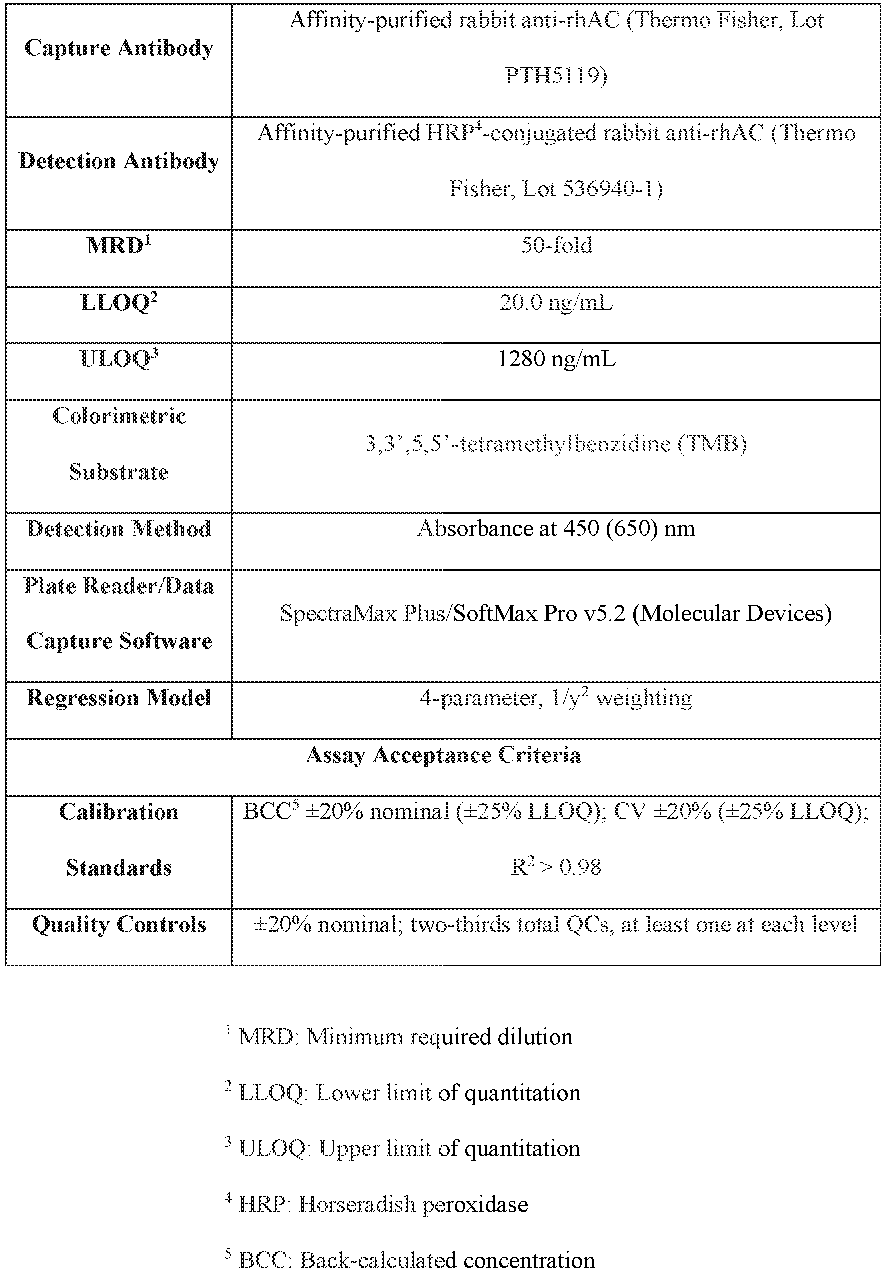

Area under the RVT-801 concentration-time curve was not calculated for Farber mice due to insufficient quantity and temporal density of data points over the post-administration period; a function of the limited number of *Asah1*^{*P361R*/*P361R*} animals available. For similar reasons, neither half-life nor clearance were calculated.

RVT-801 concentration data were reported to three significant figures, and Tₘₐₓ values were reported to two decimal places.

All incurred sample concentrations reported below the limit of quantitation (BLQ) were treated as having a value equal to zero when calculating mean concentration-time data.

### Results

Mean rhAC concentration-time data in plasma and selected tissues from Farber mice receiving either single or repeat weekly doses of RVT-801 at 10 mg/kg/dose are presented in Table 6 while individual data are detailed in Table 7.

Following a single dose of RVT-801, maximum rhAC concentration was measured at 6 hours post-administration (the first post-dose time point) across all biological matrices collected from Farber mice; however, these data may be biased by the limited number of feasible sampling time points.

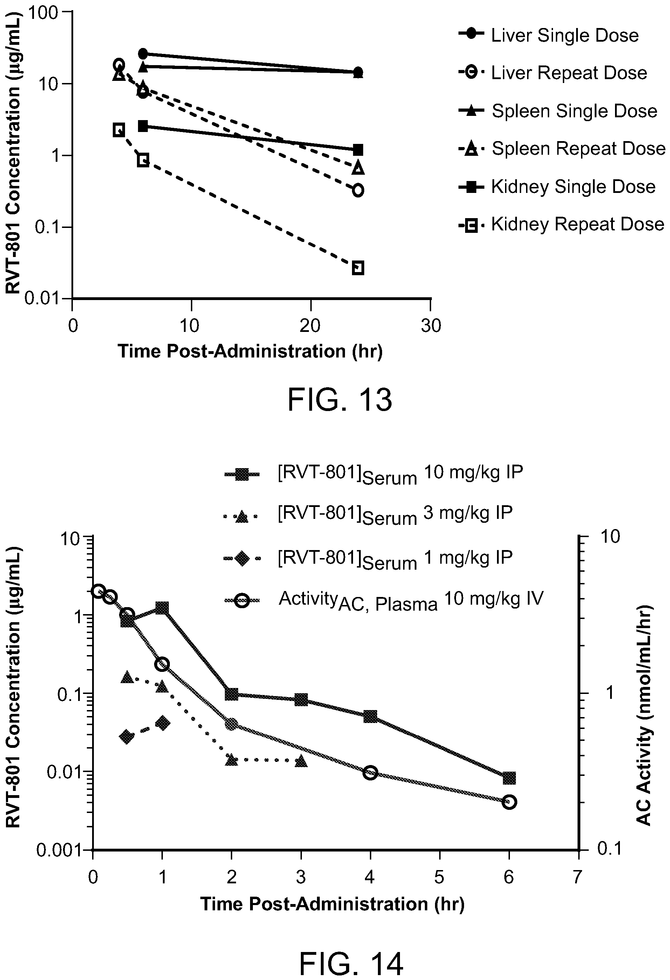

Following repeat administration of RVT-801 at 10 mg/kg/dose, rhAC levels in circulation and across the tissues collected at 6 and 24 hours post-dose were generally lower than those following a single dose of RVT-801 at 10 mg/kg in the CD-1 mice (RVT-801-9021), with multiple 24-hour repeat dose samples below the limit of quantitation. This trend is readily apparent amongst the three tissues with the highest overall rhAC content (liver, spleen, and kidney) following RVT-801 administration to Farber mice where the mean single dose 24-hour concentrations are greater than those following repeat administration by an order of magnitude (Fig. 13). The one exception to this general trend was brain, where the only samples with quantifiable rhAC concentrations were from two mice that received multiple doses of RVT-801.

Fig. 21 presents RVT-801 tissue:serum exposure ratios in BALF, blood, brain, heart, kidney, liver, lung, and spleen based on AUClast following single doses of RVT-801 of 1 mg/kg, 3 mg/kg, and 10 mg/kg to juvenile CD-1 mice.

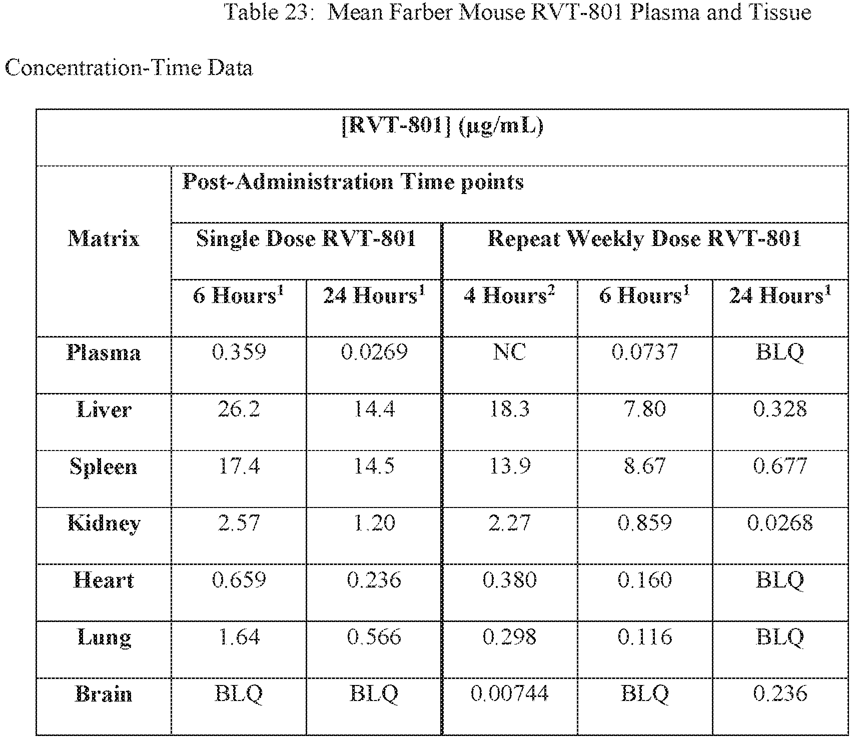

Table 23: Mean rhAC concentrations across selected biological matrices for Farber *(Asah1*^{*P361R*/*P361R*}) mice after receiving either a single dose or repeat once-weekly doses of RVT-801 at 10 mg/kg/dose via bolus IP injection. Animals in RVT-801-9025 Part B received four, once-weekly doses of RVT-801, while mice receiving multiple doses in RVT-801-9021 were administered three, once-weekly injections of RVT-801. RVT-801 sample concentration determined by sandwich ELISA. ELISA Calibrated Range: 20.0-1224 ng/mL (serum), 0.400-24.5 ng/mL (tissue), 20.0-1280 ng/mL (plasma).
BLQ: Below limit of quantitation
NC: Not collected
¹Data from study RVT-801-9021
²Data from study RVT-801-9025 Part B

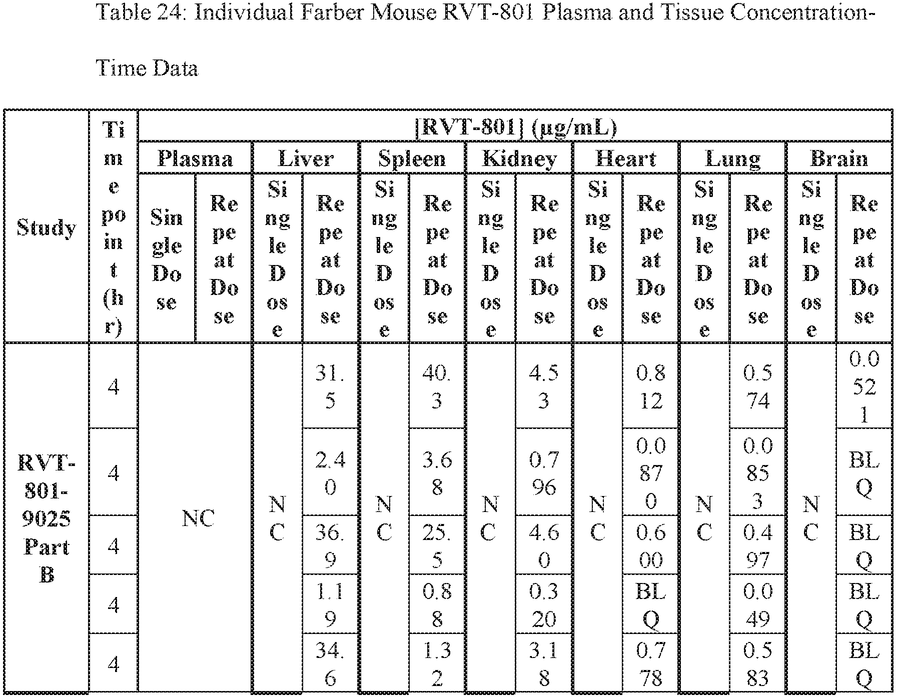

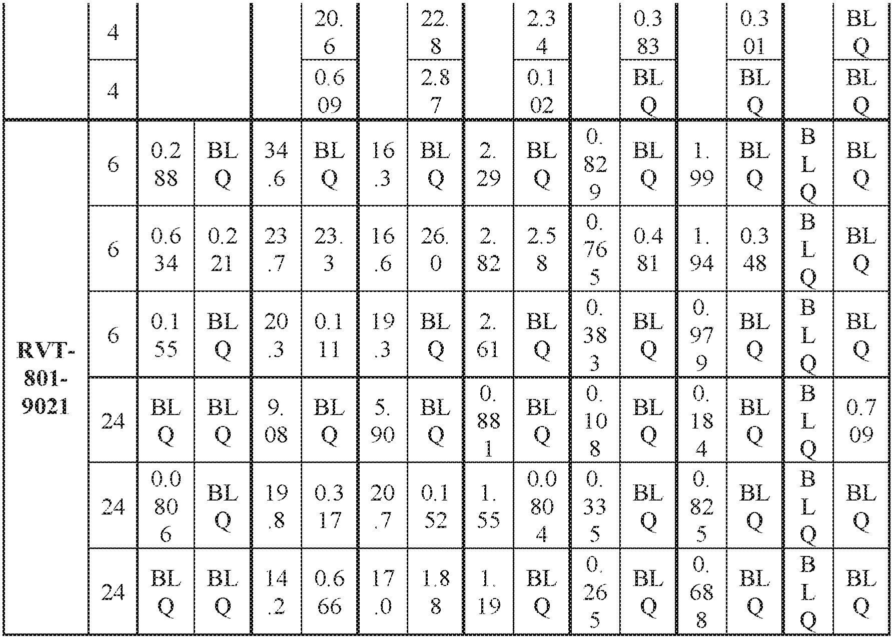

Table 24 presents individual rhAC concentrations across selected biological matrices for Farber (Asah1^{P361R/P361R}) mice after receiving either a single dose or repeat once-weekly doses of RVT-801 at 10 mg/kg/dose via bolus IP injection. Animals in RVT-801-9025 Part B received four, once-weekly doses of RVT-801, while mice receiving multiple doses in RVT-801-9021 were administered three, once-weekly injections of RVT-801. RVT-801 sample concentration determined by sandwich ELISA. ELISA Calibrated Range: 20.0-1224 ng/mL (serum), 0.400-24.5 ng/mL (tissue), 20.0-1280 ng/mL (plasma).
BLQ: Below limit of quantitation
NC: Not collected

### Results.

The murine pharmacokinetics of RVT-801 were characterized previously in healthy, juvenile male CD-1 mice, aged ~3.5 weeks (RVT-801-9013 Part A). Mice were dosed just after weaning to approximate the size of Farber mice and their age at the initiation of the efficacy studies undertaken in the severe Farber model. The decision to employ the CD-1 mouse for general ADME studies was based on the common genetic background (parental strain of the Farber mouse) along with the cachexic nature and characteristic fragility of the severe Farber mouse influencing their suitability and availability to describe a full concentration-time course of administered RVT-801.

When administered as a single IP bolus injection to healthy juvenile male CD-1 mice at 1, 3, or 10 mg/kg, the systemic exposure of RVT-801 generally increased in a supra-proportional manner with respect to dose in terms of both Cₘₐₓ and AUC, with concentrations in serum peaking within 1 hour of administration. While relatively short-lived in circulation (half-life < 1 hour), the apparent volume of distribution and apparent clearance were consistent with rapid and extensive distribution of RVT-801 beyond the vasculature as these values exceeded total body water and hepatic blood flow, respectively (Table 25).

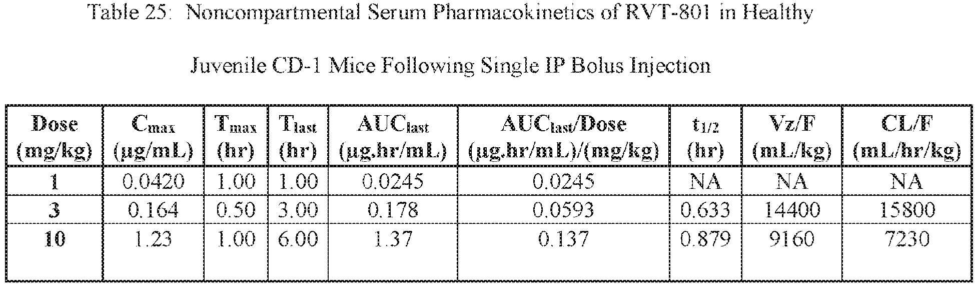

Table 25. Serum pharmacokinetics of RVT-801 following single IP bolus injection to healthy juvenile male CD-1 mice; PK samples collected to 24 hours post-administration, n=3 per time point.
Cₘₐₓ: Maximum observed concentration
Tₘₐₓ: Time of maximum observed concentration
Tₗₐₛₜ: Time of final quantifiable time point
AUCₗₐₛₜ: Area under the concentration-time curve from 0 to last quantifiable time point
t_{1/2}; Terminal phase half-life
Vz/F: Apparent volume of distribution following extravascular administration
CL/F: Apparent clearance following extravascular administration

The duration of exposure and general elimination kinetics of RVT-801 in CD-1 mice correlated closely with the duration and half-life of measured acid ceramidase activity in Farber mice following a single 10 mg/kg dose of rhAC to either strain (Fig. 14). This observation lent additional credence to the approach of using CD-1 mice to characterize the murine pharmacokinetics of RVT-801, and to extrapolate the general ADME properties to the disease model.

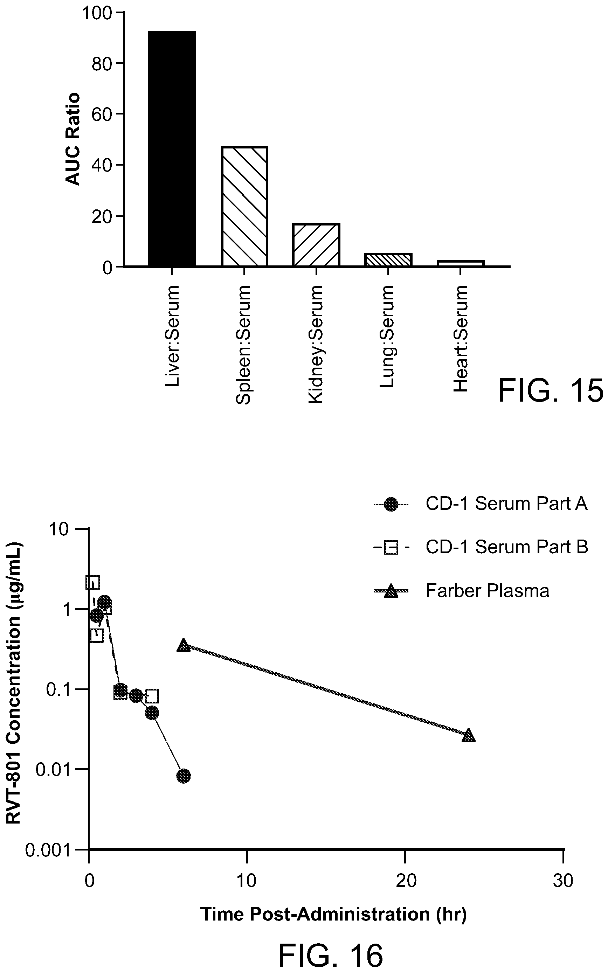

Fig. 15 shows final rhAC tissue: serum AUC ratio in healthy juvenile CD-1 mice following a single 10 mg/kg dose of RVT-801.

When administered as a single IP bolus injection to juvenile male CD-1 mice at 1, 3, or 10 mg/kg, RVT-801 achieved extensive distribution to a range of selected tissues and organs (RVT-801-9013 Part B). Ranking tissues in order of exposure indicated liver>spleen>kidney>lung>heart>serum>blood, based on AUC (Fig. 15, Table 9).

In vivo blood distribution analysis suggested that RVT-801 did not preferentially associate with erythrocytes. Significantly prolonged durations of exposure in tissue relative to serum were observed; whereas RVT-801 was quantifiable in serum for up to 4-6 hours after dosing, tissue exposure generally persisted for at least 18-24 hours post-administration.

At a dose of 10 mg/kg RVT-801 to CD-1 mice, which correlated to the maximum effective dose (MED) in the Farber mouse model based on reduction of accumulated levels of ceramide in liver and spleen, the majority of quantifiable rhAC in the tissues selected for analysis was distributed to liver, spleen, and kidney.

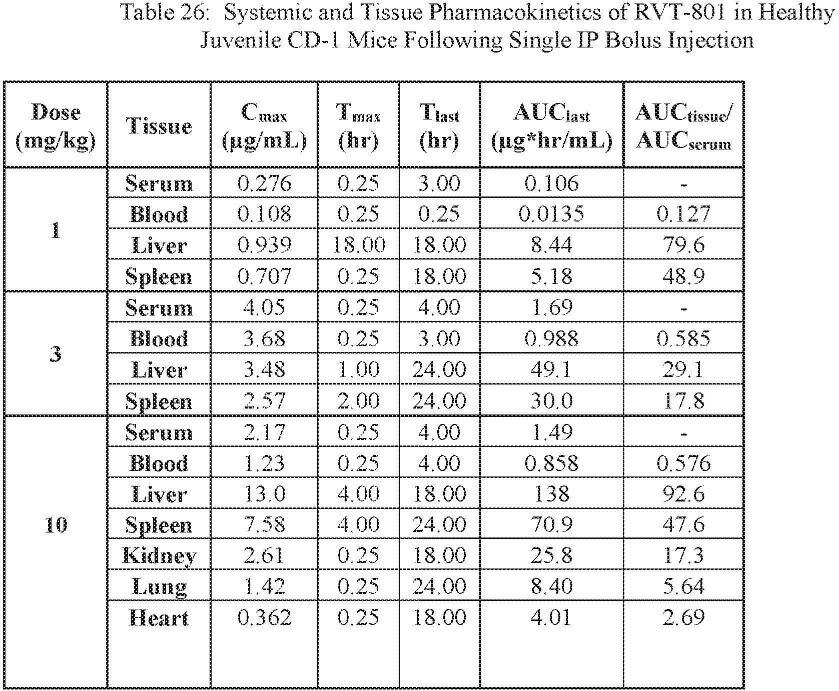

Table 26. Systemic and tissue pharmacokinetics of RVT-801 following single IP bolus injection to healthy juvenile male CD-1 mice; PK samples collected to 24 hours post-administration, n=3 per time point.
Cₘₐₓ: Maximum observed concentration
Tₘₐₓ: Time of maximum observed concentration
Tₗₐₛₜ: Time of final quantifiable time point
AUCₗₐₛₜ: Area under the concentration-time curve from 0 to last quantifiable time point

A comparison of the single-dose pharmacokinetics of RVT-801 in Farber mice (limited to two post-administration time points), to the exposure profiles characterized in CD-1 mice suggested that rhAC exposure was higher in Farber mice, with markedly increased systemic levels of rhAC and generally higher exposures across tissues at the time points analyzed, following a single 10 mg/kg bolus IP injection of RVT-801 to either strain (Table 26).

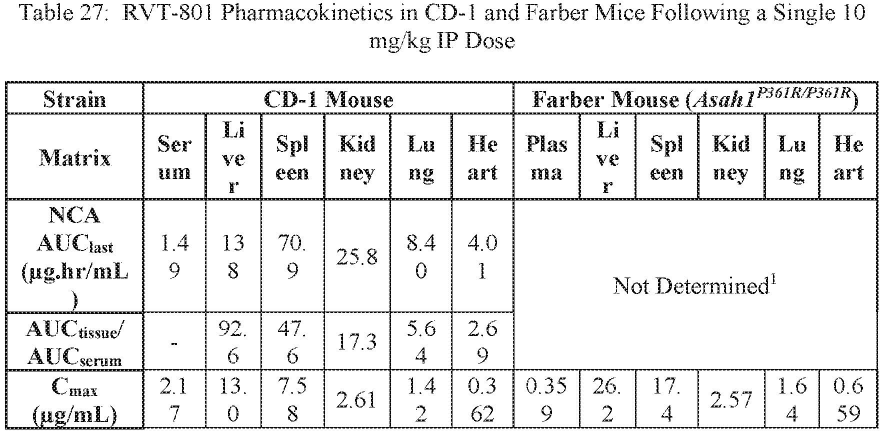

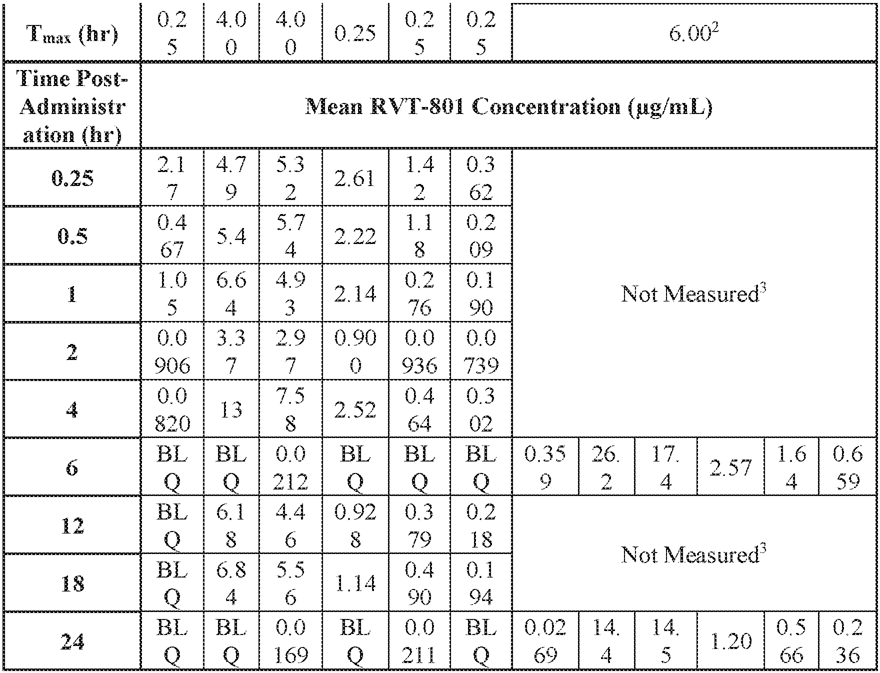

Table 27. RVT-801 pharmacokinetic parameters in healthy male CD-1 mice, aged approximately 3.5 weeks, and 4-5-week-old male and female Farber mice (*Asah1*^{*P361R*/*P361R*}), following single 10 mg/kg bolus IP injection of RVT-801 in sterile saline. 33 total CD-1 mice and 6 total Farber mice were used (n=3 per time point for each strain); mean data reported. RVT-801 sample concentration determined by sandwich ELISA. ELISA Calibrated Range: 20.0-1224 ng/mL (serum), 0.400-24.5 ng/mL (tissue), 20.0-1280 ng/mL (plasma). RVT-801 brain exposure was limited in CD-1 mice and was not quantifiable in singly-dosed Farber mice, and therefore is not shown.

¹AUC was not calculated for Farber mice due to insufficient concentration-time data points over the post-administration period, a function of the limited number of *Asah1*^{*P361R*/*P361R*} animals available.

Maximum RVT-801 concentration was measured at 6 hours post-administration (the earlier post-dose assessment) across all biological matrices collected from Farber mice; these data may be biased by the limited number of feasible sampling time points.

³Due to limited availability of Farber mice, pharmacokinetic sample collection was restricted to 6- and 24-hours post-administration.

Systemic rhAC levels, measured in Farber plasma and in CD-1 serum, following a single 10 mg/kg IP dose of RVT-801 suggest that Farber mice experienced higher systemic rhAC exposure than CD-1 mice as indicated by higher levels at 6 hours post-administration and sustained presence in circulation. with quantifiable 24-hour concentration-time points in Farber plasma, whereas rhAC was not detectable/below the limit of quantitation beyond 6 hours in CD-1 mice serum. (Fig. 16).

In Farber mice, the highest concentration of RVT-801 across all biological matrices analyzed occurred at 6 hours post-dose. While the Farber studies were not designed to explicitly define Cₘₐₓ, and a direct comparison of rhAC concentrations at 6 hours post-dose in Farber to CD-1 mice could not be made in light of a dearth of CD-1 data at that specific time point, at 6 hours the Farber liver, spleen, lung, and heart concentrations were nonetheless higher than the Cₘₐₓ observed in the corresponding CD-1 tissues. The exception was kidney, where CD-1 mice exhibited slightly higher concentrations. At 24 hours post-administration rhAC levels in CD-1 tissues were either below the limit of quantitation or falling rapidly, while concentrations in Farber mouse tissue remained significantly above the LLOQ and high relative to Tₗₐₛₜ serum data in CD-1 mice.

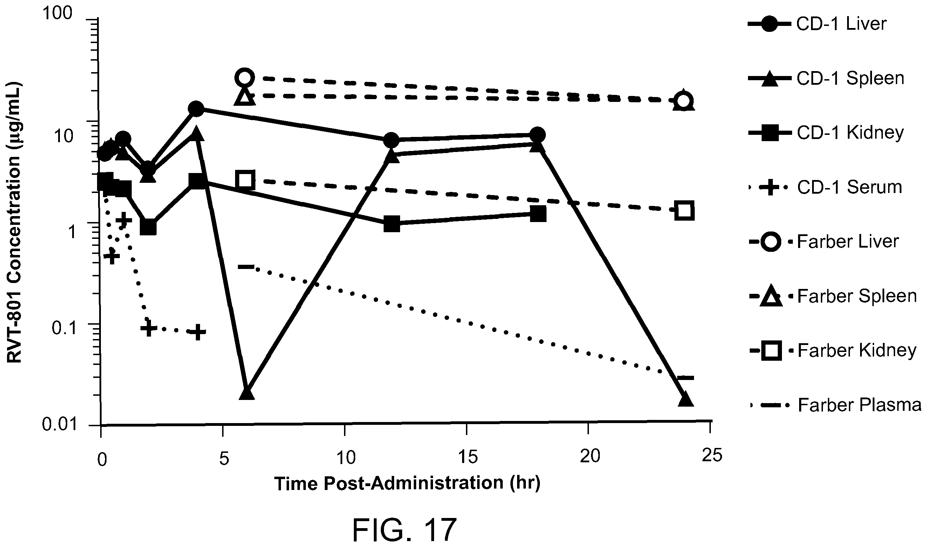

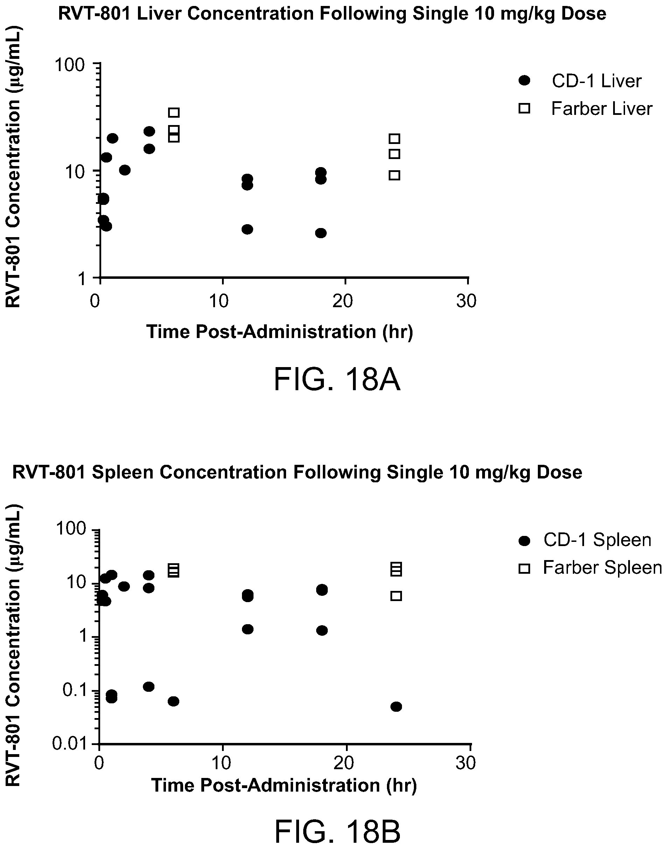

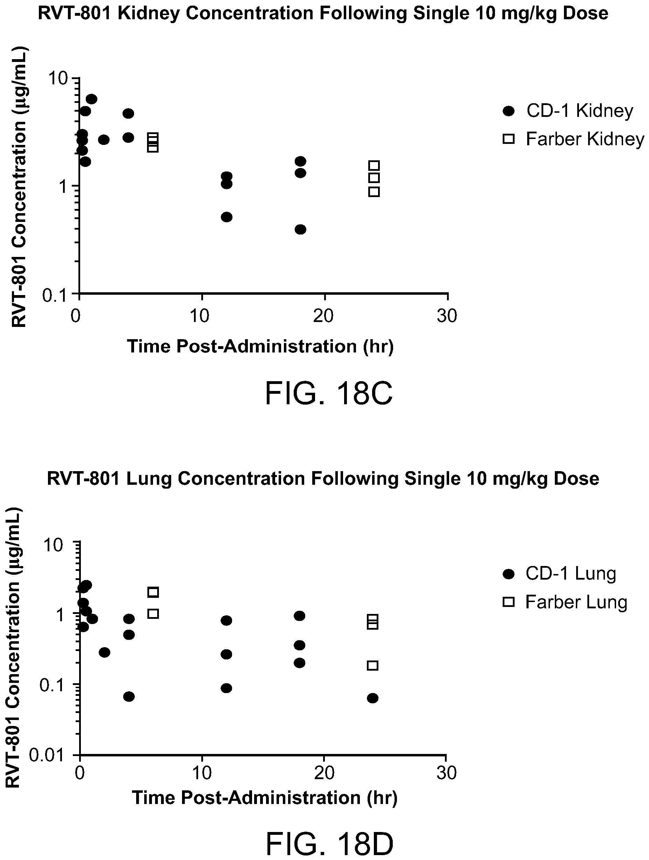

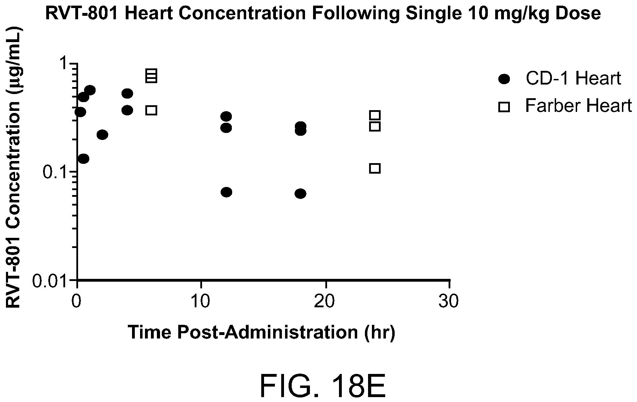

A comparison of rhAC in circulation and in the major tissues of exposure following a single dose of RVT-801 is illustrated in Fig. 17 A comparison of the spread of individual data points is presented in Fig. 18, where the variability of rhAC concentration at a given time point following a single dose of RVT-801 can be appreciated across the tissues analyzed. Again, the increased exposure in Farber relative to CD-1 mice at 24 hours post-dose is readily apparent.

Fig. 17 shows concentration of rhAC in circulation and in the three major tissues of exposure in Farber (open symbols) and CD-1 (filled symbols) mice following a single 10 mg/kg bolus IP injection of RVT-801, n=3 animals per time point. Samples were collected and analyzed out to 24 hours post-administration. Samples from Farber mice were collected at 6- and 24-hours post-dose only. The 6-hour CD-1 spleen data is driven by a single quantifiable value.

Fig. 18 presents individual rhAC concentration-time data in the liver (Fig. 18A), spleen (Fig. 18B), kidney (Fig. 18C), lung (Fig. 18D), and heart (Fig. 18E) Farber (open symbols) and CD-1 (filled symbols) mice following a single 10 mg/kg bolus IP injection of RVT-801, n=3 per time point. Samples were collected and analyzed out to 24 hours post-administration.

Juvenile, healthy CD-1 mice are considered the parental strain of the Farber disease mouse model. Fig 3 shows that the juvenile CD-1 mouse RVT-801 pharmacokinetic profile is similar to the Farber mouse experimental rhAC activity profile in blood, This indicates that the juvenile CD-1 mouse provides an appropriate approximation of the Farber mouse PK - since Farber mice are frail, difficult to mate, and not numerous enough to conduct a full PK assessment.

Although based on a small number of Farber mice and with limited overlapping data points between strains, the data suggest that systemic rhAC exposure is significantly higher in Farber relative to CD-1 mice following a single 10 mg/kg dose of RVT-801. The incurred systemic PK samples differed in a number of potentially important ways; Farber mice were of mixed-sex, were significantly smaller than the healthy, age-matched CD-1s, and exhibited profound pathology, yielded plasma samples, while serum was collected from the juvenile male CD-1 mice.

The observed differences in RVT-801 exposure are likely not the result of analyzing different matrices between strains, as the protein content (aside from clotting factors) of serum and plasma is largely similar. Additionally, the increase in measured Farber plasma exposure was not a result of increased plasma ELISA sensitivity relative to that in serum and/or the ability to report additional data points (i.e. at 24 hours post-dose) that would have been undetectable in CD-1 serum due to differences in method sensitivity. Reported Farber plasma data were well above the LLOQ and the plasma and serum methods performed similarly (Table 27).

**Table 27: ELISA Performance - Serum vs. Plasma**

| **ELISA Method** | **MRD** | **LLOQ** | **ULOQ** |
|---|---|---|---|
| **Serum (BioAgilytix)** | 50-fold | 20 ng/mL | 1224 ng/mL |
| **Plasma (Syneos)** | 50-fold | 20 ng/mL | 1280 ng/mL |

| | | | |
|---|---|---|---|
| MRD: Minimum required dilution LLOQ: Lower limit of quantitation ULOQ: Upper limit of quantitation | | | |

RhAC concentration in tissues following dosing did not appear to be dependent on the tissue to body weight ratio in Farber mice^{a}.

"Based on repeat dose Farber animals from RVT-801-9025; sex and bodyweights not recorded for RVT-801-9021.

Although marked immunological differences between wild-type and Farber mice have been demonstrated, their impact on the pharmacokinetics of RVT-801 are unclear. Anti-drug antibodies (ADA) can prolong circulation of drug-antibody complexes, but the PK differences under immediate discussion were observed within 24 hours of the administration of a single dose of RVT-801, and no ADA were detected in these samples .

It is possible that that the variance in exposure observed in Farber mice relative to CD-1 mice was a consequence of multi-organ physiological alterations of the Farber disease mouse model.

Differences in the rate of distribution of RVT-801 from the peritoneal cavity to the vasculature and/or in the uptake of rhAC from circulation into tissue between Farber and CD-1 mice could explain the PK variances observed between strains. However, these potential trends cannot be assessed from the current studies without a gross over interpretation of the limited available data

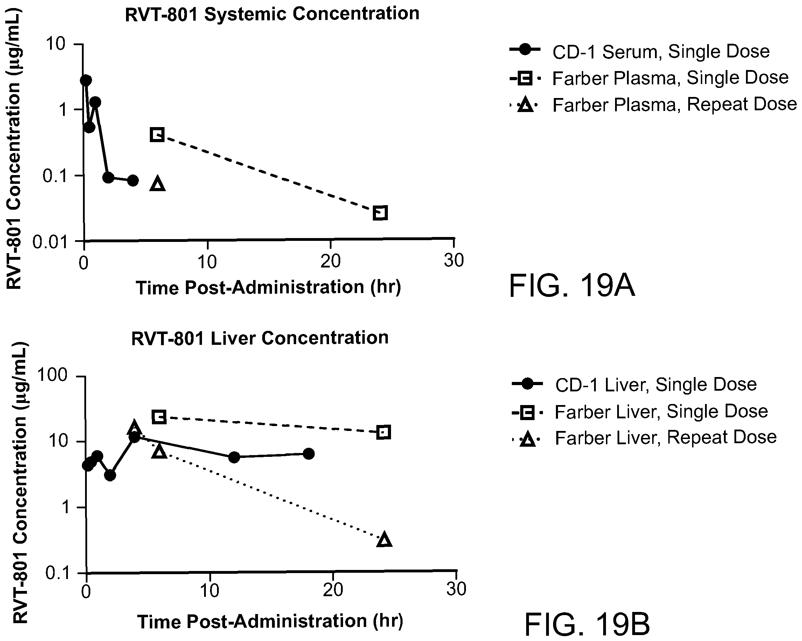

Comparing dose groups in RVT-801-9021, there was no apparent accumulation of rhAC upon repeat weekly administration of RVT-801. Rather, the data, however limited, suggest that there were lower concentrations of rhAC in circulation and across the tissues analyzed when RVT-801 was administered over multiple once-weekly doses as compared to a single injection at the same dose level (10 mg/kg/dose). Across all matrices, with the exception of brain, single dose concentration-time data at 6- and 24-hours post-administration were higher following a single dose of RVT-801 than after three weekly doses Fig. 19A-G. Further, the data are consistent with a scenario of lower exposures owing to both lesser absolute rhAC concentrations and higher rates of clearance following multiple doses of RVT-801. Further studies would be necessary to explore these apparent trends and establish whether the data are consistent with the general murine pharmacokinetics of RVT-801 upon repeat administration, or if features of the pathology inherent to the Farber disease mouse model influence the uptake, disposition, and clearance of rhAC.

Figs. 19A-G present the mean rhAC concentration in CD-1 mice following a single 10 mg/kg dose and Farber mice following either a single 10 mg/kg dose or multiple once-weekly doses at a 10 mg/kg/dose administered via bolus IP injection, in accordance with Example 5 (RVT-801-9021).

Figs. 20A and 20B show mean rhAC tissue concentration-time profiles following IP administration to juvenile CD-1 mice in RVT-801-9013 Part B (Linear Fig. 20A and Log-Linear Fig. 20B), respectively.

Fig. 21 presents RVT-801 tissue:serum exposure ratios in BALF, blood, brain, heart, kidney, liver, lung and spleen based on AUCₗₐₛₜ based on single doses of RVT-801 of 1 mg/kg, 3 mg/kg and 10 mg/kg in CD-1 mice.

Figs. 22A-D presents dose normalized PK parameters plotted verses dose level for various tissues following IP administration of RVT-801 at doses of 1 mg/kg, 3 mg/kg and 10 mg/kg in CD-1 mice.

Fig. 19A presents a plot of systemic rhAC concentrations in serum after a single dose administration of 10 mg/kg/dose via bolus IP injection of RVT-801. Fig. 19A also presents plasma concentrations in Farber mice after dose administration of 10 mg/kg/dose via bolus IP injection of RVT-801. In addition, Fig. 19A presents plasma concentrations following a repeat dose administration of injection of RVT-80. Also presented ae serum concentrations in CD-1 mice administered a single 10 mg/kg/dose via bolus IP injection of RVT-801.

Fig. 19B presents concentrations of RVT-801 in liver tissue in CD-1 mice after a single 10 mg/kg/dose via bolus IP injection of RVT-801. Fig. 19B also presents concentrations of RVT-801 in liver tissue in Farber mice treated with a single 10 mg/kg/dose via bolus IP injection of RVT-801. Fig. 19B additionally presents concentrations of RVT-801 in liver tissue in Farber mice treated with repeated 10 mg/kg/doses via bolus IP injection of RVT-801.

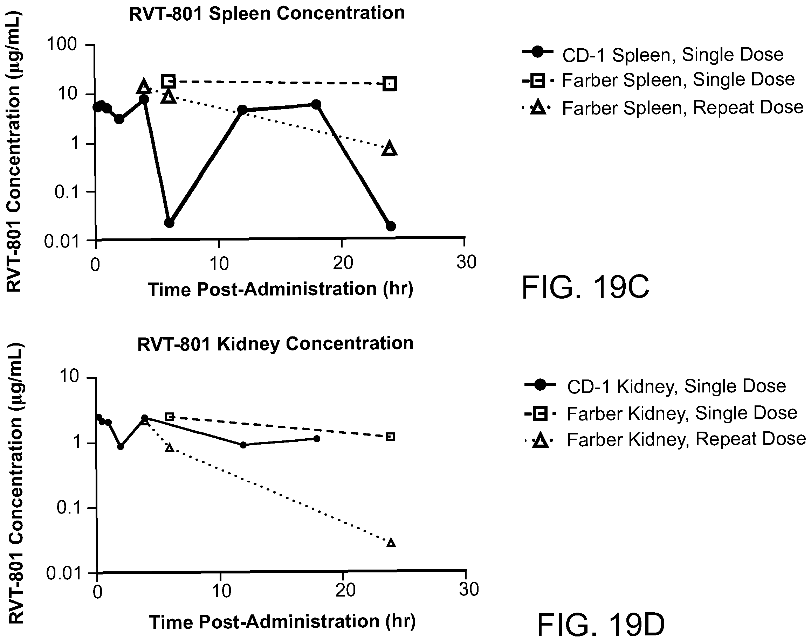

Fig. 19C presents concentrations of RVT-801 in spleen tissue in CD-1 mice after a single 10 mg/kg/dose via bolus IP injection of RVT-801. Fig. 19C also presents concentrations of RVT-801 in spleen tissue in Farber mice treated with a single 10 mg/kg/dose via bolus IP injection of RVT-801. Fig. 19C additionally presents concentrations of RVT-801 in liver tissue in Farber mice treated with repeated 10 mg/kg/doses via bolus IP injection of RVT-801.

Fig. 19D presents concentrations of RVT-801 in kidney tissue in CD-1 mice after a single 10 mg/kg/dose via bolus IP injection of RVT-801. Fig. 19D also presents concentrations of RVT-801 in kidney tissue in Farber mice treated with a single 10 mg/kg/dose via bolus IP injection of RVT-801. Fig. 19C additionally presents concentrations of RVT-801 in kidney tissue in Farber mice treated with repeated 10 mg/kg/doses via bolus IP injection of RVT-801.

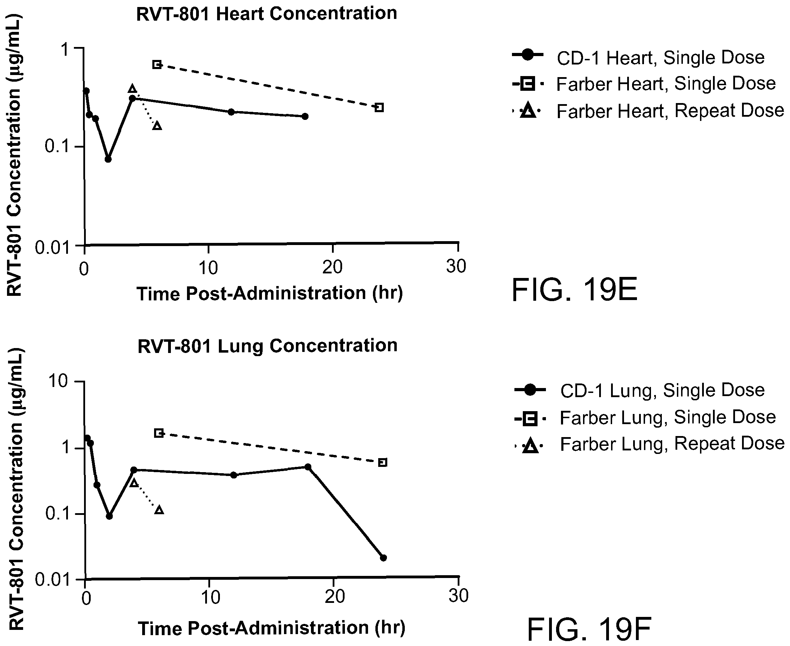

Fig. 19E presents concentrations of RVT-801 in heart tissue in CD-1 mice after a single 10 mg/kg/dose via bolus IP injection of RVT-801. Fig. 19E also presents concentrations of RVT-801 in heart tissue in Farber mice treated with a single 10 mg/kg/dose via bolus IP injection of RVT-801. Fig. 19E additionally presents concentrations of RVT-801 in heart tissue in Farber mice treated with repeated 10 mg/kg/doses via bolus IP injection of RVT-801.

Fig. 19F presents concentrations of RVT-801 in lung tissue in CD-1 mice after a single 10 mg/kg/dose via bolus IP injection of RVT-801. Fig. 19F also presents concentrations of RVT-801 in lung tissue in Farber mice treated with a single 10 mg/kg/dose via bolus IP injection of RVT-801. Fig. 19F additionally presents concentrations of RVT-801 in lung tissue in Farber mice treated with repeated 10 mg/kg/doses via bolus IP injection of RVT-801.

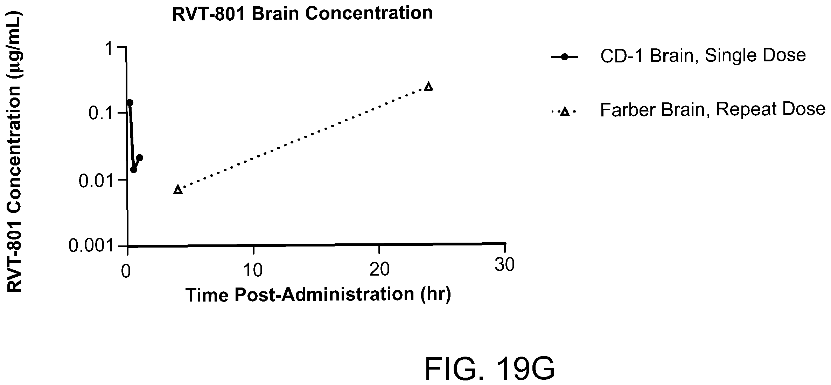

Fig. 19G presents concentrations of RVT-801 in brain tissue in CD-1 mice after a single 10 mg/kg/dose via bolus IP injection of RVT-801. Fig. 19G additionally presents concentrations of RVT-801 in brain tissue in Farber mice treated with repeated 10 mg/kg/doses via bolus IP injection of RVT-801.

### Abbreviations.

ASAH1 = acid ceramidase gene.
AUC = area under the concentration-time curve.
AUC_{(0-∞)} = area under the plasma concentration-time curve from time zero extrapolated to time infinity.
AUC₍₀₋ₜ₎ = area under the plasma concentration-time curve from time zero to last sample time.
BMI = body mass index.
BW = body weight.
cDNA = complementary deoxyribonucleic acid.
CFR = Code of Federal Regulations.
CI =confidence interval.
CL = clearance.
Cmax = maximum concentration.
CNS = central nervous system.
FDA = Food and Drug Administration.
HED = human-equivalent dose.
HSCT = hematopoietic stem cell transplantation.
IP = intraperitoneal.
IV = intravenous.
MCP-1 = monocyte chemoattractant protein 1.
PD = pharmacodynamic(s).
PK = pharmacokinetic(s).
PRN =pro re nata.
rhAC = recombinant human acid ceramidase.
SD = standard deviation.
SMA-PME = Spinal Muscular Atrophy with Progressive Myoclonic Epilepsy.
tₘₐₓ = time to maximum concentration.

References discussed in the application, which are incorporated by reference in their entirety, for their intended purpose, which is clear based upon its context.
Alayoubi, A.M., J.C. Wang, B.C. Au, S. Carpentier, V. Garcia, S. Dworski, S. El-Cihamrasni, K.N. Kirouac, M.J. Exertier, Z.J. Xiong, G.G. Prive, C.M. Simonaro, J. Casas, G. Fabrias, E.H. Schuchman, P.V. Turner, R. Hakem, T. Levade, and J.A. Medin, 2013, Systemic ceramide accumulation leads to severe and varied pathological consequences, EMBO Mol Med, 5:827-842.
Bae, J.S., Jang, K.H., Schuchman, E.H., and Jin, H.K., 2004, "Comparative effects of recombinant acid sphingomyelinase administration by different routes in Niemann-Pick disease mice," Exp Anim, 53:417-421.
Becker et al., 2010, "Acid Sphingomyelinase Inhibitors Normalize Pulmonary Ceramide and Inflammation in Cystic Fibrosis," Am. J. Respir. Cell. Mol. Biol., 42:716-724.
Berkhout J, Melchers MJ, van Mil AC, Seyedmousavi S, Lagarde CM, Nichols WW, Mouton JW, 2015 "Pharmacokinetics and penetration of ceftazidime and avibactam into epithelial lining fluid in thigh- and lung-infected mice,". Antimicrob Agents Chemother., 59(4): 2299-2304.
Bernardo, K., R. Hurwitz, T. Zenk, R.J. Desnick, K. Ferlinz, E.H. Schuchman, and K. Sandhoff, 1995, "Purification, characterization, and biosynthesis of human acid ceramidase," J Biol Chem, 270:11098-11102.
Boado, R.J., Lu, J.Z., Hui, E.K., Lin, H., and Pardridge, W.M., 2016, "Insulin receptor antibody-alpha-N-acetylglucosaminidase fusin portein penetrates the primate blood-brain barrier and reduces glycosaminoglycans in Sanfillippo type B fibroblasts," Mol. Pharm., 13:1385-92.
Bonafé L, Kariminejad A, Li J, Royer-Bertrand B, Garcia V, Mahdavi S, Bozorgmehr B, Lachman R, Mittaz-Crettol L, Campos-Xavier B, Namputhiri S, Unger S, Rivolta C, Levade T, Superti-Furga A, 2016, "Peripheral osteolysis in adults linked to ASAH1 (acid ceramidase) mutations: a new presentation of Farber disease," Arthritis Rheumatol 2016 Mar 4.
Chatelut, M., Harzer, K., Christomanou, H., Feunteun, J., Pieraggi, M.T., Paton, B.C., Kishimoto, Y., O'Brien, J.S., Basile, J.P., Thiers, J.C., Salvayre, R., and Levade, T., 1997, "Model SV40-transformed fibroblast lines for metabolic studies of human prosaposin and acid ceramidase deficiencies," Clin Chim Acta, 262:61-76.
Coppoletta JM, Wolbach SB., 1933, "Body Length and Organ Weights of Infants and Children: A Study of the Body Length and Normal Weights of the More Important Vital Organs of the Body between Birth and Twelve Years of Age," Am J Pathol. 1933 Jan;9(1):55-70.
Desnick, R.J. and Schuchman, E.H., 2012, "Enzyme replacement therapy for lysosomal storage diseases: lessons from 20 years of experience and remaining challenges," Annu Rev Genomics Hum Genet, 13:307-335*.*
Dworski, S., Berger, A., Furlonger, C., Moreau, J.M., Yoshimitsu, M., Trentadue, J., Au, B.C., Paige, C.J., and Medin, J.A., 2015," "Markedly perturbed hematopoiesis in acid ceramidase deficient mice," Haematologica, 100(5):e162-165.
Dworski, S., Lu, P., Khan, A., Maranda, B., Mitchell, J.J., Parini, R., Di Rocco, M., Hugle, B., Yoshimitsu, M., Magnusson, B., Makay, B., Arslan, N., Guelbert, N., Ehlert, K., Jarisch, A., Gardner-Medwin, J., Dagher, R., Terreri, M.T., Lorenco, C.M., Barillas-Arias, L., Tanpaiboon, P., Solyom, A., Norris, J.S., He, X., Schuchman, E.H., Levade, T., and Medin, J.A., 2017, "Acid Ceramidase Deficiency is characterized by a unique plasma cytokine and ceramide profile that is altered by therapy," Biochim Biophys Acta, 1863(2):386-394.
Ehlert K, Frosch M, Fehse N, Zander A, Roth J, Vormoor J, 2007, "Farber disease: clinical presentation,pathogenesis and a new approach to treatment," Pediatric Rheumatology, 5:15.
Eliyahu, E., Park, J.H., Shtraizent, N., He, X., and Schuchman, E.H., 2007, "Acid ceramidase is a novel factor required for early embryo survival," FASEB J., 21:1403-9.
Eliyahu, E., N. Shtraizent, K. Martinuzzi, J. Barritt, X. He, H. Wei, S. Chaubal, A.B. Copperman, and E.H. Schuchman, 2010, "Acid ceramidase improves the quality of oocytes and embryos and the outcome of in vitro fertilization," FASEB J, 24:1229-1238.
Eliyahu, E., N. Shtraizent, R. Shalgi and E.H. Schuchman, 2012, "Construction of conditional acid ceramidase knockout mice and in vivo effects on oocyte development and fertility. Cellular physiology and biochemistry," Int'r J Exper Cellular Physiol Biochem and Pharmacol," 30:735-748.
Farber, S., 1952, "A lipid metabolic disorder - disseminated 'Lipogranulomatosis' - a syndrome with similarity to, and important difference from, Niemann-Pick and Hand-Schuller-ehristian disease," Am. J. Dis. Child, 84:499.
Food and Drug Administration (FDA), 2005, July, "Estimating the Maximum Safe Starting Dose in Initial Clinical Trials for Therapeutics in Adult Healthy Volunteers." Available at:
   https://www.fda.gov/downloads/drugs/guidances/ucm078932.pdf.
   Food and Drug Administration (FDA), 2015, May, "Draft Guidance: Investigational Enzyme Replacement Therapy Products: Nonclinical Assessment." Available at: https://www.fda.gov/downloads Drugs/GuidanceComplianceRegulatoryInformation/Guid ances/UCM446569.pdf .
Frohbergh, M.E., Guevara, J.M., Greisamer, R.P., Barbe, M.F., He, X., Simonaro, C.M., and Schuchman, E.H., 2016, "Acid ceramidase treatment enhances the outcome of autologous chondrocyte implantation in a rat osteochondral defect model," Osteoarthritis Cartilage, 24:752-762.
Gatt, S., 1963, "Enzymic hydrolysis and synthesis of ceramides, "J Biol Chem, 238:3131-3133.
Hassler et al., 1993, "Ceramidase: Enzymology and metabolic roles," Adv. Lipid Res. 26:49-57*.*
He X, Okino N, Dhami R, Dagan A, Gatt S, Schulze H, Sandhoff K, Schuchman EH., 2003, "Purification and characterization of recombinant, human acid ceramidase. Catalytic reactions and interactions with acid sphingomyelinase, " J Biol Chem. 29;278(35):32978-86.
He X, Dworski S, Zhu C, DeAngelis V, Solyom A, Medin JA, Simonaro CM, Schuchman EH., 2017, "Enzyme replacement therapy for Farber disease: Proof-of-concept studies in cells and Mice, " BBA Clin. 2017 Feb 13;7:85-96.
Hollak, C.E. and Wijburg, F.A., 2014, "Treatment of lysosomal storage disorders: successes and challenges," J Inherit Metab Dis, 37:587-598.
Hügle B, Mueller L, Levade T., 2014, "Why Farber disease may be misdiagnosed as juvenile idiopathic arthritis," The Rheumatologist. 2014; 8: 35.
Jablonski, K.A., Amici, S.A., Webb, L.M., Ruiz-Rosado, J.D., Popovich, P.G., Partida-Sanchez, S., and Guerau-de-Arellano, M., 2015, "Novel Markers to Delineate Murine M1 and M2 Macrophages," PLoS ONE 10(12):e0145342.
Koch et al., 1996, "Molecular Cloning and Characterization of a Full-length Complementary DNA Encoding Human Acid Ceramidase: Identification Of The First Molecular Lesion Causing Farber Disease," J. Biol. Chem. 271:33110-33115.
Kostik M, Chikova I, Avramenko V, Vasyakina L, Le Trionnaire E, Chasnyk V, Levade T, 2013, "Farber lipogranulomatosis with predominant joint involvement mimicking juvenile idiopathic arthritis," J Inherit Metab Dis., Nov;36(6):1079-80.
Li, C.M., J.H. Park, X. He, B. Levy, F. Chen, K. Arai, D.A. Adler, C.M. Disteche, J. Koch, K. Sandhoff, and E.H. Schuchman, 1999, "The human acid ceramidase gene (asah): Structure, chromosomal location, mutation analysis, and expression," Genomics, 62(2):223-231.
Li, C.M., S.B. Hong, G. Kopal, X. He, T. Linke, W.S. Hou, J. Koch, S. Gatt, K. Sandhoff, and E.H. Schuchman, 1998, "Cloning and characterization of the full-length cDNA and genomic sequences encoding murine acid ceramidase," Genomics, 50(2):267-274.
Li, C.M., J.H. Park, C.M. Simonaro, X. He, R.E. Gordon, A.H. Friedman, D. Ehleiter, F. Paris, K. Manova, S. Hepbildikler, Z. Fuks, K. Sandhoff, R. Kolesnick, and E.H. Schuchman, 2002, "Insertional mutagenesis of the mouse acid ceramidase gene leads to early embryonic lethality in homozygotes and progressive lipid storage disease in heterozygotes," Genomics, 79(2):218-224.
Murray JM, Thompson, AM, Vitsky A, Hawes M, Chuang WL, Pacheco J, Wilson S, McPherson JM, Thurberg BL, Karey KP, and Andrews L., 2015, "Nonclinical safety assessment of recombinant human acid sphingomyelinase (rhASM) for the treatment of acid sphingomyelinase deficiency: the utility of animal models of disease in the toxicology evaluation of potential therapeutics," Mol Genet Metab, 114:217-225.
Nikolova-Karakashian et al., 2000, "Ceramidases," Methods Enzymol. 311:194-201 (2000).
Okino, N., He, X., S. Gatt, K. Sandhoff, M. Ito, and E.H. Schuchman, 2003, "The reverse activity of human acid ceramidase," J Biol Chem, 278(32):29948-29953.
Park, J.-H., Schuchman E. H., 2006, "Acid ceramidase and human disease," Biochim. Biophys. Acta. 1758(12): 2133-2138.
Ramsubir S, Nonaka T, Bedia Girbés C, Carpentier S, Levade T, Medin J, 2008, "In vivo delivery of human acid ceramidase via cord blood transplantation and direct injection of lentivirus as novel treatment approaches for Farber disease," Mol Genet Metab. November; 95(3): 133-141.
Realini, N., Palese, F., Pizzirani, D., Pontis, S., Basit, A., Bach, A., Ganesan, A., and Piomelli, D., 2015, "Acid ceramidase in melanoma: expression, localization and effects of pharmacological inhibition," J Biol Chem, N291:2422-2434.
Rennard SI, Basset G, Lecossier D, O'Donnell KM, Pinkston P, Martin PG, Crystal RG, 1986, "Estimation of volume of epithelial lining fluid recovered by lavage using urea as marker of dilution," J of Applied Physiology. 1986, 60(2): 532-538.
Roh, J.L., Park, J.Y., Kim, E.H., and Jang, H.J., 2016, "Targeting acid ceramidase sensitises head and neck cancer to cisplatin," Eur J Cancer, 52:163-72.
Sands, M., 2013, "Farber disease: understanding a fatal childhood disorder and dissecting ceramide biology," EMBO MolMed., Jun;5(6):799-801.
Shiffmann, S., Hartmann, D., Birod, K., Ferreiros, N., Schreiber, Y., Zivkovic, A., Geisslinger, G., Grösch, S., and Stark, H., 2012, "Inhibitors of Specific Ceramide Synthases," Biochimie, 94(2):558-565.
Schuchman, E.H., 2016, "Acid ceramidase and the treatment of ceramide diseases. The expanding role of enzyme replacement therapy," Biochim Bipphys Acta, 1862:1459-1471.
Schuchman, E.H. (inventor), Icahn School of Medicine at Mount Sinai (applicant), Recombinant Human Acid Ceramidase Production Process, International application under the Patent Cooperation Treaty, PCT/US2018/052463 (not yet published)
Schuchman, E. H. (inventor), Icahn School of Medicine at Mount Sinai (applicant), 2016, February 11, Therapeutic Acid Ceramidase Compositions And Methods Of Making And Using Them, published as U.S. Published Patent Application No. US 2016/0038574 A1.
Schuchman, E.H. (inventor), Icahn School of Medicine at Mount Sinai (applicant), Recombinant Human Acid Ceramidase Production Process, International application under the Patent Cooperation Treaty, PCT/US2018/052463.
Schuchman, E. H. (inventor), Icahn School of Medicine at Mount Sinai (applicant), 2018, January 12, Compositions and Methods for Treating Farber Disease, International application under the Patent Cooperation Treaty, WO 2018/132667
Simonaro, C.M., Sachot, S., Ge, Y., He, X., DeAngelis, V.A., Eliyahu, E., Leong, D.J., Sun, H.B., Mason, J.B., Haskins, M.E., Richardson, D.W., and Schuchman, E.H., 2013, "Acid ceramidase maintains the chondrogenic phenotype of expanded primary chondrocytes and improves the chondrogenic differentiation of bone marrow-derived mesenchymal stem cells," PLoS One, 8:e62715.
Shtraizent, N., E. Eliyahu, J.H. Park, X. He, R. Shalgi and E.H. Schuchman, 2008, "Autoproteolytic cleavage and activation of human acid ceramidase," J Biol Chem, 283(17):11253-11259.
Solyom A, Huegle B, Magnusson B, Makay B, Arslan N, Mitchell J, Tanpaiboon P, Guelbert N, Puri R, Jung L, Grigelioniene G, Ehlert K, Beck M, Simonaro C, Schuchman E., 2015, "Farber Disease: Important Differential Diagnostic Information for JIA and Other Inflammatory Arthritis Phenotypes Is Revealed By Data from the Largest Clinical Cohort to Date," Arthritis Rheumatol., 67 (sup.10), abstract
Sugita, M., Dulaney, J.T., and Moser, HW, 1972, "Ceramidase deficiency in Farber's disease (lipogranulomatosis)," Science, 178(4065):1100-1102
Tetzl D, Coyne K, Johnson B, Solyom A, Ehlert K., 2017, "A Qualitative Research Study Documenting the Clinical Impact of Symptoms in a Diverse Population of Patients and Caregivers with Acid Ceramidase Deficiency (Farber Disease). Journal of Inborn Errors of Metabolism & Screening. 2017;5:324.
Torcoletti M, Petaccia A, Pinto RM, Hladnik U, Locatelli F, Agostoni C, Corona F., 2014, "Farber disease in infancy resembling juvenile idiopathic arthritis: identification of two new mutations and a good early response to allogeneic haematopoietic stem cell transplantation. Rheumatology (Oxford), Aug;53(8):1533-4.
Young et al., 2013, "Sphingolipids: regulators of crosstalk between apotosis and autophagy," J. Lipid. Res. 54:5-19*.*
Zhou J, Tawk M, Tiziano FD, Veillet J, Bayes M, Nolent F, Garcia V, Servidei S, Bertini E, Castro-Giner F, Renda Y, Carpentier S, Andrieu-Abadie N, Gut I, Levade T, Topaloglu H, Melki J., 2012, "Spinal muscular atrophy associated with progressive myoclonic epilepsy is caused by mutations in ASAH," Am J Hum Genet. 2012 Jul 13; 91(1):5-14.

The disclosures of each and every patent, patent application, publication, and accession number cited herein are hereby incorporated herein by reference in their entirety.

While present disclosure has been disclosed with reference to various embodiments, it is apparent that other embodiments and variations of these may be devised by others skilled in the art without departing from the true spirit and scope of the disclosure. The appended claims are intended to be construed to include all such embodiments and equivalent variations.

### EQUIVALENTS

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims and any equivalents thereof.

As used herein, the term about refers to a numeric value, including, for example, whole numbers, fractions, and percentages, whether or not explicitly indicated. The term about generally refers to a range of numerical values (e.g., +/-5-10% of the recited range) that one of ordinary skill in the art would consider equivalent to the recited value (e.g., having the same function or result). When terms such as at least and about precede a list of numerical values or ranges, the terms modify all of the values or ranges provided in the list. In some instances, the term about may include numerical values that are rounded to the nearest significant figure.

### Paragraphs Of The Invention

1. A method of treating Farber disease in a human subject in need thereof, the method comprising administering to the human subject purified recombinant human acid ceramidase (rhAC) in activated form at a dose of about 1 mg/kg to about 10 mg/kg.
2. The method of paragraph 1, wherein the dose is about 1 mg/kg to about 5 mg/kg.
3. The method of paragraph 1, wherein the dose is about 1 mg/kg.
4. The method of paragraph 1, wherein the dose is about 2 mg/kg.
5. The method of paragraph 1, wherein the dose is about 2.5 mg/kg.
6. The method of paragraph 1, wherein the dose is about 5 mg/kg.
7. The method of paragraph 1, wherein the dose is about 10 mg/kg.
8. The method of any one of paragraphs 1 to 7, wherein the purified recombinant human acid ceramidase (rhAC) in activated form is RVT-801, wherein RVT-801 comprises a recombinantly produced acid ceramidase (rhAC) purified to a purity of at least 95% activated form by a process comprising the steps of subjecting the recombinantly produced acid ceramidase to at least two chromatography steps selected from i) cation exchange chromatography; ii) hydrophobic interaction chromatography (HIC); and iii) anion exchange chromatography; and further
   subjecting the rhAC in solution to one or more viral inactivation steps, wherein in the one or more viral inactivation steps the rhAC solution is titrated to a pH of 3.7 or less.
9. The method of paragraph 8, wherein the recombinantly produced acid ceramidase is subjected to three chromatography steps consisting essentially of i) cation exchange chromatography; ii) hydrophobic interaction chromatography (HIC); and iii) anion exchange chromatography.
10. The method of paragraph 8, wherein the one or more viral inactivation steps is performed before the chromatography steps.
11. The method of paragraph 8, wherein the one or more viral inactivation steps is conducted before one of the at least two chromatography steps.
12. The method of paragraph 8, wherein the one or more viral inactivation steps is conducted before at least two of the at least two chromatography steps.
13. The method of paragraph 8, wherein the titration to pH 3.7 is performed with citric acid.
14. The method of paragraph 9, wherein the titration to pH 3.7 is performed with citric acid.
15. The method of paragraph 10, wherein the titration to pH 3.7 is performed with citric acid
16. The method of any of the preceding paragraphs, wherein the purity of the purified recombinant human acid ceramidase (rhAC) in activated form is at least 95% by weight.
17. The method of any of the preceding paragraphs, wherein the purity of the purified recombinant human acid ceramidase (rhAC) in activated form is at least 96% by weight.
18. The method of any of the preceding paragraphs, wherein the purity of the purified recombinant human acid ceramidase (rhAC) in activated form is at least 97% by weight.
19. The method of any of the preceding paragraphs, wherein the purity of the purified recombinant human acid ceramidase (rhAC) in activated form is at least 98% by weight.
20. The method of any of the preceding paragraphs, wherein the purity of the purified recombinant human acid ceramidase (rhAC) in activated form is at least 99% by weight.
21. The method of any of the preceding paragraphs, wherein the purity of the purified recombinant human acid ceramidase (rhAC) in activated form is substantially 100% by weight.
22. The method of paragraph 1, wherein the dose in a human adult subject is about 0.8 mg/kg.
23. The method of paragraph 1, wherein the dose in a human child is about 1.2 mg/kg.
24. A method of treating a human subject with Farber disease, comprising administering to the human subject a therapeutically effective dose of recombinant human acid ceramidase (rhAC) wherein the therapeutically effective dose may be determined based on the pharmacokinetic profile obtained in juvenile, healthy CD-1 mice receiving a single, maximum effective dose of 10mg/kg of rhAC intraperitoneally, wherein the rhAC elicits in the healthy CD-1 mice one or more of: Tₘₐₓ of about 0.25 to 1.0 hours, Cₘₐₓ of about 1.23 to about 2.17 µg/mL, or area under the curve (AUC) of about 1 hr*µg/mL to about 2 hr*µg/mL.
25. The method of paragraph 24, wherein the AUC is about 1.37 hr*µg/mL to about 1.49 hr* µg/mL.
26. The method of paragraph 24, wherein the AUC is about 1.37 hr*µg/mL to about 1.49 hr*µg/mL and the Cₘₐₓ is about 1.23 to about 2.17 µg/mL.
27. The method of paragraph 24, wherein the Tₘₐₓ is about 0.25 to 1.0 hours.
28. The method of paragraph 24, wherein the Cₘₐₓ is about 1.23 µg/mL to 2.17 µg/mL.
29. The method of paragraph 24, wherein the AUC of about 1.37 to 1.49 hr*µg/mL.
30. The method of any one of paragraphs 24 to 29, wherein the purified recombinant human acid ceramidase (rhAC) in activated form is RVT-801.
31. A method of treating a human subject with Farber disease, comprising administering to the subject a therapeutically effective dose of rhAC wherein the therapeutically effective dose may be determined based on the pharmacokinetic profile, obtained in juvenile, healthy CD-1 mice receiving a single, maximum effective dose ("MED") of 10 mg/kg dose of rhAC intraperitoneally, wherein the rhAC elicits in the healthy CD-1 mouse a Cₘₐₓ, AUC₀₋ₗₐₛₜ or AUCₜᵢₛₛᵤₑ/AUCₛₑᵣᵤₘ in accordance with the values set forth in Fig. 7.
32. The method of paragraph 31, wherein the rhAC elicits in the healthy CD-1 mouse an AUC_{0 to last} of about 138 hr*ug/mL in liver tissue, and/or a Cₘₐₓ for the drug of about 13 µg/mL in liver tissue.
33. The method of paragraph 31, wherein the rhAC elicits in the healthy CD-1 mouse an AUC_{0 to last} of about 70.9 hr*µg/mL in splenic tissue, and/or a Cₘₐₓ for the drug of about 7.58 µg/mL in splenic tissue.
34. The method of paragraph 31, wherein the rhAC elicits in the healthy CD-1 mouse an AUC_{0 to last} of about 25.8 hr*µg/mL in kidney tissue, and/or a Cₘₐₓ for the drug of about 2.61 µg/mL in kidney tissue.
35. The method of paragraph 31, wherein the rhAC elicits in the healthy CD-1 mouse an AUC_{0 to last} of about 4 hr* µg/mL in heart tissue, and/or a Cₘₐₓ for the drug of about 0.362 µg/mL in heart tissue.
36. The method of paragraph 31, wherein the rhAC elicits in the healthy CD-1 mouse an AUC_{0 to last} of about 0.0419 hr*µg/mL in liver tissue, and/or a Cₘₐₓ for the drug of about 0.147 µg/mL in liver tissue.
37. The method of paragraph 31, wherein the rhAC elicits in the healthy CD-1 mouse an AUC_{0 to last} of about 0.858 hr*µg/mL in blood, and/or a Cₘₐₓ for the drug of about 1.23 µg/mL in blood tissue.
38. The method of paragraph 31, wherein the rhAC elicits in the healthy CD-1 mouse an AUC_{0 to last} of about 0.000245 hr* µg/mL in BALF, and/or a Cₘₐₓ for the drug of about 0.00196 µg/mL in BALF.
39. The method of paragraph 31, wherein the rhAC elicits in the healthy CD-1 mouse an AUC_{0 to last} of about 8.4hr*µg/mL in lung tissue, and/or a Cₘₐₓ for the drug of about 1.42 µg/mL in lung tissue.
40. The method of any one of paragraphs 31 to 39, wherein the purified recombinant human acid ceramidase (rhAC) in activated form is RVT-801.
41. The method of any one of the preceding paragraphs wherein the purified recombinantly produced acid ceramidase has no detectable acid sphingomyelinase activity.
42. The method of any one of the preceding paragraphs, further comprising administering to the human subject an antipyretic, an antihistamine, a corticosteroid, or any combination thereof prior to, concurrently with, or after administration of the purified recombinant human acid ceramidase (rhAC) in activated form.
43. The method of any one of the preceding paragraphs, wherein the purified recombinant human acid ceramidase (rhAC) in activated form is administered in a therapeutic composition.
44. The method of paragraph 43, wherein the therapeutic composition is administered orally, by inhalation, by intranasal instillation, topically, transdermally, parenterally, subcutaneously, by intravenous injection, by intra-arterial injection, by intramuscular injection, intraplurally, intraperitoneally, intrathecally, or by application to a mucous membrane.
45. The method of paragraphs 43 or 44, further comprising one or more repeat administrations of the therapeutic composition.
46. The method of any one of preceding paragraphs, further comprising administering one or more additional agents which reduce ceramide levels.

## Claims

1. Purified recombinant human acid ceramidase (rhAC) for use in a method of treating Farber disease in a human subject in need thereof, the method comprising administering to the human subject purified recombinant human acid ceramidase (rhAC) at a dose of 1 mg/kg to 10 mg/kg.

2. The purified recombinant human acid ceramidase for use according to claim 1, wherein the dose is 1 mg/kg to 5 mg/kg.

3. The purified recombinant human acid ceramidase for use according to claim 1, wherein the dose is about 1 mg/kg, or about 2 mg/kg, or about 2.5 mg/kg, or about 5 mg/kg, or about 10 mg/kg.

4. The purified recombinant human acid ceramidase for use according to claim 1, wherein the dose in a human child is about 1.2 mg/kg.

5. Purified recombinant human acid ceramidase (rhAC) for use in a method of treating Farber disease in a human adult subject in need thereof, the method comprising administering to the human adult subject purified recombinant human acid ceramidase (rhAC) at a dose of about 0.8 mg/kg.

6. The purified recombinant human acid ceramidase for use according any preceding claim, wherein the purified recombinant human acid ceramidase is in activated form.

7. The purified recombinant human acid ceramidase for use claim 6, wherein the purified recombinant human acid ceramidase (rhAC) in activated form is RVT-801.

8. The purified recombinant human acid ceramidase for use according any preceding claim, wherein the purified recombinant human acid ceramidase (rhAC) is administered in a therapeutic composition.

9. The purified recombinant human acid ceramidase for use according to claim 8, wherein the therapeutic composition is administered orally, by inhalation, by intranasal instillation, topically, transdermally, parenterally, subcutaneously, by intravenous injection, by intra-arterial injection, by intramuscular injection, intraplurally, intraperitoneally, intrathecally, or by application to a mucous membrane.

10. The purified recombinant human acid ceramidase for use according to claim 8, further comprising one or more repeat administrations of the therapeutic composition.

11. The purified recombinant human acid ceramidase for use according to claim 10, wherein composition is administered about once a week, once every 2, 3, or 4 weeks, or once a month, for about 10, about 20, or about 30 weeks, 1, 5, 10, or 25 years, or the duration of a patient's life.
